# EUROPEAN PATENT APPLICATION

(11) **EP 4 707 279 A1**
(43) Date of publication of application: **11.03.2026**
(21) Application number: 24800132.3
(22) Date of filing: 02.05.2024
(51) Int. Cl.: C07D 487/14, A61K 31/519, A61P 31/14

(54) **SUBSTITUTED TRICYCLIC HETEROCYCLIC COMPOUND AS PHARMACEUTICAL FOR TREATMENT OR PREVENTION OF COVID-19**

(30) Priority: 02.05.2023 JP 2023076174
(71) Applicant: Albius Sciences Beta Pte. Ltd., Singapore 058357 (SG)
(72) Inventor: MATSUOKA, Shigeru, Oita-shi, Oita 870-1192 (JP); TSUCHIKAWA, Hiroshi, Oita-shi, Oita 870-1192 (JP); YAMADA, Kentaro, Oita-shi, Oita 870-1192 (JP); KATO, Akira, Oita-shi, Oita 870-1192 (JP); SUN, Zhongdong, Hong Kong Science Park, Hong Kong (HK)
(74) Representative: Bird & Bird LLP
(86) International application number: PCT/JP2024/016900
(87) International publication number: WO 2024/228401

(57) **Abstract**

The present invention provides a compound useful for the treatment or prevention of SARS-CoV-2 novel coronavirus infection.

The present invention relates to a compound represented by formula (I), its enantiomer, or a pharmaceutically acceptable salt thereof: 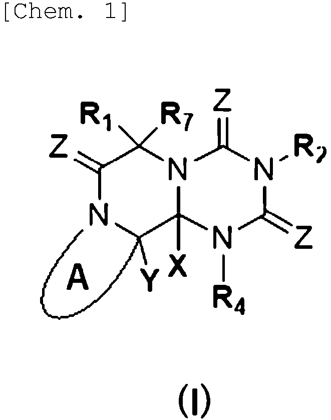 wherein
ring A, R1 and R7, R2 and R4, X and Y, as well as Z are according to the definitions as described in the specification;
use thereof for the treatment or prevention of SARS-CoV-2 novel coronavirus infection; and pharmaceutical compositions comprising the same.

## Description

### TECHNICAL FIELD

The present application claims the priority and benefits under the Paris Convention based on Japanese Patent Application No. 2023-076174 (filed on May 2, 2023), which is incorporated herein by reference in its entirety.

The present invention generally relates to substituted tricyclic heterocyclic compounds for the treatment and/or prevention of novel coronavirus infections, and to pharmaceutical compositions comprising the same.

### BACKGROUND TECHNOLOGY

SARS-CoV-2 novel coronavirus disease (COVID-19) that began spreading worldwide at the end of 2019, continues as pandemic as of February 2023, nearly three years after the outbreak. So far, new therapeutic agents, including the mRNA vaccine and other preventive drugs, have been developed, but the number of newly diagnosed positive cases remains around 2 million per day, and there is no sign of convergence (Non-patent document 1). For this reason, there is a significant possibility that new variants of the SARS-CoV-2 virus will continue to emerge from infected individuals, and there are concerns about the emergence of resistant strains that could evade existing vaccines and therapeutic drugs. Further, the new pandemic is likely to emerge from zoonotic diseases such as the coronavirus and the influenza virus, with the coronavirus also ranking high among the candidates for pathogens that could cause the next pandemic (Non-patent document 2). So, there is an extremely high social demand for the development of therapeutic drugs for new coronavirus disease, which have unprecedented mechanisms of action and molecular structures, and which are effective against resistant strains of existing drugs.

As of February 2023, three types of COVID-19 treatments: antiviral drugs, neutralizing antibody drugs, and antiinflammatory drugs have been approved. Among them, the low molecular weight drugs that can be used as oral medications are generally storable at room temperature and do not require injections, allowing for widespread distribution worldwide regardless of local transportation and medical infrastructure. Therefore, if the low molecular weight drugs can be provided safely, inexpensively, and in large quantities, they could contribute to the resolution of the pandemic. As low molecular weight drugs, three agents have been approved abroad: Remdesivir (1), Molnupiravir (2), and Nirmatrelvir (3)/Ritonavir (4). In addition to them, Enshitrevir (5) has been approved in Japan.

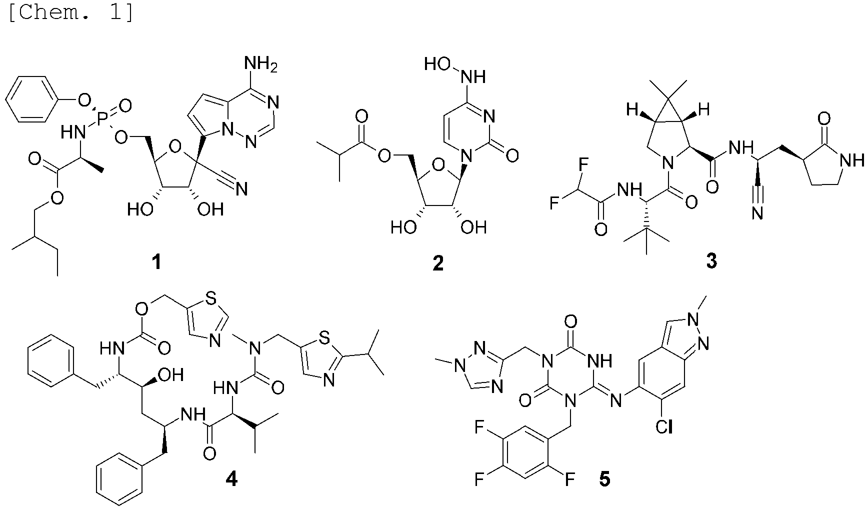

Remdesivir and Molnupiravir are viral RNA-dependent RNA polymerase inhibitors, while Nirmatrelvir and Encochlebit are also viral 3C-like protease inhibitors (Non-patent document 3).

### CITATION LIST

### NON-PATENT DOCUMENTS

Non-Patent Document 1: "Daily confirmed COVID-19 cases and deaths, World". Published online at OurWorldInData.org. Retrieved from: 'https://ourworldindata.org/grapher/daily-covid-cases-deaths' [Online Resource]
Non-Patent Document 2: Zoeo L. Grange, Tracey Goldstein, Christine K. Johnson and Jonna A. K. Mazet, "Ranking the risk of animal-to-human spillover for newly discovered viruses" Proc. Natl. Acad. Sci. 2021, 118, e2002324118; 'https://doi.org/10.1073/pnas.2002324118'
Non-Patent Document 3: Renata Esposito, Davida Mirra, Liberata Sportiello, Giuseppe Spaziano, D'Agostino Bruno, "Overview of Antiviral Drug Therapy for COVID-19: Where Do We Stand?", Biomedicines 2022, 10, 2815; 'https://doi.org/10.3390/biomedicines10112815'

### SUMMARY OF THE INVENTION

### PROBLEMS TO BE SOLVED BY THE INVENTION

Any of the aforementioned low molecular weight drugs are inhibitors of viral proteins, and the emergence of resistant strains is anticipated. Therefore, the development of candidate for pharmaceutical compounds with different mechanisms of action and molecular structures from those drugs is desired.

### MEANS TO SOLVE PROBLEMS

The inventors have newly explored a group of tricyclic heterocyclic compounds that have a completely different molecular structure from the aforementioned low molecular weight drugs for the treatment of COVID-19, and discovered among them a compound that exhibits a powerful antiviral activity of an IC50 = 1.7 µM against the Delta variant of SARS-CoV-2at and a selectivity of over 235 times in terms of cytotoxicity/antiviral activity (CC50/IC50). The compounds of the present invention for treatment and/or prevention of novel coronavirus can be used as pharmaceutical drugs for the treatment and/or prevention of COVID-19 that are safe and highly active. Further, due to having a unique molecular structure not seen in past reports, there is a high likelihood that the effectiveness will not be lost even if resistant strains to existing drugs emerge.

Therefore, the present invention encompasses the aspects as follows.

### <Compounds>

[1] A compound represented by formula (I), its enantiomer, or a pharmaceutically acceptable salt thereof: 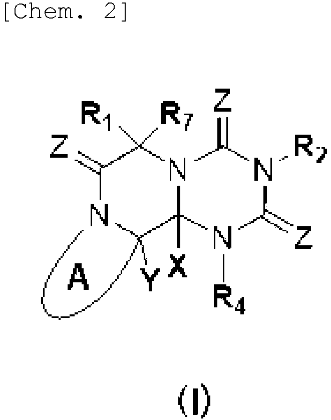 wherein
   ring A is a moiety represented by 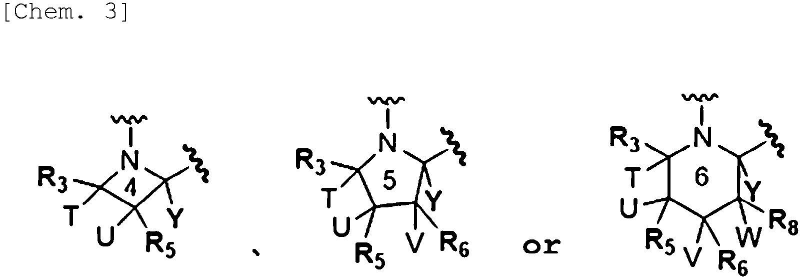
   R1 and R7 are each independently a hydrogen, an optionally substituted C1-6 alkyl, an optionally substituted C6-10 aryl, or an optionally substituted C5-10 heteroaryl, or R1 and R7 together with the carbon atom to which they are attached form a C3-6 spiro ring;
   R2 and R4 are each independently a hydrogen, an optionally substituted C1-6 alkyl group, an optionally substituted C6-10 aryl group, an optionally substituted C5-10 heteroaryl group, an optionally substituted carbonyl, an optionally substituted sulfonyl, or an optionally substituted sulfinyl;
   X is a hydrogen, a hydroxy, an optionally substituted C1-6 alkyl, or an optionally substituted C1-6 alkoxy;
   Y is a hydrogen or an optionally substituted C1-6 alkyl, or
   X and Y combine with an adjacent carbon atom to form a double bond;
   Z is independently either an oxygen or a sulfur;
   R3 is an optionally substituted C1-6 alkyl, an optionally substituted C5-10 heteroaryl, or an optionally substituted carbonyl;
   R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
   T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
   in which the substituents in "optionally substituted" are selected from the following:
      a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.
[2] The compound according to [1] represented by formula (I), its enantiomer, or a pharmaceutically acceptable salt thereof: 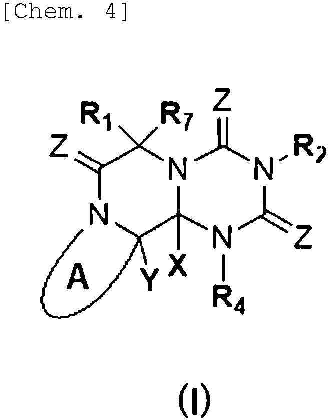 wherein
   ring A is a moiety represented by 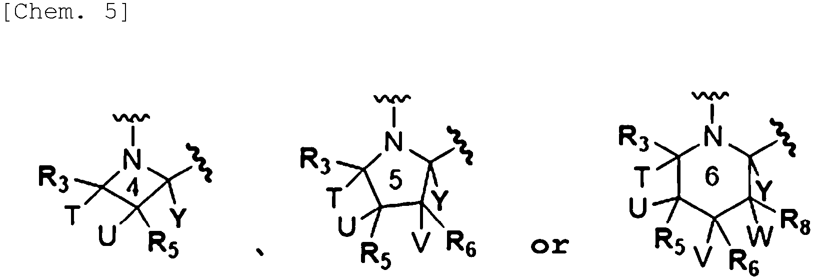
   R1 and R7 are each independently a hydrogen, an optionally substituted C1-6 alkyl, an optionally substituted C6-10 aryl, or an optionally substituted C5-10 heteroaryl, or R1 and R7 together with the carbon atom to which they are attached form a C3-6 spiro ring;
   R2 and R4 are each independently a hydrogen, an optionally substituted C1-6 alkyl group, an optionally substituted C6-10 aryl group, or an optionally substituted C5-10 heteroaryl group;
   X is a hydrogen, or a hydroxy;
   Y is a hydrogen or an optionally substituted methyl, or
   X and Y combine with an adjacent carbon atom to form a double bond;
   Z is independently either an oxygen or a sulfur;
   R3 is an optionally substituted C1-6 alkyl, an optionally substituted C5-10 heteroaryl, or an optionally substituted carbonyl;
   R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
   T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
   in which the substituents in "optionally substituted" are selected from the following:
      a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.
[3] The compound according to [1] represented by formula (II), its enantiomer, or a pharmaceutically acceptable salt thereof: 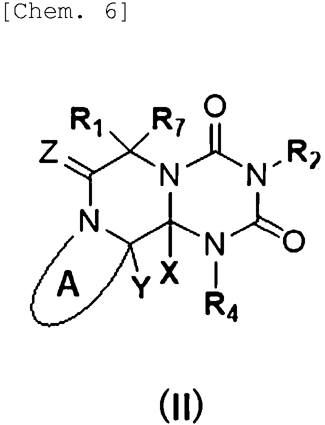 wherein
   ring A, R1 and R7, R2 and R4, X and Y, as well as Z are the same as defined above
[4] The compound according to [1] or [2] represented by formula (I), its enantiomer, or a pharmaceutically acceptable salt thereof:
   wherein
   ring A is a moiety selected from 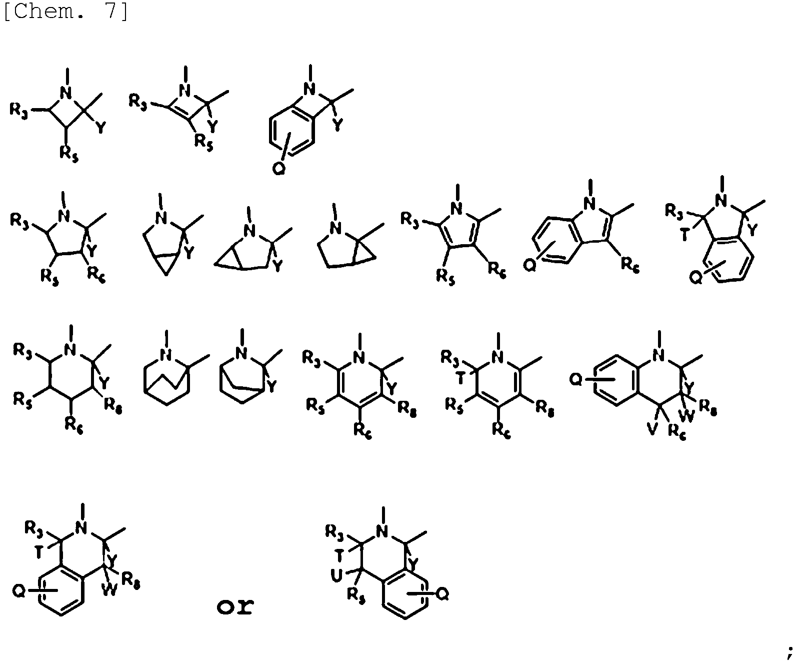 wherein
   R3, R5, R6 and R8, Y, T, U, V, as well as W are the same as defined above;
   Q is a hydrogen, a hydroxy, a halogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C5-10 heteroaryl.
[5] The compound according to [4] represented by formula (I), its enantiomer, or a pharmaceutically acceptable salt thereof:
   wherein
   ring A is a moiety selected from 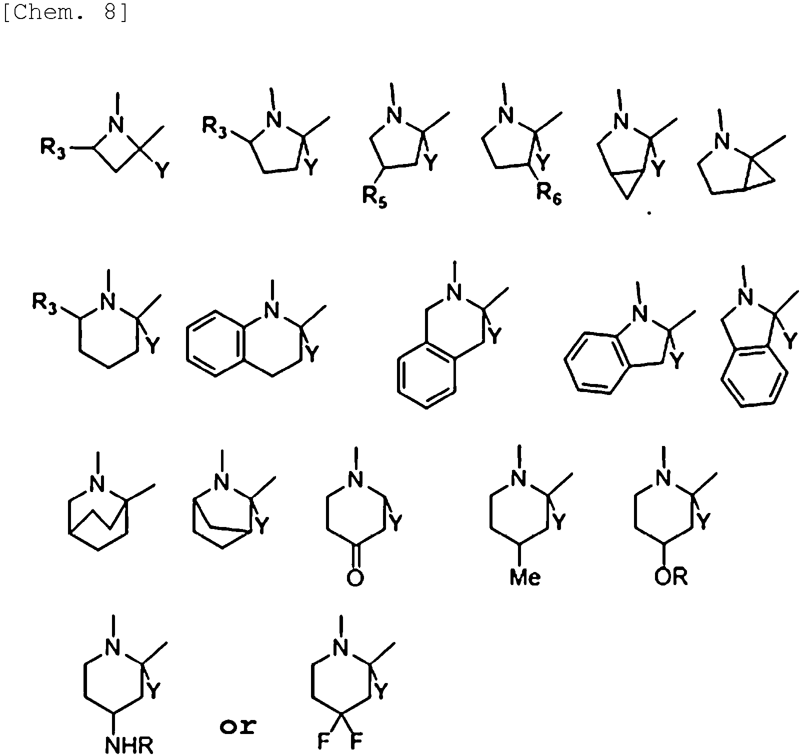 wherein
   R3, R5, R6 and Y are the same as defined above, and
   R is a hydrogen, an optionally substituted carbonyl, an optionally substituted C1-6 alkyl, an optionally substituted C6-10 aryl, or an optionally substituted C5-10 heteroaryl; and
   X is a hydroxy.
[6] The compound according to [1] represented by formula (I), its enantiomer, or a pharmaceutically acceptable salt thereof, wherein R2 and R4 are the same group.

### <Preferred compounds>

[7]
   The compound according to [1] represented by formula (III), its enantiomer, or a pharmaceutically acceptable salt thereof: 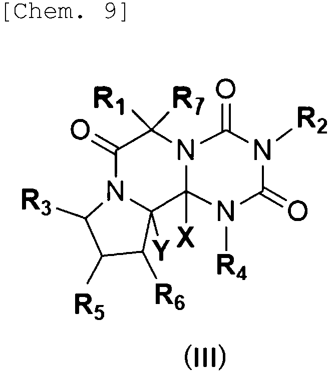 wherein
   R1 and R7 are each independently a hydrogen, an optionally substituted C1-6 alkyl, an optionally substituted C6-10 aryl, or an optionally substituted C5-10 heteroaryl, or R1 and R7together with the carbon atom to which they are attached form a C3-6 spiro ring;
   R2 and R4 are each independently an optionally substituted C1-6 alkyl group, an optionally substituted C6-10 aryl group, or an optionally substituted C5-10 heteroaryl group;
   R3 is an optionally substituted C1-6 alkyl, an optionally substituted C5-10 heteroaryl, or an optionally substituted carbonyl;
   R5, and R6 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
   X is a hydroxy;
   Y is a hydrogen or an optionally substituted methyl, or
   X and Y combine with an adjacent carbon atom to form a double bond;
   in which the substituents in "optionally substituted" are selected from the following:
      a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.
[8]
   The compound according to [7], its enantiomer, or a pharmaceutically acceptable salt thereof:
   wherein
   either R1 or R7 is a hydrogen, and the other is a group represented by the formula: 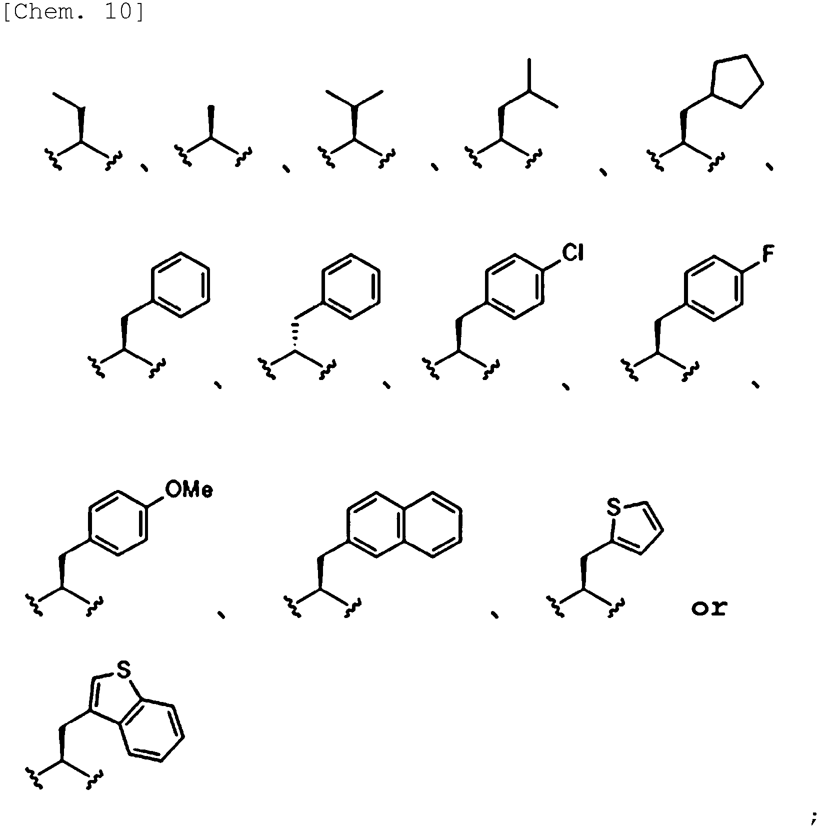 or
   R1 and R7 combine together to form a spiro ring represented by the formula: 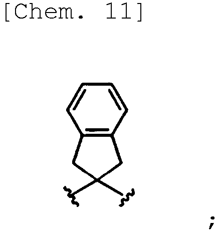
   the triazacyclohexane ring by which R2, R4, and X are substituted, is selected from the groups represented by the formula: 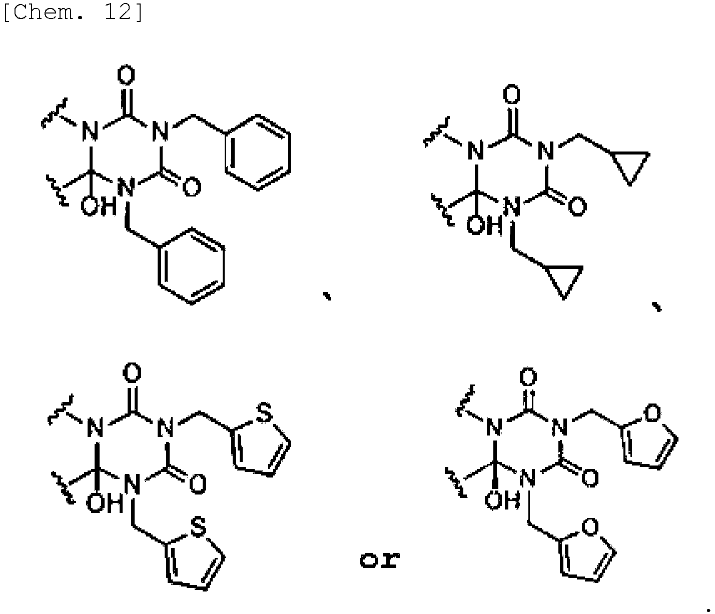
[9]
   The compound according to [7], its enantiomer, or a pharmaceutically acceptable salt thereof, wherein X and Y combine together with an adjacent carbon to form a double bond.
[10]
   The compound according to [9], its enantiomer, or a pharmaceutically acceptable salt thereof, wherein the compound is represented by either: 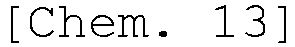 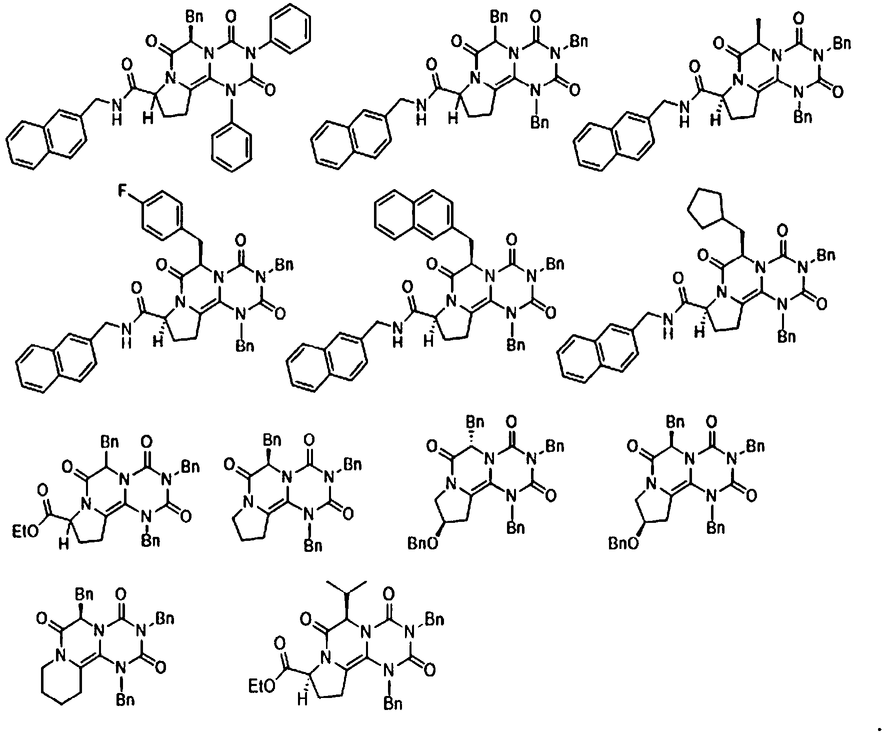
[11]
   The compound according to [7], its enantiomer, or a pharmaceutically acceptable salt thereof:
   wherein
   R3 is a group selected from: 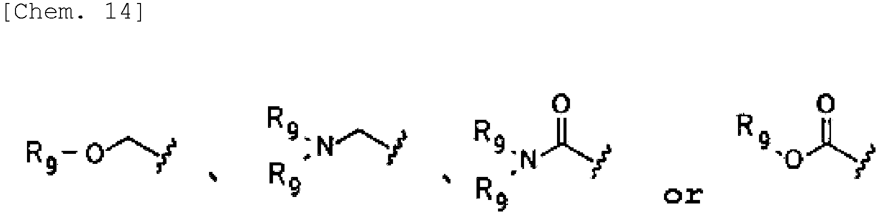 wherein
   R9 is independently a hydrogen, an optionally substituted C1-6 alkyl, an optionally substituted C6-10 aryl, an optionally substituted C5-10 heteroaryl, or a protecting group, or
   when R9 appears twice, they crosslink together with the nitrogen to which they are attached to form an optionally substituted 3- to 6-membered ring.
[11-2]
   The compound according to [11-2], its enantiomer, or a pharmaceutically acceptable salt thereof:
   wherein
   R3 is a group represented by: 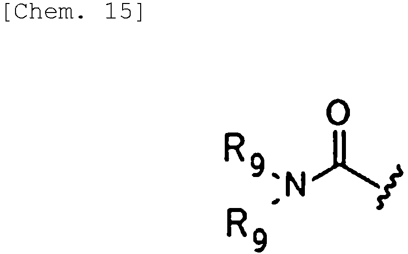 wherein
   R9s crosslink together with the nitrogen to which they are attached to form an optionally substituted 5-membered ring.
[11-3]
   The compound according to [11-2], its enantiomer, or a pharmaceutically acceptable salt thereof:
   wherein
   the group represented by: 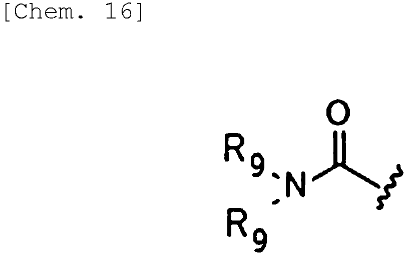 is an optionally substituted 5-membered ring, and is a group represented by: 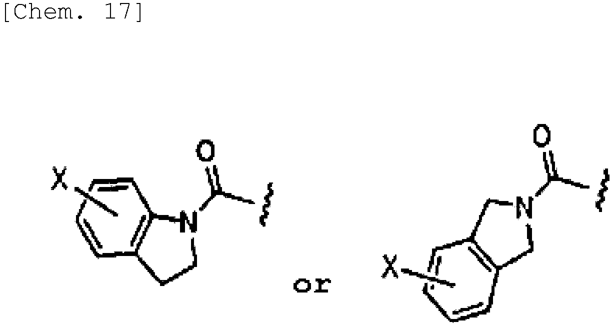 wherein
   X is a hydrogen, a hydroxy, a halogen, a cyano, a carbamoyl, an optionally substituted C1-6 alkyl, an optionally substituted C1-6 alkoxy, or an optionally substituted C1-6 alkylamino.
[12]
   The compound according to [11], its enantiomer, or a pharmaceutically acceptable salt thereof:
   wherein
   R3 is a group selected from: 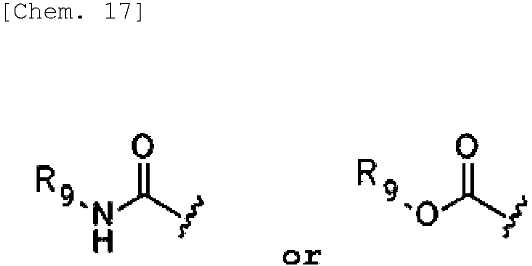 wherein
   R9 is independently a hydrogen, an optionally substituted C1-6 alkyl groups, an optionally substituted C6-10 aryl groups, an optionally substituted C5-10 heteroaryl groups, or a protecting group.
[13]
   The compound according to [12], its enantiomer, or a pharmaceutically acceptable salt thereof:
   wherein
   R3 is a group represented by: 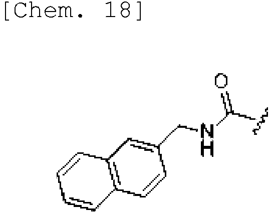
[14]
   The compound according to [7], its enantiomer, or a pharmaceutically acceptable salt thereof, wherein R2 and R4 are the same group.
[15]
   The compound according to [7], its enantiomer, or a pharmaceutically acceptable salt thereof, wherein X and Y combine together with an adjacent carbon atom to form a double bond.

### <Pharmaceutical compositions>

[16]
   A pharmaceutical composition which comprises the compound according to any one of [1] to [15], its enantiomer, or a pharmaceutically acceptable salt thereof.
[17]
   The pharmaceutical composition according to [16 for the treatment or prevention of SARS-CoV-2 novel coronavirus infection.

### EFFECT OF INVENTION

According to the present invention, it is possible to effectively treat or prevent the SARS-CoV-2 novel coronavirus infection.

### ASPECTS FOR CARRYING OUT INVENTION

In an aspect, the present invention relates to a compound represented by formula (I), its enantiomer, or a pharmaceutically acceptable salt thereof: 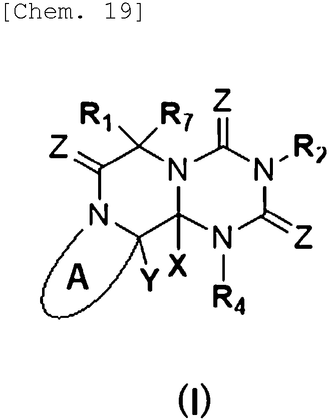 wherein
ring A is a moiety represented by 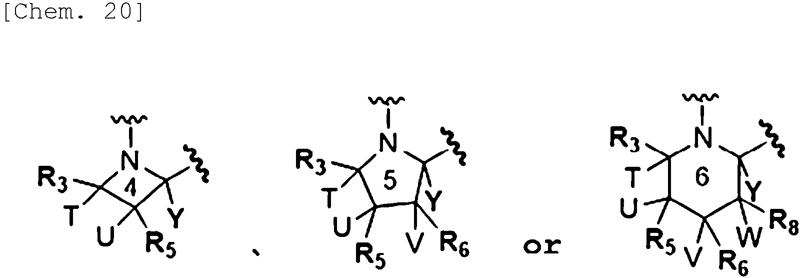
R1 and R7 are each independently a hydrogen, an optionally substituted C1-6 alkyl, an optionally substituted C6-10 aryl, or an optionally substituted C5-10 heteroaryl, or R1 and R7together with the carbon atom to which they are attached form a C3-6 spiro ring;
R2 and R4 are each independently a hydrogen, an optionally substituted C1-6 alkyl group, an optionally substituted C6-10 aryl group, an optionally substituted C5-10 heteroaryl group, an optionally substituted carbonyl, an optionally substituted sulfonyl, or an optionally substituted sulfinyl;
X is a hydrogen, a hydroxy, an optionally substituted C1-6 alkyl, or an optionally substituted C1-6 alkoxy;
Y is a hydrogen or an optionally substituted C1-6 alkyl, or
X and Y combine with an adjacent carbon atom to form a double bond;
Z is independently either an oxygen or a sulfur;
R3 is an optionally substituted C1-6 alkyl, an optionally substituted C5-10 heteroaryl, or an optionally substituted carbonyl;
R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
in which the substituents in "optionally substituted" are selected from the following:
   a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.

In this aspect, the preferred compounds of the present invention include those represented by formula (II), their enantiomers, or pharmaceutically acceptable salts thereof: 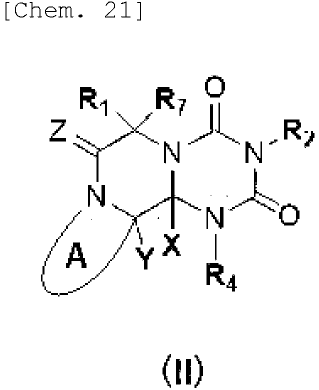 wherein
ring A, R1 and R7, R2 and R4, X and Y, as well as Z are the same as defined above.

In this aspect, the preferred compounds of the present invention are the those wherein A is a moiety selected from 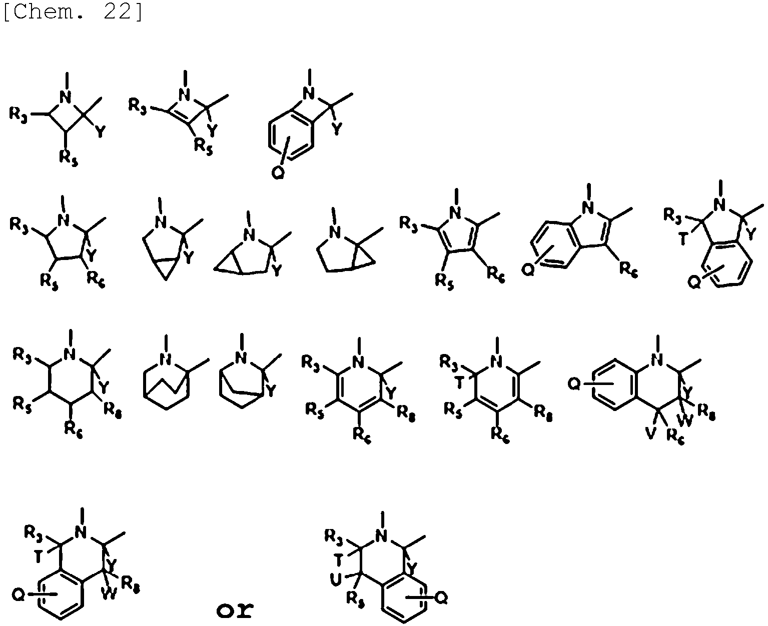 wherein
R3, R5, R6 and R8, Y, T, U, V, as well as W are the same as defined above;
Q is a hydrogen, a hydroxy, a halogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C5-10 heteroaryl;
their enantiomers, or pharmaceutically acceptable salts thereof.

In this aspect, the further preferred compounds of the present invention are the those wherein A is a moiety selected from 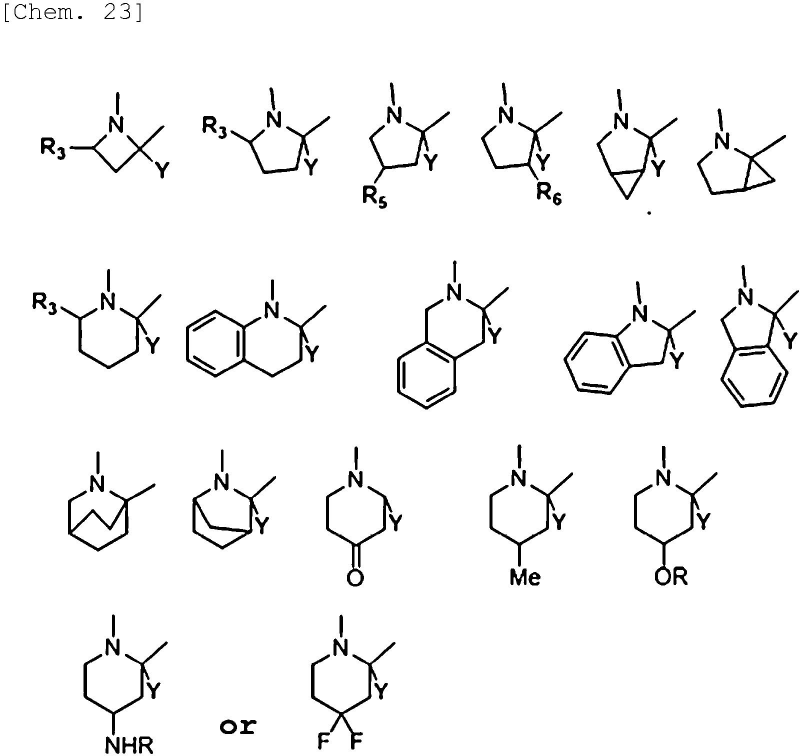 wherein
R3, R5, R6 and Y are the same as defined above, and
R is a hydrogen, an optionally substituted carbonyl, an optionally substituted C1-6 alkyl, an optionally substituted C6-10 aryl, or an optionally substituted C5-10 heteroaryl; and
X is a hydroxy;
their enantiomers, or pharmaceutically acceptable salts thereof.

In this aspect, the further preferred compounds of the present invention are the compounds of the invention, their enantiomers, or pharmaceutically acceptable salts thereof, wherein R2 and R4 are the same group.

The specific compounds represented by formula (I) include a compound represented by any one of:

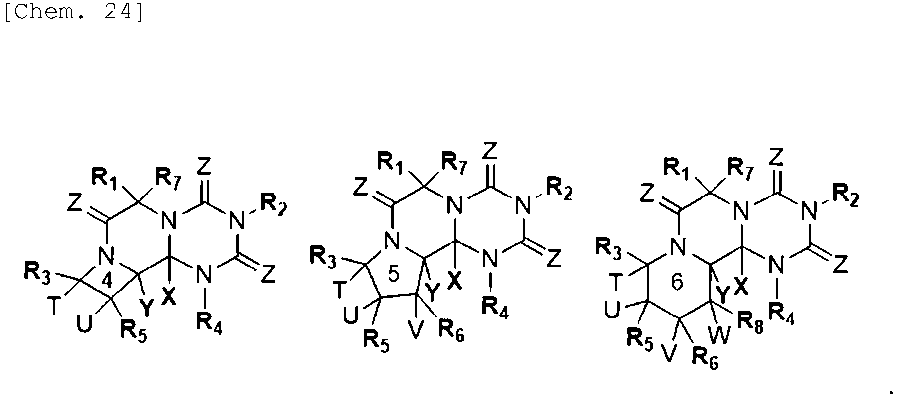

In this aspect, the preferred compounds include the compounds represented by formula (III), their enantiomers, or pharmaceutically acceptable salts thereof: 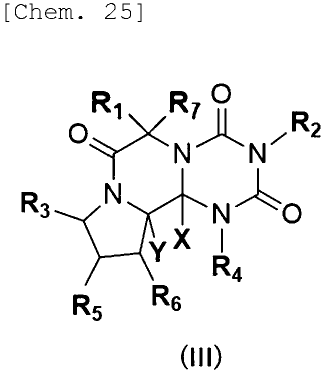 wherein
R1 and R7 are each independently a hydrogen, an optionally substituted C1-6 alkyl, an optionally substituted C6-10 aryl, or an optionally substituted C5-10 heteroaryl, or R1 and R7 together with the carbon atom to which they are attached form a C3-6 spiro ring;
R2 and R4 are each independently an optionally substituted C1-6 alkyl group, an optionally substituted C6-10 aryl group, or an optionally substituted C5-10 heteroaryl group;
R3 is an optionally substituted C1-6 alkyl, an optionally substituted C5-10 heteroaryl, or an optionally substituted carbonyl;
R5, and R6 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
X is a hydroxy;
Y is a hydrogen or an optionally substituted methyl, or
X and Y combine with an adjacent carbon atom to form a double bond;
in which the substituents in "optionally substituted" are selected from the following:
   a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.

In this aspect, the further preferred compounds of the present invention include the compounds represented by formula (III), their enantiomers, or pharmaceutically acceptable salts thereof:
wherein
either R1 or R7 is a hydrogen, and the other is a group represented by the formula: 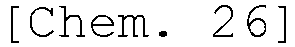 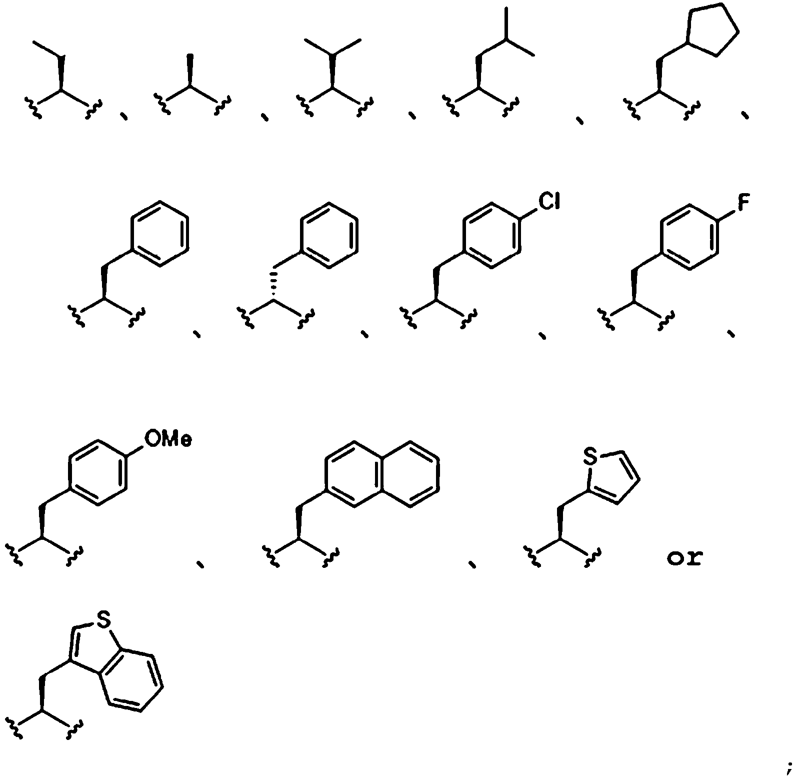 or
R1 and R7 combine together to form a spiro ring represented by the formula: 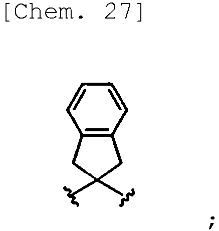
the triazacyclohexane ring by which R2, R4, and X are substituted, is selected from the groups represented by the formula:

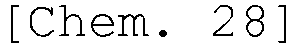

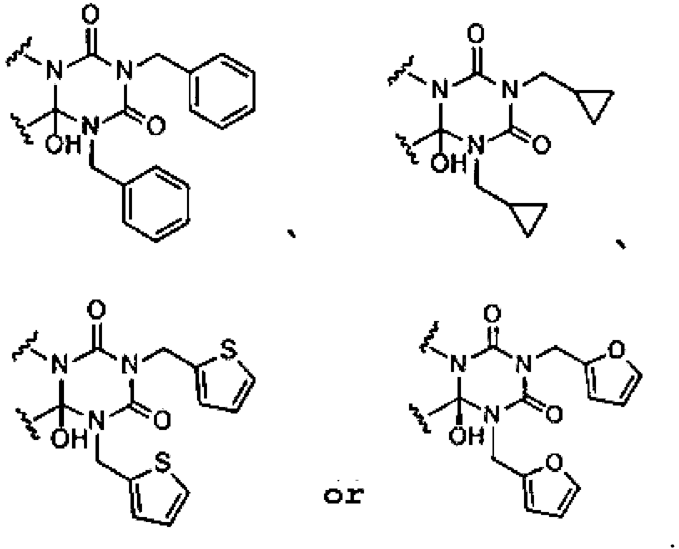

In this aspect, the further preferred compounds of the present invention are represented by the formula: 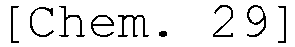 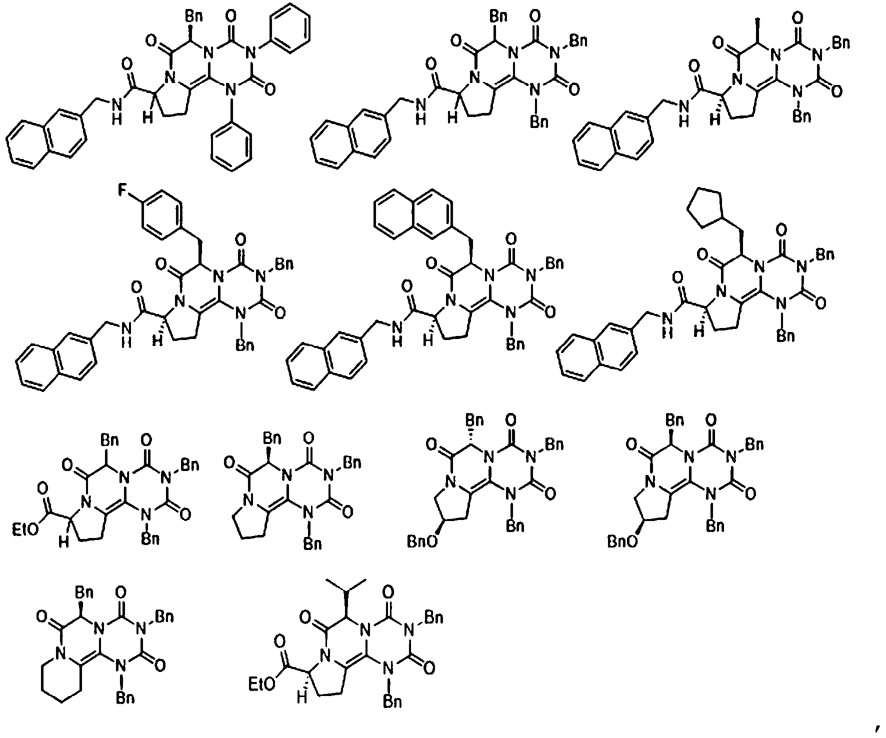 its enantiomer, or its pharmaceutically acceptable salt.

In this aspect, another embodiment of the present invention include the compounds according to formula (III), its enantiomer, or a pharmaceutically acceptable salt thereof wherein R3 is a group selected from: 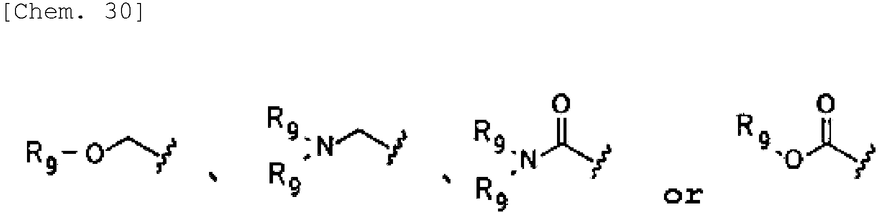 wherein
R9 is independently a hydrogen, an optionally substituted C1-6 alkyl, an optionally substituted C6-10 aryl, an optionally substituted C5-10 heteroaryl, or a protecting group, or
when R9 appears twice, they crosslink together with the nitrogen to which they are attached to form an optionally substituted 3- to 6-membered ring. Specific embodiments include the compound, its enantiomer, or a pharmaceutically acceptable salt thereof:
   wherein
   R3 is a group represented by: 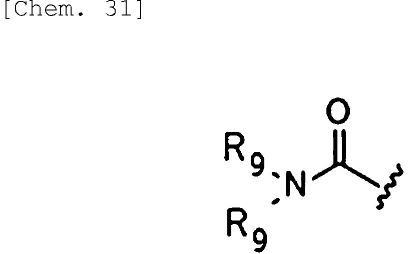 wherein
   R9s crosslink together with the nitrogen to which they are attached to form an optionally substituted 5-membered ring, preferably a group represented by: 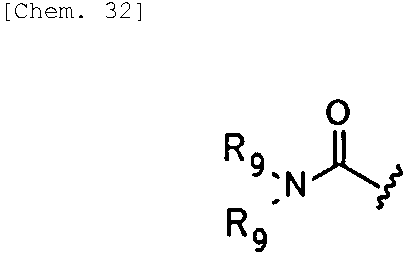 is an optionally substituted 5-membered ring, and is a group represented by: 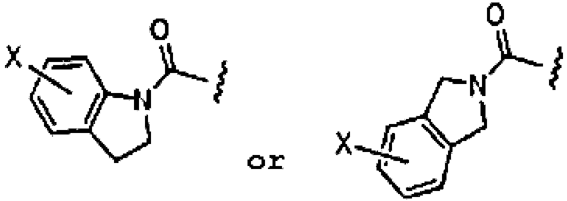 wherein
   X is a hydrogen, a hydroxy, a halogen, a cyano, a carbamoyl, an optionally substituted C1-6 alkyl, an optionally substituted C1-6 alkoxy, or an optionally substituted C1-6 alkylamino.

Preferably, the compounds include the compound, its enantiomer, or a pharmaceutically acceptable salt thereof: wherein
R3 is a group selected from: 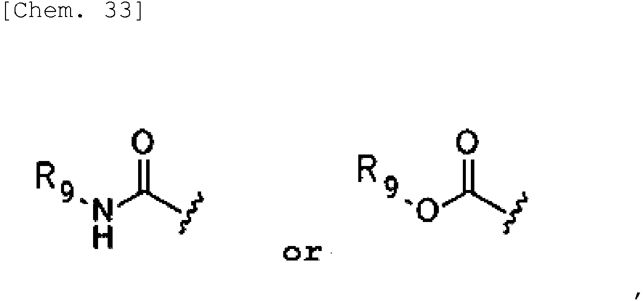 and further preferably, a group represented by:

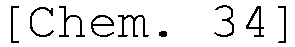

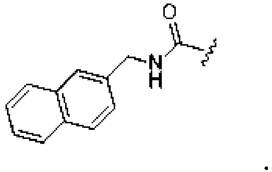

In this aspect, the preferred compounds of the present invention include those in which R2 and R4 are the same group, in formulas (I), (II), and (III) of the present invention, as well as their enantiomers, or their pharmaceutically acceptable salts.

In this aspect, the preferred compounds of the present invention include those in which X and Y, together with adjacent carbon atoms, form a double bond, in formulas (I), (II), and (III) of the present invention, as well as their enantiomers, or their pharmaceutically acceptable salts.

The terms as used herein are employed in their commonly accepted meanings in the art, unless otherwise specified. Therefore, all technical and scientific terms as used herein shall have the same meaning as generally understood by those skilled in the art to which the invention pertains, unless otherwise defined.

### <Definitions>

As used herein, the term "group" means a monovalent group unless otherwise specified. An example that is not a monovalent group include an alkylene group (divalent), and so on. Additionally, in the descriptions of the substituents listed below, the term "group" may be omitted.

As used herein, the number of substituents as defined in "optionally substituted " or "substituted" is not particularly limited in cases where there are no specific restrictions is one or more when substitution is possible. Further, unless specifically instructed otherwise, the description of each substituent also applies to a part or another substituent related to each substituent.

As used herein, with respect to a group modified by "optionally substituted" or "substituted", any part of the group may be substituted. For example, "optionally substituted aryl alkyl" and "substituted aryl alkyl" may be substituted at the aryl part, or the alkyl part, or at both of the parts.

As used herein, "C1-6" means that the number of carbon atoms is from 1 to 6. The same applies to other numbers; for example, "C1-4" means that the number of carbon atoms is from 1 to 4, while "C1-3" means that the number of carbon atoms is from 1 to 3.

As used herein, "heteroatom" refers to atoms other than carbon atoms and hydrogen atoms, such as oxygen atoms, nitrogen atoms, sulfur atoms, etc.

As used herein, "hydroxy" refers to a monovalent -OH group. This group may be referred to as the "hydroxy group" or "hydroxyl".

As used herein, "halogen" refers to an atom belonging to the halogen group, including fluorine atom, a chlorine atom, a bromine atom, or an iodine atom. Preferably, it is a fluorine atom or a chlorine atom. More preferably, it is a fluorine atom. The term "halogen" may be referred to as "halogen atom" or simply "halogen".

As used herein, "carboxy" refers to a monovalent radical -COOH. This group may be referred to as "carboxy group", "carboxy", "carboxyl", or "carboxylic acid group".

As used herein, "cyano" refers to a monovalent radical -CN.

As used herein, "amino" refers to a monovalent radical -NH2. This group may be referred to as "amino group".

As used herein, "alkyl" refers to a straight-chain or branched-chain saturated aliphatic hydrocarbon group. "C1-6 alkyl" refers to an alkyl group with a carbon atom count ranging from 1 to 6, with "C1-4 alkyl" being a preferred example, more preferably "C1-3 alkyl", and even more preferably "C1-2 alkyl". Specific examples of "C1-4 alkyl" include, but are not limited to, methyl, ethyl, propyl, isopropyl, n-butyl, isobutyl, tert-butyl, and sec-butyl. Examples of C1-6 alkyl include C1-4 alkyl, n-pentyl, isopentyl, neopentyl, tert-pentyl, 1,2-dimethylpropyl, and n-hexyl.

As used herein, "alkenyl" refers to a straight-chain or branched-chain aliphatic hydrocarbon group that has a double bond. "C2-6 alkenyl" is an alkenyl group with a carbon atom count of 2 to 6, with preferred examples being the "C2-4 alkenyl" and more preferably the "C2-3 alkenyl". Specific examples of "C2-4 alkenyl" include, but are not limited to, vinyl, propenyl, isopropenyl, n-butyl, isobutyl, and sec-butyl. Examples of C2-6 alkenyl include C2-4 alkenyl, n-pentenyl, isopentenyl, neopentenyl, 1,2-dimethylpropenyl, and n-hexenyl.

As used herein, "alkynyl" refers to a straight-chain or branched-chain aliphatic hydrocarbon group that has a triple bond. "C2-6 alkynyl" refers to alkynyl groups with a carbon atom count ranging from 2 to 6, with preferred examples being the "C2-4 alkynyl" and more preferably the "C2-3 alkynyl. Specific examples of "C2-4 alkynyl" include, but are not limited to, ethynyl, propynyl, isopropynyl, n-butylnyl, isobutylnyl, and sec-butylnyl. Examples of C2-6 alkynyl include C2-4 alkynyl, n-pentynyl, isopentynyl, neopentynyl, 1,2-dimethylpropynyl, and n-hexynyl.

As used herein, "aryl" refers to a monovalent group of a monocyclic or bicyclic aromatic hydrocarbon ring, and "C6-10 aryl" refers to an aryl group with a carbon atom count of 6 to 10. Specific examples of "aryl" include, but are not limited to, C6 aryl, C10 aryl, and so on. Specific examples of C6 aryl group include, but are not limited to, phenyl an so on. Specific examples of C10 aryl include, but are not limited to, 1-naphthyl, 2-naphthyl, and so on.

As used herein, "heteroaryl" means a monovalent group of a monocyclic or bicyclic aromatic heterocycle that contains one to four heteroatoms, which are the same or different, selected from the group consisting of an oxygen atom, a nitrogen atom, and a sulfur atom.

As used herein, "C5-10 heteroaryl" refers to a monovalent group of a monocyclic or bicyclic aromatic heterocycle consisting of 5 to 10 atoms, which contains one to four heteroatoms selected from the group consisting of an oxygen atom, a nitrogen atom, and a sulfur atom, which may be the same or different. Specific examples of "C5-10 heteroaryl" include, but are not limited to, pyrrolidinyl, imidazole, oxadiazole, triazole, pyridyl, pyridazinyl, pyrimidinyl, pyrazinyl, quinolyl, isoquinolyl, naphthyridinyl, quinoxalinyl, sinapyl, quinazolinyl, phthalazinyl, imidazopyridyl, imidazothiazolyl, imidazooxazolyl, benzothiazolyl, benzoxazolyl, benzimidazolyl, indolyl, isoindolyl, indazolyl, pyrrolopyridyl, thienopyridyl, flupyridyl, benzothiazolyl, benzoxadiazolyl, pyridopyrimidinyl, benzofuryl, benzothienyl, benz[1,3]dioxole, thienofuryl, chromenyl, chromanil, coumarinyl, quinolinyl, and so on.

As used herein, "alkoxy" refers to a monovalent radical of -O-alkyl. Preferred examples of alkoxy include a C1-6 alkoxy, i.e., a C1-6 alkyl-O-, a C1-4 alkoxy, i.e., a C1-4 alkyl-O-, and so on. Specific examples of "C1-4 alkoxy" include, but are not limited to, methoxy (CH₃O-), ethoxy (CH₃CH₂O-), n-propoxy (CH₃(CH₂)₂O-), isopropoxy ((CH₃)₂CHO-), n-butoxy (CH₃(CH₂)₃O-), isobutoxy ((CH₃)₂CHCH₂O-), tert-butoxy ((CH₃)₃CO-), and sec-butoxy (CH₃CH₂CH(CH₃)O-) , and so on. Specific examples of "C1-6 alkoxy" include, but are not limited to, C1-4 alkoxy, n-pentoxy (CH₃(CH₂)₄O-), isopentoxy ((CH₃)₂CHCH₂CH₂O-), neopentoxy ((CH₃)₃CCH₂O-), tert-pentoxy (CH₃CH₂C(CH₃)₂O-), and 1,2-dimethylpropoxy (CH₃CH(CH₃)CH(CH₃)O-), and so on.

As used herein, " optionally substituted carbonyl" refers to "a substituted carbonyl" and "a carbonyl that is substituted", while "non-substituted carbonyl" means a carboxylic acid group. The term "substituted carbonyl" encompasses an ester or an amide of carboxylic acids, a hydrocarbonyl group, a optionally substituted lower alkyl carbonyl group, a optionally substituted aryl carbonyl group, and so on.

For example, "alkyl carbonyl" is exemplified, which is a monovalent group of "-C(=O)-alkyl. Preferred example of an alkyl carbonyl includes a C1-6 alkyl carbonyl. Specific examples of " C1-6 alkyl carbonyl" include, but are not limited to, acetyl(CH₃C(=O)-), n-propanoyl (CH₃CH₂C(=O)-), n-butanoyl (CH₃CH₂CH₂C(=O)-), n-pentanoyl (CH₃(CH₂)₃C(=O)-), n-hexanoyl (CH₃(CH₂)₄C(=O)-), and n-heptanoyl (CH₃(CH₂)₅ C(=O)-), and so on.

Further, "alkoxycarbonyl" is exemplified, which is a monovalent group of -C(=O)-O-alkyl. Specific examples of " alkoxycarbonyl" include, but are not limited to, C1-6 alkoxycarbonyl, preferably C1-4 alkoxycarbonyl, and so on. Specific examples of "C1-4 alkoxycarbonyl" include methoxycarbonyl, ethoxycarbonyl, n-propoxycarbonyl, isopropoxycarbonyl, n-butoxycarbonyl, sec-butoxycarbonyl, tert-butoxycarbonyl, and isobutoxycarbonyl. Specific examples of C1-6 alkoxycarbonyls include, but are not limited to, C1-4 alkoxycarbonyl, n-pentoxycarbonyl, isopentoxycarbonyl, neopentoxycarbonyl, tert-pentoxycarbonyl, 1,2-dimethylpropoxycarbonyl, n-hexoxycarbonyl, and so on.

As used herein, "aryl carbonyl" refers to a monovalent group of -C(=O)-aryl. Preferred examples of an aryl carbonyl include a C6-10 aryl carbonyl. Specific examples of "C6-10 aryl carbonyl" include, but are not limited to, benzoyl (i.e., phenyl-C(=O)-), 1-naphthyl carbonyl, and 2-naphthyl carbonyl, and so on.

The amides contained in the "substituted carbonyl" include all of the substituted amides represented by R10R11NC=O, which includes H₂NC=O in which R10 and R11 are each independently a optionally substituted C1-6 alkyl, a optionally substituted C6-10 aryl, or a optionally substituted C5-10 heteroaryl. Further, the amide also includes an imide structure such as R12(C=O)NH(C=O), which includes an acyl group as a substituent, wherein R12 is a optionally substituted C1-6 alkyl, a optionally substituted C6-10 aryl, or a optionally substituted C5-10 heteroaryl.

As used herein, "optionally substituted sulfonyl" refers to "substituted sulfonyl" and "sulfonyl that may be substituted. The "substituted sulfonyl" refers to an ester of a sulfonic acid group or its amide, a hydrosulfonyl group, an optionally substituted lower alkyl sulfonyl group, an optionally substituted aryl sulfonyl group, and so on.

For example, "C1-6 alkyl sulfonyl" is exemplified, which refers to a sulfonyl group substituted with the aforementioned "C1-6 alkyl". Preferred "C1-6 alkyl sulfonyl" is "C1-4 alkyl sulfonyl". Specific examples of " C1-6 alkyl sulfonyl" include, but are not limited to, methyl sulfonyl, propyl sulfonyl, butyl sulfonyl, and so on.

As used herein, "optionally substituted sulfinyl" refer to "substituted sulfinyl" and "sulfinyl that is substituted". "Substituted sulfinyl" refers to an ester or amide of sulfinic acid, a hydrosulfinyl group, an optionally substituted lower alkyl sulfinyl groups, an optionally substituted aryl sulfinyl group, and so on.

"Protective group" refers to a group of atoms that, when bonded to a reactive functional group within a molecule, either shields, reduces, or prevents the reactivity of the functional group. Typically, the protective group can be selectively removed during the synthetic process if desired. Examples of the protective group may be found in Peter G. M. Wuts, "Greene's Protective Groups in Organic Synthesis", 5th Ed., John Wiley & Sons, Inc., Hoboken, New Jersey (2014) and Harrison, et.al., Compendium of Synthetic Organic Methods, vols 1-8, John Wiley & Sons, NY, and so on. As used herein, the "protective group" may correspond to the substituent *α* definition. Typical examples of nitrogen protecting groups include, but not limited to, formyl, acetyl, trifluoroacetyl, benzyl, benzyl oxycarbonyl ("CBZ"), tert-butoxycarbonyl ("Boc"), trimethylsilyl ("TMS"), 2-trimethylsilylethanesulfonyl ("TES"), trityl and substituted trityl groups, allyloxycarbonyl, 9-fluorenylmethyl oxycarbonyl ("FMOC"), and nitroveratryloxycarbonyl ("NVOC"), and so on. Representative examples of hydroxyl protecting groups include, but not limited to, hydroxyl groups acylated (esterified) or alkylated, including, for example, benzyl and triethyl ether, as well as alkyl ethers, tetrahydropyranyl ethers, trialkylsilyl ethers (for example, TMS, triethylsilyl, t-butyldimethylsilyl (TBDMS), triisopropylsilyl (TIPS)), alkylaryl silyl ethers (for example, t-butyldiphenylsilyl (TBDPS)), trialkylsilyl ethers (for example, triphenylsilyl), glycol ethers (for example, ethylene glycol ethers, propylene glycol ethers, etc.), allyl ethers, and so on.

The compounds of the present invention exist as stereoisomers and optical isomers such as tautomeric forms and geometric isomers, depending on the type of substituent, and the present invention encompasses all of them, and the mixtures thereof. That is, when one or more asymmetric carbon atoms are present in the compound of the present invention, diastereomers and optical isomers exist, and the present invention encompass mixtures of these diastereomers and optical isomers, as well as those isolated individually.

As another embodiment, the present invention encompasses various hydrates, solvent hydrates, and crystal forms.

Further, in another embodiment, the present invention encompasses prodrugs corresponding to the compounds of the present invention. According to the present invention, a prodrug refers to a derivative that, through acid hydrolysis or enzymatic decomposition in the body, yields a compound represented by formula (I). For example, in case that the compound represented by formula (I) has a hydroxyl, amino, or carboxyl group, the group can be modified according to the usual methods to produce prodrugs. The technology for prodrug preparation is described in, for example, C. GWermuth, "The Practice of Medicinal Chemistry", 4th Ed., Academic Press, (2015), Chapter 28. For example, in the case of a compound having a carboxyl group, the prodrugs are exemplified by compounds in which the carboxyl group has become an alkoxycarbonyl group, a thioalkylcarbonyl group, or an alkylaminocarbonyl group. Further, for example, in the case of a compound having an amino group, the prodrugs are exemplified by compounds in which the amino group is substituted with an alkanoic acyl group to form an alkanoic acyl amino group, a compound substituted with an alkoxycarbonyl group to form an alkoxycarbonyl amino group, a compound that has become an alkanoic acyl oxy methyl amino group, or a compound that has become a hydroxylamine. Further, for example, in the case of a compound that has a hydroxyl group, the prodrugs are exemplified by compounds in which the hydroxyl group is replaced by the alkanoil group to form an alkanoiloxy group, compounds that have become phosphate esters, or compounds that have become alkanoiloxymethyloxy groups.

As used herein, "pharmaceutically acceptable salts" refers to the acid addition salts and base addition salts that are permitted for pharmaceutical use. Examples of "pharmaceutically acceptable salts" include, but are not limited to, acetate, propionate, butyrate, formate, trifluoroacetate, maleate, fumarate, tartrate, citrate, stearate, succinate, ethyl succinate, malonate, lactobionate, gluconate, glucosamine, benzoate, methanesulfonate, benzenesulfonate, para-toluenesulfonate (tosylate), lauryl sulfate, malate, ascorbate, mandelate, saccharinate, xylanoate, pamitate, cinnamate, adipate, cysteine salt, N-acetylcysteine salt, hydrochloride, hydrobromide, phosphate, sulfate, iodide, niacinate, oxalate, picrate, thiocyanate, undecanoate, acrylic acid polymer salt, carboxyvinyl polymer, and other acid addition salts; lithium salts, sodium salts, potassium salts, calcium salts, and other inorganic base addition salts; organic base addition salts such as morpholine and piperidine; and addition salts with amino acids such as aspartate and glutamate.

### <Production method of the compounds of the present invention>

The following describes a general method for the production of the compounds of the present invention, with examples, provided that the present invention is not limited to these.

The compounds of the present invention can be produced by the production method described below. These production methods can be appropriately improved based on the knowledge of those who are proficient in organic synthesis chemistry. In the production method described below, the compounds used as starting materials may be used in their salt forms, provided that they do not interfere with the reaction.

In the production method described below, the target compounds can be obtained, even if the use of protective groups is not explicitly stated, when any functional group other than the reaction site changes under the reaction conditions, or when it is unsuitable for post-reaction processing, the sites other than the reaction site should be protected as necessary, and the sites are deprotecting after the reaction is complete or after a series of reactions. The protective groups as used in these processes may include the conventional protective groups described in the literature (Peter G. M. Wuts, "Greene's Protective Groups in Organic Synthesis", 5th Ed., John Wiley & Sons, Inc., Hoboken, New Jersey (2014)). Further, the introduction and removal of the protecting groups can be carried out using methods commonly employed in organic synthesis chemistry (for example, the methods described in the aforementioned literature) or methods similar to those.

The starting materials and intermediates in the production method described below are available either by purchasing them as commercial products or by synthesizing them according to methods described in the literature or known methods from the known compounds. Further, these starting materials and intermediates may be used in their salt forms, provided that they do not interfere with the reaction.

The intermediates and target compounds in the production method described below can also be converted into other compounds encompassed in the present invention by appropriately transforming their functional groups. The conversion of the functional groups in this context can be carried out by a method commonly used in organic synthesis chemistry (for example, RC. Larock, "Comprehensive Organic Transformations", 2nd Ed., John Wiley and Sons, Inc., New York (1999)), or any methods according to the method.

The inert solvent in the production method described below refers to a solvent that does not react with the starting materials, reagents, bases, acids, catalysts, ligands, and the like as used in the reaction (hereinafter may be referred to as "starting materials used in the reaction"). Further, even if a solvent used in each process reacts with the starting materials used in the reaction, the solvent can still be used as an inert solvent as long as the desired reaction proceeds and the target compound is obtained.

The preparation of the compounds of the present invention is based on the reaction of the piperazine framework, and the subsequent formation reaction of the triazadione framework.

The starting materials carboxylate compounds are available as commercial products.

### Synthesis of the substituted tricyclic heteroring compounds

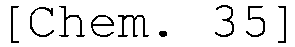

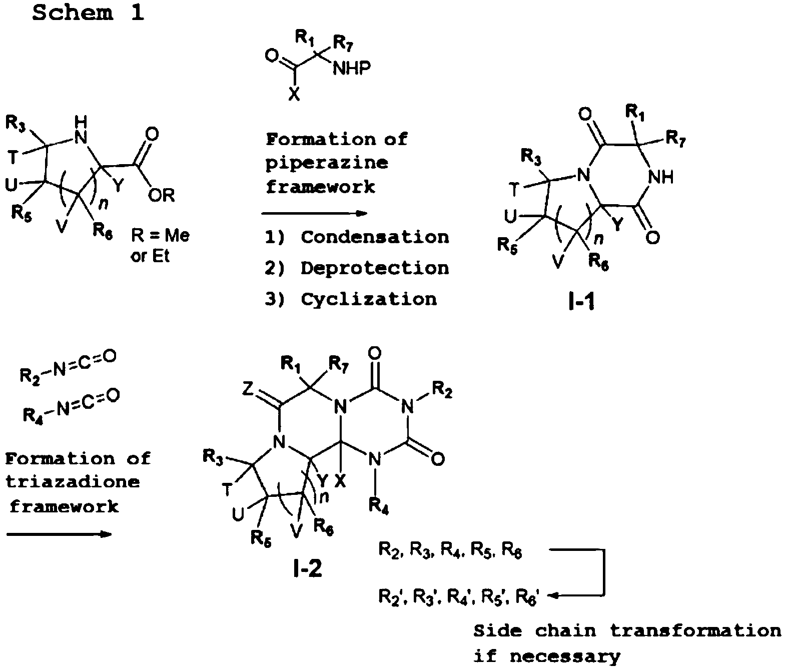

wherein
ring R1 to R7 are the same as defined above,
n is an integer of 0, 1, or 2, and
p is a protective group.

### Step 1: Formation of Piperazine Framework

Step 1 includes a condensation reaction that introduces the amino acid structure, and a deprotection of the amino group and a subsequent cyclization reaction to form the piperazine framework. The deprotection reaction is well known to those skilled in the art, and involves the deprotection of tert-butyloxycarbonyl group and the deprotection of 9-fluorenylmethyl oxycarbonyl group.

### Step 2: Formation of triazadione framework

Step 2 includes a reaction that forms a triazadione framework through the reaction with isocyanate.

### <Pharmaceutical compositions>

In an aspect, the compound of the present invention is a compound that has an antiviral activity against the SARS-CoV-2 novel coronavirus. In detail, the compound of the present invention enables the treatment or prevention of the SARS-CoV-2 novel coronavirus infections.

Therefore, in another aspect, the present invention provides a pharmaceutical composition comprising the compound of the present invention, its enantiomer, or its pharmaceutically acceptable salt, and specifically a pharmaceutical composition for the treatment or prevention of SARS-CoV-2 novel coronavirus infections.

As used herein, "treatment" means a method or process aimed at (1) delaying or preventing the onset of a disease or condition; (2) slowing down or halting the progression, aggravation or exacerbation of the onset of a disease or condition; (3) inducing remission of the onset of a disease or condition; or (4) facilitating the cure of a disease or condition. Treatment may be given as a prophylactic measure before the onset of the disease or the condition, or treatment may be given after the onset of the disease.

As used herein, "prevention" refers to the prevention of the onset of the SARS-CoV-2 novel coronavirus infection.

In the present invention, a pharmaceutical composition refers, in general, to a drug for the treatment, management, or prevention of a disease or condition, or for testing and diagnosis.

In one embodiment, the compound of the present invention can be formulated into a pharmaceutical or pharmaceutical composition for administration, either by oral or non-oral administration, using either direct administration or suitable dosage forms. Examples of these dosage forms include, but are not limited to, the tablet, the capsule, the powder, the granule, the liquid, the suspension, the injection, the patch, and the paste. Further, these formulations can be made using additives that are commonly used as pharmaceutical excipients, by known methods.

These additives can include, depending on the purpose, the excipient, the disintegrant, the binder, the flow agent, the lubricant, the coating agent, the solvent, the solubilizing agent, the thickening agent, the dispersing agent, the stabilizer, the sweetener, the flavoring agent, etc. Examples of these additives include, but are not limited to, lactose, mannitol, crystalline cellulose, low-substituted hydroxypropyl cellulose, corn starch, partially α-amylopectin, calcium carmellose, sodium carmellose, hydroxypropyl cellulose, hydroxypropyl methylcellulose, polyvinyl alcohol, magnesium stearate, sodium stearoyl fumarate, polyethylene glycol, propylene glycol, titanium dioxide, talc, and so on.

The dose of the compound of the present invention is appropriately selected based on the subject to be administered, the administration route, the disease, the age of the subject, the weight, and the symptoms. For example, in the case of oral administration, the lower limit is 0.01 mg (preferably 100 mg) and the upper limit is 10,000 mg (preferably 6,000 mg) for adults, and this amount can be administered once a day or divided into several doses.

In another aspect, the present invention relates to a method for treating or preventing SARS-CoV-2 novel coronavirus infections, which comprises administering to a subject in need of such treatment a compound of the present invention, an enantiomer thereof, or a pharmaceutically acceptable salt thereof, and preferably a method that involves administering an effective amount of the compound of the present invention, an enantiomer thereof, or a pharmaceutically acceptable salt thereof to such a subject.

Further, in another aspect, the present invention relates to compounds of the present invention for the treatment or prevention of SARS-CoV-2 novel coronavirus infections, their enantiomers or their pharmaceutically acceptable salts.

In yet another aspect, the present invention relates to the use of the compounds of the present invention, their enantiomers, or their pharmaceutically acceptable salts for the manufacture of a medicine for the treatment or prevention of SARS-CoV-2 novel coronavirus infections.

The patent documents of the patent and the patent application as well as the Non-patent document such as academic literatures referenced in the present specification are incorporated herein by reference to the same extent as if they were fully disclosed herein.

As described above, in order to facilitate the understanding of the invention, a preferred embodiment has been explained. Hereinafter, the present invention is described in further detail based on the preparation examples and examples; however, it should be noted that the above description and the following examples do not limit the scope of the present invention and are merely illustrative.

### Examples

### Preparation example 1

### (3R,6S,8aR)-3-benzyl-1,4-dioxo-octahydropyrrolo[1,2-a]pyrazine-6-carboxylate 1-1

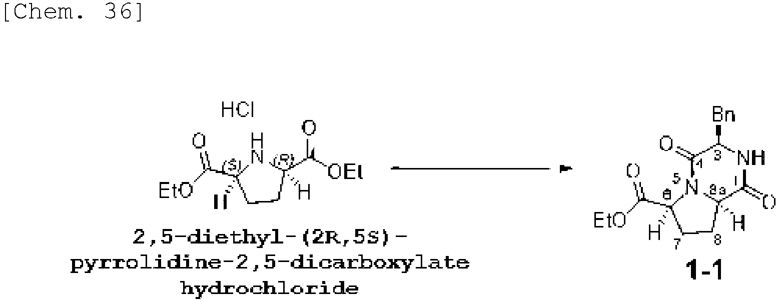

N-Fmoc-D-phenylalanine (518 mg, 1.31 mmol was added to a 25 ml egg-plant shaped flask, and was dissolved in dichloromethane (5.96 ml). Next, HATU (1-[bis(dimethylamino)methylene]-1H-1,2,3-triazolo[4,5-b]pyridinium 3-oxide hexafluorophosphate) (544 mg, 1.43 mmol) and 2,5-diethyl-(2R,5S)-pyrrolidine-2,5-dicarboxylate hydrochloride (300 mg, 1.19 mmol), followed by diisopropylethylamine (0.830 ml, 4.77 mmol) were added, and the mixture was stirred under a nitrogen atmosphere at room temperature for one and a half hours. After removing the stirrer bar and distilling off the solvent, the residue was purified using a silica gel column Q-Pack SI30 size 60 (hexane:ethyl acetate = 50:50). After distilling off the solvent, the intermediate condensate (669 mg, 1.14 mmol) was obtained. This was dissolved in dichloromethane (5.96 ml), and piperidine (0.933 ml, 9.15 mmol) was added thereto, followed by stirring the mixture at room temperature for 14 hours. After distilling off the solvent, the obtained residue was purified using a silica gel column Q-Pack SI30 size 60 (chloroform:methanol = 100:0 to 97:3). After distilling off the solvent, the desired compound 1-1 (white amorphous solid) was obtained in an amount of 313 mg (0.989 mmol, 83% after 2 steps).

### Preparation examples 2-15

According to the same reaction conditions as in Preparation Example 1, various amine raw materials were used in place of the amine raw material 2,5-diethyl-(2R,5S)-pyrrolidine-2,5-dicarboxylate hydrochloride, and various carboxylic acid raw materials were used in place of the carboxylic acid raw material N-Fmoc-D-phenylalanine, resulting in the synthesis of the 14 compounds shown in Table 1.

**[Table 1-1]**

| Table 1 | | | | | |
|---|---|---|---|---|---|
| Pre. No. | Com. No. | Chemical Structure | Amine Raw Materials | Carboxylic Acid Raw Materials | Yields (%) (After Two Steps) |
| 2 | 2-1 | 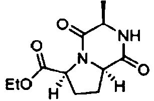 | 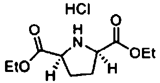 | 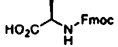 | 86 |
| 3 | 3-1 | 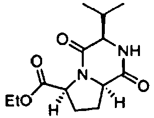 | 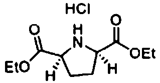 | 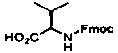 | 59 |
| 4 | 4-1 | 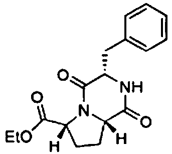 | 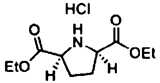 | 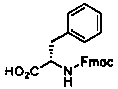 | 77 |
| 5 | 5-1 | 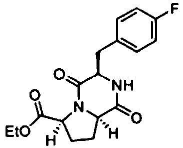 | 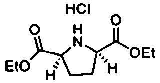 | 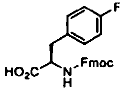 | 58 |
| 6 | 6-1 | 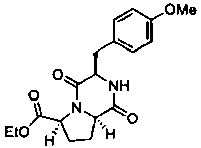 | 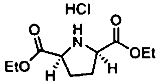 | 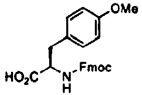 | 77 |
| 7 | 7-1 | 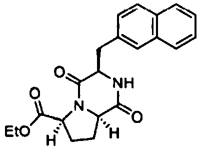 | 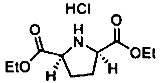 | 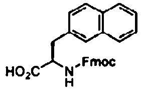 | 68 |
| 8 | 8-1 | 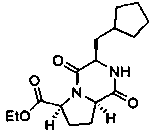 | 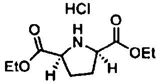 | 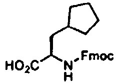 | 52 |

**[Table 1-2]**

| | | | | | |
|---|---|---|---|---|---|
| 9 | 9-1 | 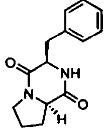 | 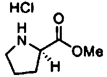 | 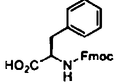 | 98 |
| 10 | 10-1 | 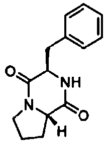 | 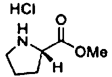 | 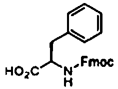 | 86 |
| 11 | 11-1 | 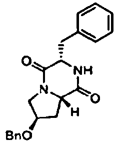 | 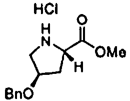 | 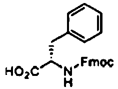 | 60 |
| 12 | 12-1 | 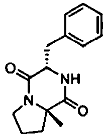 | 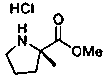 | 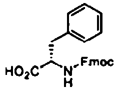 | 75 |
| 13 | 13-1 | 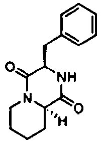 | 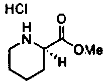 | 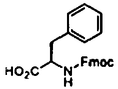 | 63 |
| 14 | 14-1 | 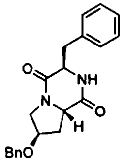 | 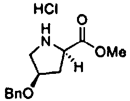 | 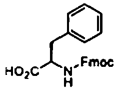 | 58 |
| 15 | 15-1 | 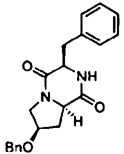 | 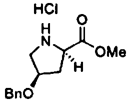 | 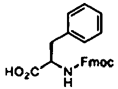 | 27 |

### Example 1

### Ethyl (6R,9S,11aR,11bR)-1,3,6-tribenzyl-11b-hydroxy-2,4,7-trioxodecahydro-2H-pyrrolo[2',1':3,4]pyrazino[1,2-a][1,3,5]triazine-9-carboxylate 1A and

### Ethyl (6R,9S)-1,3,6-tribenzyl-2,4,7-trioxo-1,3,4,6,7,9,10,11-octahydro-2H-pyrrolo[2',1':3,4]pyridazine[1,2-a][1,3,5]triazine-9-carboxylate 1B

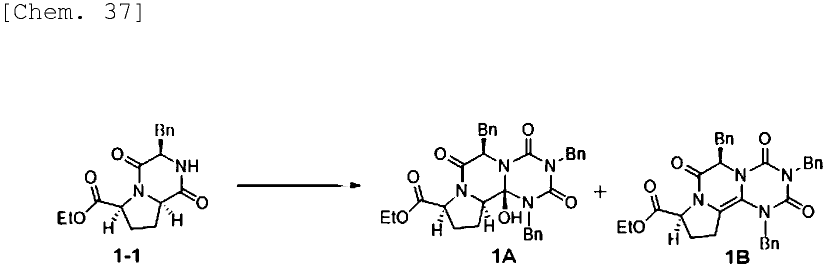

Compound 1-1 prepared in Preparation Example 1 (30.0 mg, 94.8 µmol) was added to a 10 ml egg-plant shaped flask, and was dissolved in 2.37 ml of tetrahydrofuran. Next, benzyl isocyanate (35.8 µl, 0.284 mmol) and 60% sodium hydride in oil (34.1 mg, 0.854 mmol) were added thereto, and the mixture was stirred under a nitrogen atmosphere at room temperature for 20 minutes. After the reaction was quenched with the saturated ammonium chloride aqueous solution, the mixture was extracted with ethyl acetate. After distilling off the solvent, the residue was purified using a silica gel column Q-Pack SI30 size 10 (hexane: ethyl acetate = 50:50). After distilling off the solvent, the target compound 1A (white amorphous solid) was obtained in an amount of 9.3 mg (16.0 µmol, 33%), along with its dehydrated compound 1B (white amorphous solid) in an amount of 6.1 mg (10.8 µmol, 11%).

### Examples 2-14

According to the same reaction conditions as in Example 1, the 15 compounds shown in Table 2 were synthesized by replacing the starting material, Compound 1-1, with compounds 2-1 to 15-1.

**[Table 2-1]**

| Table 2 | | | | | |
|---|---|---|---|---|---|
| Exam. No. | Com. No. | Chemical Structure | Compound Name | Mta | Yd (%) |
| 2 | 2A | 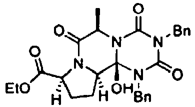 | Ethyl (6R,9S,11aR,11bR)-1,3-dibenzyl-11b-hydroxy-6-methyl-2,4,7-trioxodecahydro-2H-pyrrolo[2',1':3,4]pyrazino[1,2-a] [1.3.5]triazine-9-carboxylate | 2-1 | 39 |
| 3 | 3A | 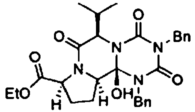 | Ethyl (6R,9S,11aR,11bR)-1,3-dibenzyl-11b-hydroxy-6-isopropyl-2,4,7-trioxodecahydro-2H-pyrrolo[2',1':3,4]pyrazino [1,2-a][1,3,5]triazine-9-carboxylate | 3-1 | 24 |
| 4 | 4A | 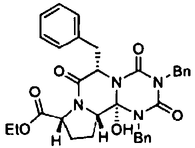 | Ethyl (6S,9R,11aS,11bS)-1,3,6-tribenzyl-11b-hydroxy-2,4.7-trioxodecahydro-2H-pyrrolo [2',1':3,4]pyrazino[1,2-a][1,3,5] triazine-9-carboxylate | 4-1 | 30 |
| 4 | 4B | 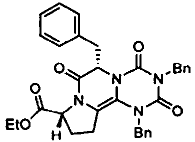 | Ethyl (6R,9S)-1,3,6-tribenzyl-2, 4,7-trioxo-1,3,4,6,7,9,10,11-octahydro-2H-pyrrolo[2',1':3,4] pyrazino[1,2-a][1,3,5]triazine-9-carboxylate | 4-1 | 10 |
| 5 | 5A | 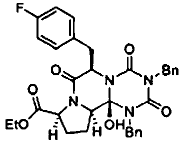 | Ethyl (6R.9S,11aR.11bR)-1.3-dibenzyl-6-(4-fluorobenzyl)-11b-hydroxy-2,4,7-trioxodecahydro-2H⁻pyrrolo[2',1':3,4]pyrazino [1,2-al [1,3,5]triazine-9-carboxylate | 5-1 | 30 |
| 6 | 6A | 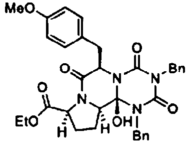 | Ethyl (6R,9S,11aR,11bR)-1,3-dibenzyl-11b-hydroxy-6-(4-methoxybenzyl)-2,4.7-trioxodecahydro-2H-pyrrolo [2',1':3,4]pyrazino[1,2-a][1,3,5] triazine-9-carboxylate | 6-1 | 28 |
| 7 | 7A | 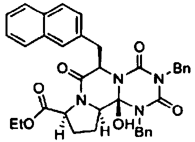 | Ethyl (6R,9S,11aR,11bR)-1,3-dibenzyl-11b-hydroxy-6-(naphthalen-2-ylmethyl)-2,4,7-trioxodecahydro-2H-pyrrolo[2',1' :3,4]pyrazino[1,2-a][1,3,5]triazin e-9-carboxylate | 7-1 | 35 |

**[Table 2-2]**

| | | | | | |
|---|---|---|---|---|---|
| 8 | 8A | 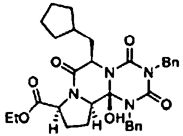 | Ethyl (6R,9S,11aR,11bR)-1,3-dibenzyl-6-(cyclopentylmethyl)-11b-hydroxy-2,4,7-trioxodecahydro-2H-pyrrolo [2',1':3,4]pyrazino[1,2-a][1,3,5] triazine-9-carboxylate | 8-1 | 43 |
| 9 | 9B | 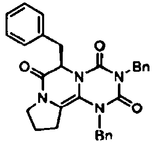 | (R) 1.3.6-Tribenzyl-1,9,10,11-tetrahydro-2H-pyrrolo[2',1':3,4] pyridazino[1,2-a][1,3,5]triazine-2,4,7(3H,6H)-trione | 9-1 | 15 |
| 10 | 11B | 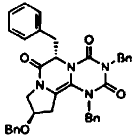 | (6S,10R)-1,3,6-Tribenzyl-10-(benzoxy)-1,9,10,11-tetrahydro-2 H-pyrrolo[2',1':3,4]pyridazino [1,2-a][1,3,5]triazine-2,4,7 (3H,6H)-trione | 11-1 | 4 |
| 11 | 12A | 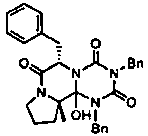 | (6S,11a8)-1,3,6-tribenzyl-11b-hydroxy-11a-methylhexahydro-2H-pyrrolo[2',1':3,4]pyrazino [1,2-a][1,3,5]triazine-2,4,7(3H,6 H)-trione | 12-1 | 25 |
| 12 | 13B | 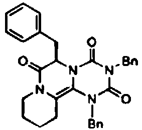 | (R) 1,3,6-Tribenzyl-9,10,11,12-tetrahydropyrido[2',1':3,4] pyrazino[1,2-a][1,3,5]triazine-2, 4,7(1H,3H,6H)-trione | 13-1 | 17 |
| 13 | 14A | 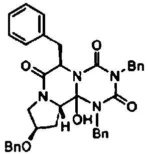 | (6R,10R.11aS)-1,3.6-tribenzyl-10-(benzyl oxy)-11b-hydroxyhexahydro-2H-pyrrolo [2',1':3,4]pyridazino[1,2-a][1,3,5 triazine-2,4,7(3H.6H)-trione | 14-1 | 6 |
| 14 | 15A | 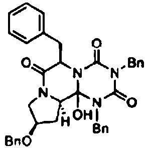 | (6R,10R,11aR)-1.3,6-tribenzyl-10-(benzyl oxy)-11b-hydroxyhexahydro-2H-pyrrolo [2',1':3,4]pyrazino[1,2-a][1.3,5] triazine-2,4,7(3H.6H)-trione | 15-1 | 9 |
| 14 | 15B | 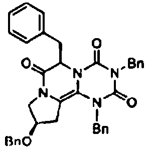 | (6R,10R)-1,3,6-tribenzyl-10-(benzyl oxy)-1,9,10,11-tetrahydro-2H-pyrrolo[2',1':3,4] pyridazino[1,2-a][1,3,5] triazine-2,4,7(3H,6H)-trione | 15-1 | 7 |

| | | | | | |
|---|---|---|---|---|---|
| Exam. No.: Example Number Com. No.: Compound Number Mta: Material Yd: Yield | | | | | |

### Examples 15-17

According to the same reaction conditions as in Example 1, the three compounds shown in Table 3 were synthesized by replacing benzyl isocyanate with various isocyanates.

### [Table 3]

**Table 3**

| Exam. No. | Com. No. | Chemical Structure | Compound Name | isocyanate | Yd (%) |
|---|---|---|---|---|---|
| 15 | 16B | 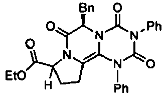 | Ethyl (6R) 6 benzyl 2. 4.7 trioxo 1.3 diphenyl 1.3.4.6.7.9.10.11-octahydro 2H pyrrolo[2 '.1':3.4]pyrazino[1,2-a] [1,3,5]triazine 9-carboxylate | phenyl isocyanate | 14 |
| 16 | 17A | 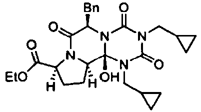 | Ethyl (6R.9S.11aR.11b R) 6 benzyl 1.3 bis(cyclopropylmethyl)-11b hydroxy-2,4,7-trioxodecahydro 2H py rrolo[2',1':3,4]pyrazino [1.2 a][1,3,5]triazine 9-carboxylate | isocyanate methyl-cyclopropane | 21 |
| 17 | 18A | 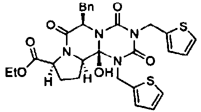 | Ethyl (6R.9S.11aR.11b R) 6 benzyl 11b-hydroxy 2,4,7 trioxo-1.3 bis(thiophen 2 ylm ethyl)decahydro-2H-pyrrolo[2',1':3,4] pyridazine[1,2-a] [1,3,5]triazine 9 carboxylate | 2 isocyanate-methylthiophene | 50 |

| | | | | | |
|---|---|---|---|---|---|
| Yd: Yield | | | | | |

### Example 18

### (6R,9S,11aR,11bR)-1,3,6-Tribenzyl-11b-hydroxy-N-(naphthalen-2-ylmethyl)-2,4,7-trioxo-2H-pyrrolo[2',1':3,4] pyridazine[1,2-a][1,3,5]triazine-9-carboxamide 1D

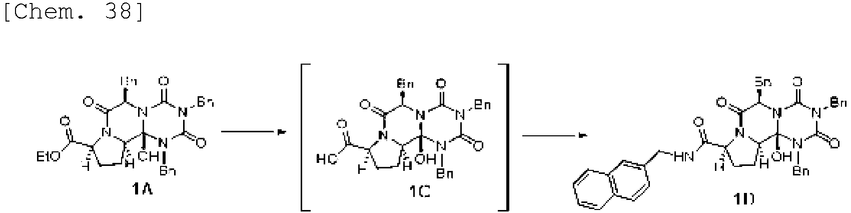

### 18-1: (6R,9S,11aR,11bR)-1,3,6-Tribenzyl-11b-hydroxy-2,4,7-trioxo-2H-pyrrolo[2',1':3,4]pyridazino[1,2-a][1,3,5] triazine-9-carboxylic acid 1C

Compound 1A prepared in Example 1 (70 mg, 0.120 mmol) was added to a 25 ml egg-plant shaped flask, and was dissolved in tetrahydrofuran (1.27 ml), methanol (1.92 ml), and water (0.632 ml). Next, lithium hydroxide (126 mg, 3.00 mmol) was added thereto, and the mixture was stirred at room temperature for 3 hours. After the reaction was quenched with 1M hydrochloric acid aqueous solution, the mixture was extracted with ethyl acetate, and after distilling off the solvent, the desired compound 1C (white solid) was obtained in an amount of 63.9 mg (0.115 µmol, 96%).

### 18-2: compound 1D

Next, to compound 1C (63.9 mg, 0.115 mmol) contained in a 10 ml egg-plant shaped flask, dichloromethane (3.84 ml), then HATU (87.6 mg (0.230 mmol) and 1-(2-naphthyl)methylamine (38.1 mg, 0.230 mmol), and finally diisopropylethylamine (0.120 ml, 0.691 mmol) were added, and the mixture was stirred under a nitrogen atmosphere at room temperature for one and a half hours. After removing the stirrer bar and distilling off the solvent, water was added and extracted with ethyl acetate. The residue was purified using a silica gel column Q-Pack SI30 size 20 (hexane: ethyl acetate = 17:83), and after the solvent was removed, the desired 1D (white solid) was obtained in an amount of 73.1 mg (0.105 mmol, 91%).

### Examples 19-27

According to the same reaction conditions as in Example 18, the ten compounds shown in Table 4 were synthesized by replacing the starting material compound 1A with compounds 2A, 8A, 17A, and 18A.

**[Table 4-1]**

| Table 4 | | | | | |
|---|---|---|---|---|---|
| Exam. No. | Com. No. | Chemical Structure | Compound Name | Mat | Yd (%) (After Two Steps) |
| 19 | 2D | 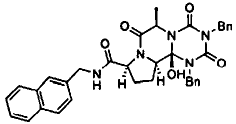 | (6R.9S.11aR.11bR)-1.3-Dibenzyl-11b-hydroxy-6-methyl-N-(naphthalen-2-ylmethyl)-2,4,7-trioxodecahydro-2H-pyrrolo[2',1':3.4] pyridazino[1,2-a][1,3,5] triazine-9-carboxamide | 2A | 77 |
| 20 | 3D | 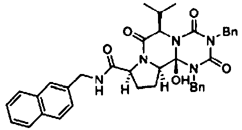 | (6R,9S,11aR,11bR)-1,3-Dibenzyl-11b-hydroxy-6-isopropyl-N-(naphthalen-2-ylmethyl)-2,4,7-trioxodecahydro-2H-pyrrolo[ 2',1':3,4]pyrazino[1,2-a] [1,3,5]triazine⁻9⁻carbamide | 3A | 75 |
| 21 | 4C | 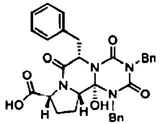 | (6S,9R,11aS,11bS)-1,3,6-Tri benzyl-11b-hydroxy-2,4,7-tri oxodecahydro-2H-pyrrolo [2',1':3,4]pyridazine[1,2-a] [1,3,5]triazine-9-carboxylic acid | 4A | 100 |
| 21 | 4D | 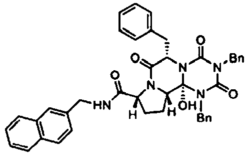 | (6S,9R,11aS,11bS)-1,3,6-Tribenzyl-11b-hydroxy-N-(naphthalen-2-ylmethyl)-2,4 ,7-trioxo-2H-pyrrolo [2',1':3,4]pyrazino [1,2-a][1,3,5]triazine-9-carboxamide | 4A | 84 |
| 22 | 5D | 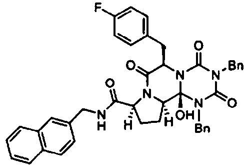 | (6R.9S.11aR.11bR)-1,3-Dibenzyl-6-(4-fluorobenzyl)-11b-hydroxy-N-(naphthalen-2-yl methyl)-2,4,7-trioxodecahydro-2H-pyrrolo[2',1':3,4]pyridazine [1,2-a][1,3,5]triazine-9-carboxamide | 5A | 91 |

**[Table 4-2]**

| | | | | | |
|---|---|---|---|---|---|
| 23 | 6D | 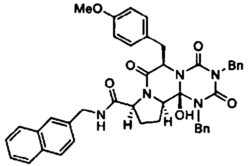 | (6R,9S,11aR,11bR)-1,3-Dibenzyl-11b-hydroxy-6-(4 -methoxybenzyl)-N-(naphthalen-2-ylmethyl)-2,4,7-trioxodecahydro-2H-pyrrolo[2',1':3,4] pyridazino[1,2-a][1.3.5] triazine-9-carboxamide | 6A | 56 |
| 24 | 7D | 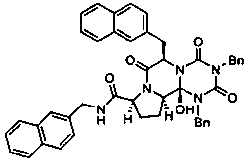 | (6R,9S,11aR,11bR)-1,3-Dibenzyl-11b-hydroxy-N,6-bis(naphthalen-2-ylmethyl)-2.4.7-trioxodecahydro-2H-pyrrolo[2'.1':3,4] pyrazino[1,2-a][1,3,5] triazine-9-carboxamide | 7A | 82 |
| 25 | 8D | 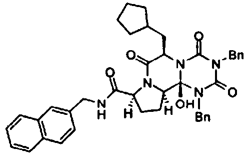 | (6R,9S,11aR,11bR)-1,3-Dibenzyl-6-(cyclopentylmethyl)-11b-h ydroxy-N,N-(naphthalen-2-ylmethyl)-2,4,7-trioxodecahydro-2H-pyrrolo[2',1':3,4]pyrazino [1,2-a][1,3,5]triazine-9-carboxamide | 8A | 100 |
| 26 | 17D | 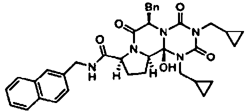 | (6R,9S,11aR,11bR)-6-Benzyl-1,3-bis (cyclopentylmethyl)-11b-h ydroxy-N-(naphthalen-2-yl methyl)-2,4,7-trioxodecahydro-2H-pyrrolo[2',1':3,4] pyridazino[1,2-a][1,3,5] triazine-9-carboxamide | 17A | 100 |
| 27 | 18D | 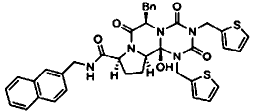 | (6R,9S,11aR,11bR)-6-benzyl-11b-hydroxy-N-(naphthalen-2-ylmethyl)-2 ,4,7-trioxo-1,3-bis (thien-2-ylmethyl) decahydro-2H-pyrrolo [2',1':3,4]pyridazino[1,2-a] [1.3,5]triazine-9-carboxamide | 18A | 50 |

| | | | | | |
|---|---|---|---|---|---|
| Exam. No.: Example Number Com. No.: Compound Number Mta: Material Yd: Yield | | | | | |

### Example 28

### (6R,9S)-6-Benzyl-N-(naphthalen-2-ylmethyl)-2,4,7-trioxo-1,3-diphenyl-1,3,4,6,7,9,10,11-octahydro-2H-pyrrolo [2',1':3,4]pyrazino[1,2-a][1,3,5]triazine-9-carboxamide 16E

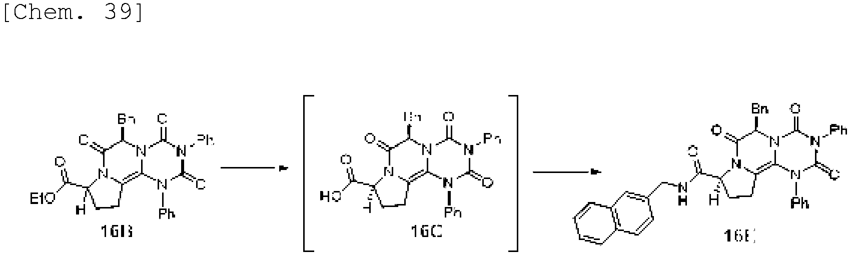

### 28-1: (6R,9S)-6-benzyl-11b-2,4,7-trioxo-1,3,4,6,7,9,10,11-octahydro-2H-pyrrolo[2',1':3,4]pyridazine[1,2-a][1,3,5] triazine-9-carboxylic acid 16C

Compound 16B prepared in Example 15 (3.5 mg, 6.5 µmol) was added to a 4 ml vial, and was dissolved in tetrahydrofuran (0.069 ml), methanol (0.104 ml), and water (0.034 ml). Next, lithium hydroxide (6.8 mg, 0.163 mmol) was added thereto, and the mixture was stirred at room temperature for 3 hours. After the reaction was quenched with 1M hydrochloric acid aqueous solution, the mixture was extracted with ethyl acetate, and after distilling off the solvent, the desired compound 16C (white solid) was obtained in an amount of 3.0 mg (5.9 µmol, 91%).

### 28-2: compound 16E

Next, to compound 16C (3.0 To 5.9 mg (18 µmol) contained in a 4 ml vial, dichloromethane (0.295 ml), then HATU (6.7 mg, 18 µmol) and 1-(2-naphthyl)methylamine (2.9 mg, 18 µmol), and finally diisopropylethylamine (9.2 µl, 53 µmol) were added, and the mixture was stirred at room temperature for 1 hour. After distilling off the solvent, water and saturated saline solution were added thereto and the mixture was extracted with ethyl acetate. The residue was purified using the silica gel column Q-Pack SI30 size 10 (hexane: ethyl acetate = 34:66), and after distilling off the solvent, the desired 16E (white solid) was obtained in an amount of 2.2 mg (3.4 µmol, 59%).

### Example 29

### (6R,9S)-1,3,6-Tribenzyl-N-(naphthalen-2-ylmethyl)-2,4,7-trioxo-1,3,4,6,7,9,10,11-octahydro-2H-pyrrolo[2',1':3,4] pyrazino[1,2-a][1,3,5]triazine-9-carboxamide1E

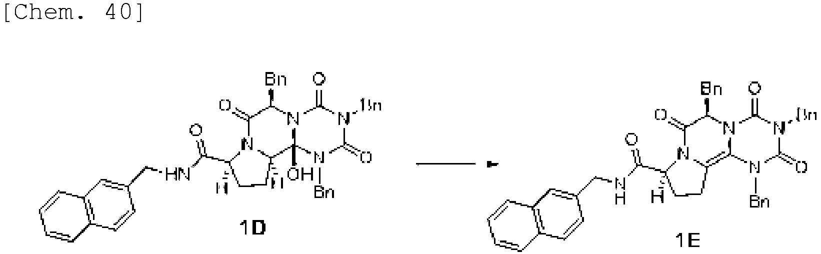

To compound 1D prepared in Example 18 (6.3 mg, 9.1 µmol) contained in a 4 ml vial, dichloromethane (0.908 ml) and trifluoroacetic acid (7.0 µl, 91 µmol) were added, and the mixture was stirred at room temperature for one and a half hours. The saturated sodium bicarbonate solution was added thereto for neutralization, and the mixture was extracted with ethyl acetate. After drying the organic layer with anhydrous magnesium sulfate, it was filtered off, and after distilling off the solvent, the desired 1E (white solid) was obtained in an amount of 6.1 mg (9.1 µmol, 100%).

### Examples 30-34

According to the same reaction conditions as in Example 29, the five compounds shown in Table 5 were synthesized by replacing the starting material, compound 1D, with compounds 2D (Example 19), 4D (Example 21), 5D (Example 22), 7D (Example 24), and 8D (Example 25).

### [Table 5]

**Table 5**

| Exam. No. | Com. No. | Chemical Structure | Compound Name | Mta | Yd (%) |
|---|---|---|---|---|---|
| 30 | 2E | 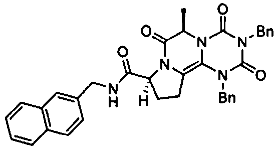 | (6R,9S)-1,3-Dibenzyl-6-methyl-N-(naphthalen-2-ylmethyl)-2,4,7-trioxo-1,3,4 ,6,7,9,10,11-octahydro-2H-pyrrolo[2',1': 3,4]pyrazino[1,2-a][1,3,5]tr iazine-9-carboxamide | 2D | 100 |
| 31 | 4E | 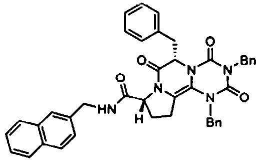 | (6S,9R)-1,3,6-Tribenzyl-N-( naphthalen-2-ylmethyl)-2,4,7-trioxo-1,3,4,6,7,9,10,1 1-octahydro-2H-pyrrolo [2',1':3,4]pyrazino[1,2-a] [1,3,5]triazine-9-carboxamide | 4D | 100 |
| 32 | 5E | 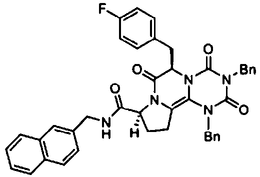 | (6R.9S)-1.3-benzyl-6-(4-fluorobenzyl)-N-(naphthalen-2-ylmethyl)-2, 4,7-trioxo-1,3,4,6,7,9,10,11-octahydro-2H-pyrrolo [2',1':3,4]pyridazine [1,2-a][1,3,5]triazine-9-carboxamide | 5D | 100 |
| 33 | 7E | 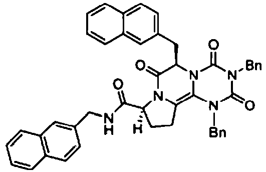 | (6R,9S)-1,3-Dibenzyl-N,6-Bis(naphthalen-2-ylmethyl) -2,4,7-trioxo-1,3,4,6,7,9,10, 11-octahydro-2H-pyrrolo [2',1':3,4]pyrazino[1,2-a] [1,3,5]triazine-9-carboxamide | 7D | 100 |
| 34 | 8E | 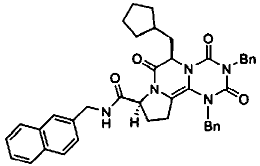 | (6R,9S)-1,3-Dibenzyl-6-(cyclopentylmethyl)-N-(naphthalen-2-ylmethyl)-2, 4,7-trioxo-1,3,4,6,7,9,10,11-octahydro-2H-pyrrolo [2',1':3,4]pyrazino[1,2-a] [1,3,5]triazine-9-carboxamide | 8D | 91 |

| | | | | | |
|---|---|---|---|---|---|
| Exam. No.: Example Number Com. No.: Compound Number Mta: Material Yd: Yield | | | | | |

### Preparation examples 16-23

According to the same reaction conditions as in Preparation Example 1, the eight compounds shown in Table 6 were synthesized by replacing the amine raw material 2,5-diethyl-(2R,5S)-pyrrolidine-2,5-dicarboxylate hydrochloride with various amine raw materials, and by replacing the carboxylic acid raw material N-Fmoc-D-phenylalanine with various carboxylic acid raw materials. However, in Preparation Examples 21, 22, and 23, since Boc protected compounds were used as carboxylic acid raw materials, after condensation, the Boc groups were removed using trifluoroacetic acid, and they were treated directly with a base (triethylamine or piperidine) to induce cyclization.

**[Table 6-1]**

| Table 6 | | | | | |
|---|---|---|---|---|---|
| Pre. No. | Com . No. | Chemical Structure | Amine Raw materials | Carboxylic Acid Raw Materials | Yds (%) (Post 2 Steps) |
| 16 | 19 1 | 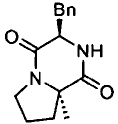 | 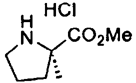 | 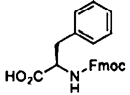 | 87 |
| 17 | 201 | 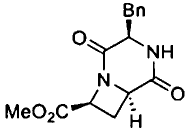 | 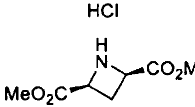 | 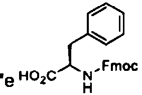 | 23 |
| 18 | 21 1 | 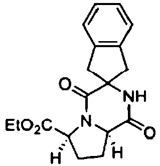 | 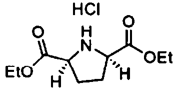 | 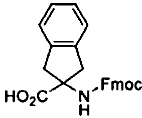 | 20 |
| 19 | 24 1 | 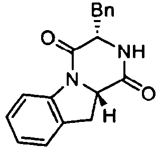 | 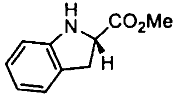 | 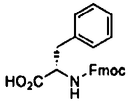 | 22 |
| 20 | 25 1 | 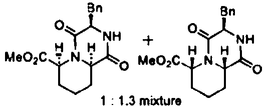 | 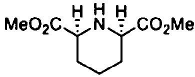 | 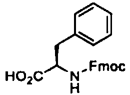 | 13 |

**[Table 6-2]**

| | | | | | |
|---|---|---|---|---|---|
| 21 | 261 | 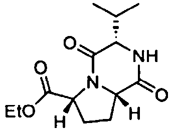 | 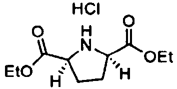 | 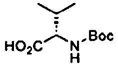 | 39 |
| 22 | 291 | 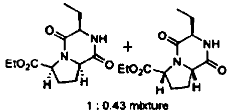 | 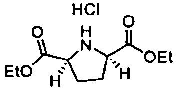 | 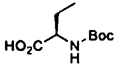 | 82 |
| 23 | 30 1 | 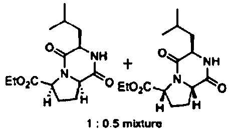 | 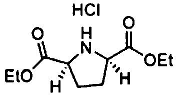 | 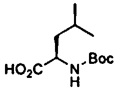 | 83 |

### Examples 35-46

According to the same reaction conditions as in Example 1, the 14 compounds shown in Table 7 were synthesized by replacing the starting material compound 1-1 with various synthesized diketopiperazines, and replacing benzyl isocyanate with various isocyanates.

**[Table 7-1]**

| Table 7 | | | | | | |
|---|---|---|---|---|---|---|
| Exam. No. | Com .No. | Chemical Structure | Compound Name | Mat | isocyan ate | Yds (%) |
| 35 | 19A | 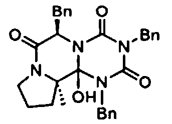 | (6R.11aR)-1,3,6-tribenzyl-11b-hydroxy-11a-methylhexahydro-2H-pyrrolo[2',1':3,4] pyrazino[1,2-a][1,3,5] triazine-2,4,7(3H,6H)-trione | 19-1 | benzyl isocyan ate | 40 |
| 36 | 20A | 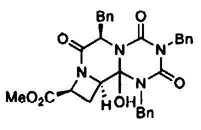 | Methyl (6R,9S,10aR)-1,3,6-tribenzyl-10b-hydroxy-2,4,7-trioxodecahydroazeto [2',1':3,4]pyridino[1,2-a][1,3,5]triazine-9-carboxylate | 20-1 | benzyl isocyan ate | 4 |
| 36 | 20C | 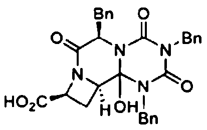 | (6R,9S,10aR)-1,3,6-tribenzyl-10b-hydroxy-2,4,7-trioxodecahydroazeto [2',1':3,4]pyridazine [1,2-a][1,3,5]triazine-9-carboxylic acid | 20-1 | benzyl isocyan ate | 56 |
| 37 | 21C | 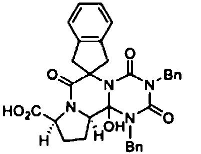 | (9'S,11a'R)-1',3'-Dibenzyl-11b'-hydroxy-2',4',7'-trioxo-1,1',3,3',4',9',10',11',11a ',11b'-decahydro-2'H,7'H-spiro[indene-2,6'-pyrrolo[2',1':3,4] pyrazino[1,2-a][1,3,5] triazinel-9'-carboxylic | 21-1 | benzyl isocyan ate | 8 |

**[Table 7-2]**

| | | | | | | |
|---|---|---|---|---|---|---|
| | | | acid | | | |
| 38 | 22A | 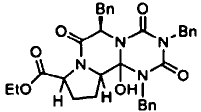 | Ethyl (6R)-1,3,6-tribenzyl-11b-hydroxy-2,4,7-trioxodecahydro-2H-pyrrolo[2',1':3,4] pyridazino[1,2-a] [1,3,5]triazine-9-carboxylate | 1-1 | benzyl isocyan ate | 7 |
| 39 | 23A | 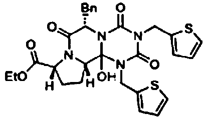 | Ethyl (6S,9R,11aS)-6-benzyl-11b-hydroxy-2,4.7-trioxo-1,3-bis(thiophen-2-ylmethyl) decahydro-2H-pyrrolo[2',1':3,4] pyrazino[1,2-a][1,3,5] triazine-9-carboxylate | 4-1 | 2-Isocyan ate methyl thiophe ne | 17 |
| 40 | 24A | 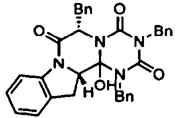 | (6S,13aS)-1,3,6-tribenzyl-13b-hydroxy-1,13,13a,13b-tetrahydro-2H-[1,3,5] triazino[2',1':3,4]pyraz ino[1,2-a]indole-2,4.7(3H,6H)-trione | 24-1 | benzyl isocyan ate | 59 |
| 41 | 25A | 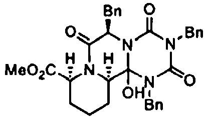 | Methyl (6R,9S,12aR)-1,3,6-tribenzyl-12b-hydroxy-2,4,7-trioxododecahydropyri do[2',1':3,4]pyrazino[1, 2-a][1,3,5]triazine-9-carboxylate | 25-1 | benzyl isocyan ate | 14 |

**[Table 7-3]**

| | | | | | | |
|---|---|---|---|---|---|---|
| 42 | 26A | 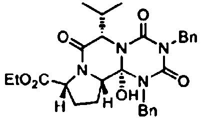 | Ethyl (6S,9R,11aS,11bS)-1,3-dibenzyl-11b-hydroxy-6-isopropyl-2,4,7-trioxodecahydro-2H-pyrrolo[2',1':3,4]pyrazi no[1,2-a][1,3,5] triazine-9-carboxylate | 26-1 | benzyl isocyan ate | 38 |
| 43 | 27A | 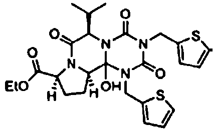 | Ethyl (6R,9S,11aR)-11b-hydroxy-6-isopropyl-2.4,7-trioxo-1,3-bis(thiophen-2-ylmethyl)decahydro-2H-pyrrolo[2',1':3,4] pyrazino[1,2-a][1,3,5] triazine-9-carboxylate | 3-1 | 2-Isocyan ate methyl thiophe ne | 36 |
| 44 | 28A | 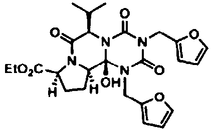 | Ethyl (6R,9S,11aR,11bR)-1,3-bis(furan-2-ylmethyl)-11b-hydroxy-6-isopropyl-2,4,7-trioxodecahydro-2H-pyrrolo[2',1':3,4] pyrazino[1,2-a][1,3,5] triazine-9-carboxylate | 3-1 | 2-Isocyan ate methyl furan | 82 |
| 45 | 29A | 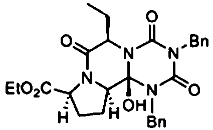 | Ethyl (6R,9S,11aR,11bR)-1,3-dibenzyl-6-ethyl-11b-hydroxy-2,4,7-trioxodecahydro-2H-pyrrolo[2',1':3,4]pyrazi no[1,2-a][1,3,5] triazine-9- | 29-1 | benzyl isocyan ate | 50 |

**[Table 7-4]**

| | | | | | | |
|---|---|---|---|---|---|---|
| | | | carboxylate | | | |
| 46 | 30A | 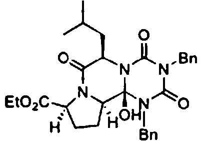 | Ethyl (6R,9S,11aR,11bR)-1.3 dibenzyl 11b-hydroxy 6 isobutyl-2.4.7 trioxodecahydro-2H pyrrolo[2',1':3,4] pyrazino[1.2 a][1.3.5] triazine 9 carboxylate | 301 | benzyl isocyan ate | 64 |
| 3 | 3B | 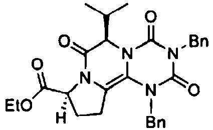 | Ethyl (6R.9S) 1,3-dibenzyl 6 isopropyl-2.4.7 trioxo-1,3,4,6,7,9,10,11-octahydro 2H-pyrrolo[2',1':3.4]pyrazi no[1,2 a][1,3,5] triazine 9 carboxylate | 3 1 | benzyl isocyan ate | 23 |

In the table, compound 3B is a byproduct obtained during the synthesis of compound 3A in Example 3.

### Examples 47-52

According to the same reaction conditions as in Example 18, the seven compounds shown in Table 8 were synthesized by replacing the starting material compound 1A with various synthesized triazine trione compounds. However, in Examples 47 and 48, compounds 20D and 21D were synthesized in one step from the carboxylic acid compounds 20C and 21C obtained in Examples 36 and 37, respectively.

**[Table 8-1]**

| Table 8 | | | | | |
|---|---|---|---|---|---|
| Exam. No. | Com .No. | Chemical Structure | Compound Name | Mat | Yds (%) (Post 2 Steps) |
| 47 | 20D | 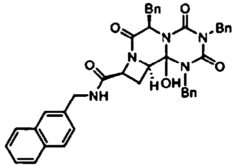 | (6R,9S,10aR)-1,3,6-tribenzyl-10b-hydroxy-N-(naphthalen-2-ylmethyl)-2,4,7-trioxodecahydroazeto[2',1 ':3,4]pyrazino[1.2-a][1,3,5]triazine-9-carboxamide | 20C | 49 |
| 48 | 21D | 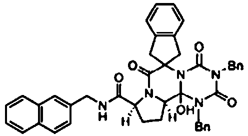 | (9'S,11a'R)-1',3'-Dibenzyl -11b'-Hydroxy-N-(naphthalen-2-ylmethyl)-2',4',7'-trioxo-1,1',3,3',4',9',10',11',11a',1 1b'-decahydro-2'H,7'H-spiro[indene-2,6'-pyrrolo[2',1':3,4]pyrazino[ 1,2-a][1,3,5]triazine]-9'-carboxamide | 21C | 65 |
| 49 | 26D | 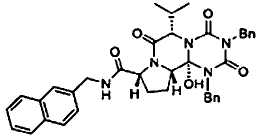 | (6S,9R,11aS,11bS)-1,3-Dibenzyl-11b-hydroxy-6-isopropyl-N-(naphthalen-2⁻ylmethyl)-2,4,7-trioxodecahydro-2H-pyrrolo[2',1':3,4]pyrazino[ 1,2-a][1,3,5]triazine-9-carboxamide | 26A | 84 |
| 50 | 27D | 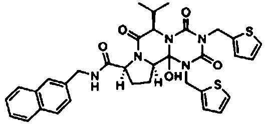 | (6R,9S,11aR)-11b-Hydroxy-6-isopropyl-N-(naphthalen-2-ylmethyl)-2,4,7-trioxo-1,3-bis(thien-2-ylmethyl)decahydro- | 27A | 68 |

**[Table 8-2]**

| | | | | | |
|---|---|---|---|---|---|
| | | | 2H-pyrrolo[2',1':3,4] pyridazine[1,2-a][1,3,5] triazine-9-carboxamide | | |
| 51 | 28D | 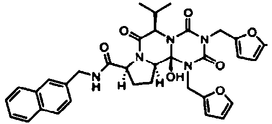 | (6R,9S,11aR,11bR)-1,3-bis(furan-2-ylmethyl)-11b-hydroxy-6-isopropyl-N-(naphthalen-2-ylmethyl)-2,4,7-trioxodecahydro-2H-pyrrolo[2',1':3,4]pyridazin e[1,2-a] [1,3,5]triazine-9-carboxamide | 28A | 92 |
| 52 | 29C | 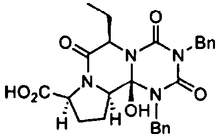 | (6R,9S,11aR,11bR)-1,3-Dibenzyl-6-ethyl-11b-hydroxy-2,4,7-trioxodecahydro-2H-pyrrolo[2',1':3,4]pyridazin e[1,2-a][1,3,5]triazine-9-carboxylic acid | 29A | 97 |
| 52 | 29D | 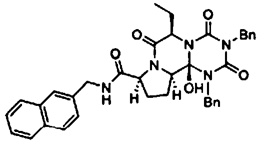 | (6R,9S,11aR,11bR)-1,3-Dibenzyl-6-ethyl-11b-hydroxy-6-isopropyl-N-(naphthalen-2-ylmethyl)-2,4,7-trioxodecahydro-2H-pyrrolo[2',1':3,4] pyrazino[1,2-a][1,3,5] triazine-9-carboxamide | 29A | 77 |

### Example 53

### (6R,9R)-1,3,6-Tribenzyl-N-(naphthalen-2-ylmethyl)-2,4,7-trioxo-1,2,3,4,6,7,9,10,11,12-decadihydropyrido[2',1':3,4] pyrazino[1,2-a][1,3,5]triazine-9-carboxamide 25E

### Example 53-1: compound 25C

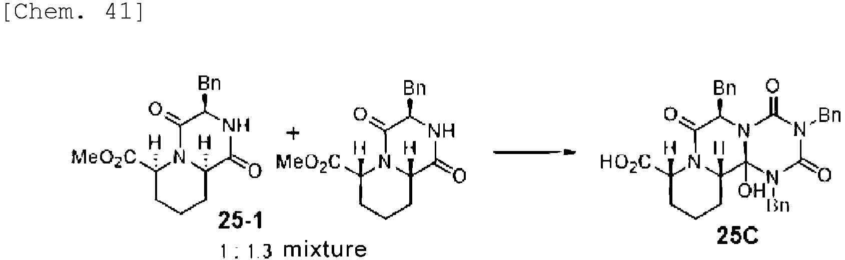

Compound 25-1 prepared in Preparation Example 20 (37.7 mg, 0.119 mmol) of magnesium (Mg) was added to a 10 ml egg-plant shaped flask, and was dissolved in tetrahydrofuran (1.19 ml). Next, benzyl isocyanate (45 µl, 0.358 mmol) and 60% sodium hydride (42.9 mg, 1.073 mmol) were added thereto, and the mixture was stirred under a nitrogen atmosphere at room temperature for 20 minutes. After the reaction was quenched with the saturated ammonium chloride aqueous solution, the mixture was extracted with ethyl acetate. After distilling off the solvent, the residue was purified using a silica gel column Q-Pack SI30 size 10 (hexane: ethyl acetate = 50:50 and chloroform: methanol = 95:5 to 75:25). After distilling off the solvent, the desired compound 25C (white amorphous solid) was obtained in an amount of 20 mg (35 µmol, 30%).

### Example 53-2: compound 25D

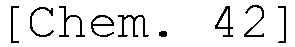

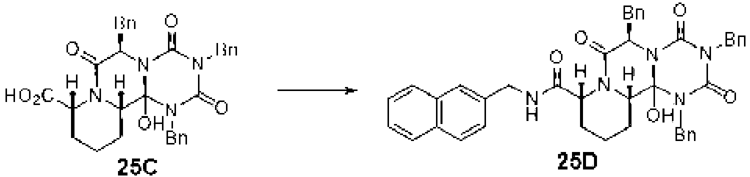

To compound 25C prepared in Example 53-1 (20 mg, 35 µmol) contained in a 10 ml egg-plant shaped flask, dichloromethane (1.17 ml), then HATU (26.7 mg, 70 µmol) and 1-(2-naphthyl)methanamine (11.6 mg, 70 µmol), and finally diisopropylethylamine (36.8 µl, 0.211 mmol) were added, and the mixture was stirred under a nitrogen atmosphere at room temperature for two and a half hours. After removing the stirrer bar and distilling off the solvent, water was added thereto and the mixture was extracted with ethyl acetate. The residue was purified using a silica gel column Q-Pack SI30 size 10 in a two-stage process (hexane: ethyl acetate = 60:40 to 50:50). After distilling off the solvent, the desired 25D (white solid) was obtained in an amount of 17.6 mg (25 µmol, 71%).

### Example 53-3 : the compound of Example

### (6R,9R)-1,3,6-Tribenzyl-N-(naphthalen-2-ylmethyl)-2,4,7-trioxo-1,2,3,4,6,7,9,10,11,12-decahydropyrido[2',1':3,4] pyrazino[1,2-a][1,3,5]triazine-9-carboxamide 25E

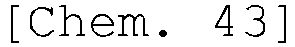

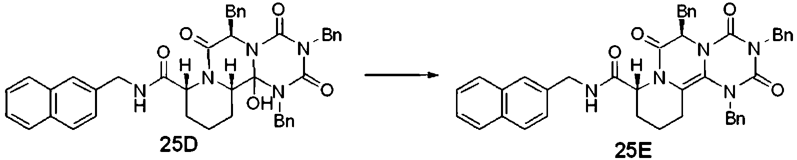

To compound 25D prepared in Example 53-2 (4.2 mg, 5.9 µmol) contained in a 4 ml vial, dichloromethane (0.593 ml) and trifluoroacetic acid (4.5 µl, 59.3 µmol) were added, and the mixture was stirred at room temperature for one and a half hours. The saturated sodium bicarbonate solution was added thereto for neutralization, and the mixture was extracted with ethyl acetate. After drying the organic layer with anhydrous magnesium sulfate, it was filtered off, and after distilling off the solvent, the desired 25E (amorphous solid) was obtained in an amount of 4.1 mg (5.9 µmol, 100%).

### Example 54

### (6R,9S,11aR,11bR)-6-Benzyl-11b-hydroxy-N-methyl-2,4,7-trioxo-1,3-bis(thiophen-2-ylmethyl)decahydro-2H-pyrrolo [2',1':3,4]pyridazine[1,2-a][1,3,5]triazine-9-carboxamide 18F

### Example 54-1: compound 18C

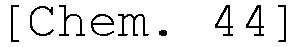

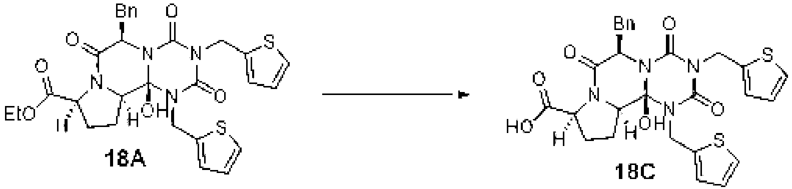

Compound 18A prepared in Example 17 (13.1 mg, 22 µmol) was added to a 4 ml vial, and was dissolved in tetrahydrofuran (0.232 ml), methanol (0.352 ml), and water (0.116 ml). Next, lithium hydroxide (23.1 mg, 0.551 mmol) was added thereto, and the mixture was stirred at room temperature for 3 hours. After the reaction was quenched with 1M hydrochloric acid aqueous solution, the mixture was extracted with ethyl acetate, and after distilling off the solvent, the desired compound 18C (white solid) was obtained in an amount of 12.4 mg (22 µmol, 100%).

### Example 54-2 : the compound of Example 54

### (6R,9S,11aR,11bR)-6-benzyl-11b-hydroxy-N-methyl-2,4,7-trioxo-1,3-bis(thiophen-2-ylmethyl)decahydro-2H-pyrrolo [2',1':3,4]pyrazino[1,2-a][1,3,5]triazine-9-carboxamide 18F

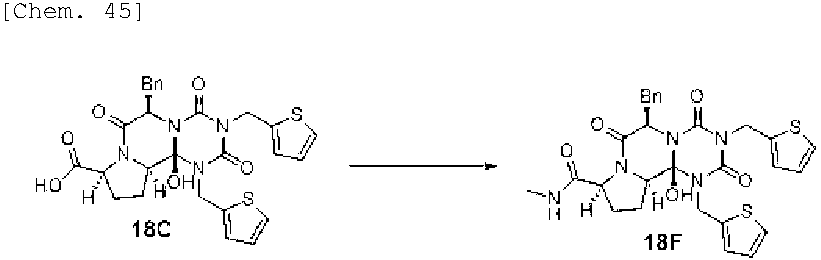

To compound 18C prepared in Example 54-1 (7.3 mg, 12.9 µmol) contained in a 4 ml vial, dichloromethane (0.368 ml), then HATU (9.8 mg, 26 µmol) and methylamine hydrochloride (2.7 mg, 39 µmol), and finally diisopropylethylamine (13.5 µl, 77 µmol) were added, and the mixture was stirred at room temperature for 1 hour. After distilling off the solvent, a saturated solution of sodium bicarbonate was added and extracted with ethyl acetate. The residue was purified using the silica gel column Q-Pack SI30 size 10 (hexane: ethyl acetate = 50:50 and chloroform: methanol = 100:0 to 95:5). After distilling off the solvent, the desired compound 18F (white solid) was obtained in an amount of 7.1 mg (12.2 µmol, 93%).

### Example 55

### Isopropyl (6R,9S,11aR,11bR)-6-benzyl-11b-hydroxy-2,4,7-trioxo-1,3-bis(thiophen-2-ylmethyl)decahydro-2H-pyrrolo [2',1':3,4]pyridazine[1,2-a][1,3,5]triazine-9-carboxylate 18G and

### (6R,9S,11aR)-6-benzyl-N-((3-(dimethylamino)propyl) carbamoil)-N-ethyl-11b-Hydroxy-2,4,7-trioxo-1,3-bis(thiophen-2-ylmethyl)decahydro-2H-pyrrolo[2',1':3,4] pyrazino[1,2-a][1,3,5]triazine-9-carboxamide 18H

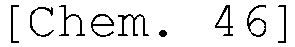

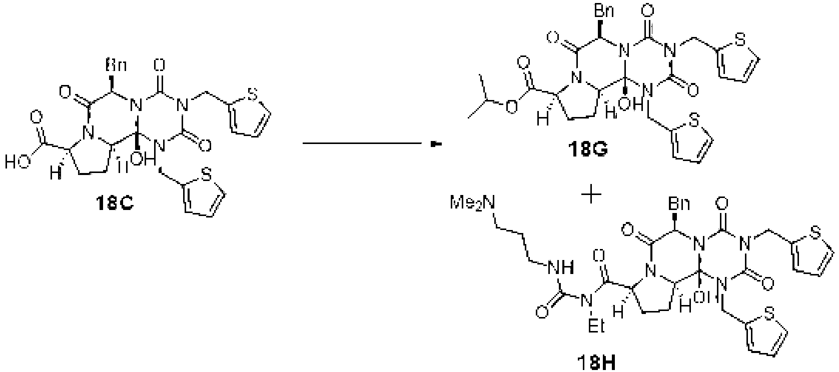

To compound 18C prepared in Example 54-1 (10 mg, 17.6 µmol) contained in a 4 ml vial, dichloromethane (0.441 ml) and DMF (0.22 ml), then DMAP (1.1 mg, 9.0 µmol), isopropanol (27.6 µl, 35.3 µmol), and EDCI (4.5 mg, 23 µmol) were added, and the mixture was stirred at room temperature for 4 hours. Since no progress of the reaction was observed, HATU (20.5 mg, 53 µmol) and diisopropylethylamine (28.2 µl, 0.159 mmol) were subsequently added thereto, and the mixture was stirred at room temperature for 2 hours. The saturated sodium bicarbonate solution was added to quench the reaction, and the mixture was extracted with ethyl acetate. After distilling off the solvent, the residue was purified using a silica gel column Q-Pack SI30 size 10 (hexane: ethyl acetate = 80:20 to 50:50 and chloroform: methanol = 100:0 to 95:5). After distilling off the solvent again, the desired compound 18G (amorphous solid) was obtained in an amount of 2.2 mg (3.6 µmol, 21%) and compound 18H (amorphous solid) in an amount of 5.2 mg (7.2 µmol, 41%).

### Example 56

### (6R,9S,11aR)-6-benzyl-11b-hydroxy-9-(3-methyl-1,2,4-oxadiazol-5-yl)-1,3-bis(thiophen-2-ylmethyl)hexahydro-2H-pyrrolo[2',1':3,4]pyridazine[1,2-a][1,3,5]triazine-2,4,7(3H,6H)-trione 18I

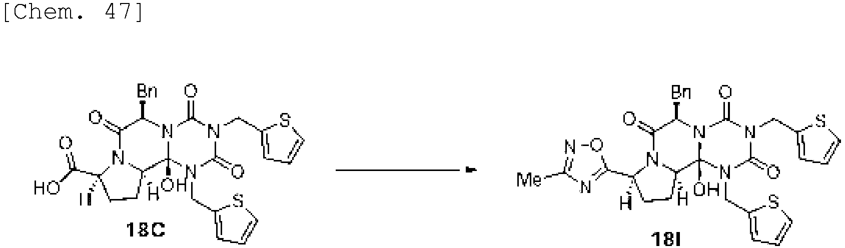

To compound 18C prepared in Example 54-1 (14.5 mg, 21 µmol) contained in a 4 ml vial, DMF (0.417 ml), and then HATU (23.8 mg, 63 µmol) and diisopropylethylamine (32.7 µl, 0.188 mmol) were added, and the mixture was stirred at room temperature for 8 minutes. Subsequently, (Z)-N'-hydroxyacetimidamide (6.5 mg, 83 µmol) was added thereto and the mixture was stirred at room temperature for 30 minutes. Saturated sodium chloride solution was added thereto to quench the reaction, and the mixture was extracted with ethyl acetate. After distilling off the solvent, the residue was purified using a silica gel column Q-Pack SI30 size 10 (hexane:ethyl acetate = 50:50 and chloroform:methanol = 100:0 to 95:5). After distilling off the solvent, the intermediate condensate was obtained in an amount of 9.8 mg (16 µmol, 75%). This was dissolved in toluene (1.57 ml) and dioxane (0.393 ml), and molecular sieves 4Å (98 mg) was added thereto, and the mixture was stirred at 100°C for 21.5 hours. The molecular sieves 4Å were washed with chloroform and removed by filtration. After distilling off the solvent, the residue was purified using a silica gel column Q-Pack SI30 size 10 (hexane:ethyl acetate = 75:25 to 40:60). After distilling off the solvent again, the desired compound 18I (white solid) was obtained in an amount of 7.4 mg (12 µmol, 78%).

### Example 57

### (6R,9S,11aR,11bR)-N,1,3-Tribenzyl-11b-hydroxy-6-isopropyl-2,4,7-trioxodecahydro-2H-pyrrolo[2',1':3,4]pyrazino[1,2-a] [1,3,5]triazine-9-carboxamide 3F

### Example 57-1: compound 3C

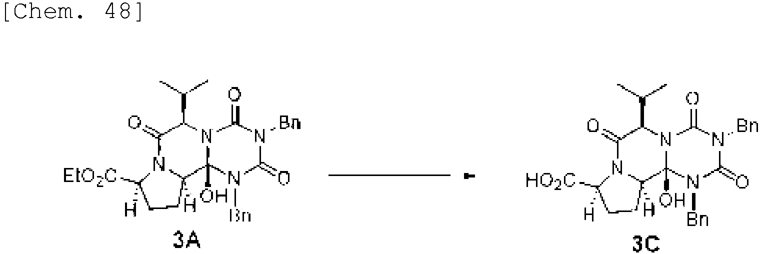

Compound 3A prepared in Example 3 (486.5 mg, 0.910 mmol) was added to a 25 ml egg-plant shaped flask, and was dissolved in tetrahydrofuran (2.40 ml), methanol (3.64 ml), and water (1.21 ml). Next, lithium hydroxide (190.9 mg, 4.55 mmol) was added thereto, and the mixture was stirred at room temperature for 3 hours. After the reaction was quenched with 1M hydrochloric acid aqueous solution, the mixture was extracted with ethyl acetate, and after distilling off the solvent, the desired compound 3C (a white solid) was obtained in an amount of 461 mg (0.910 mmol, 100%).

### Example 57-2 : the compound of Example 57

### (6R,9S,11aR,11bR)-N,1,3-tribenzyl-11b-hydroxy-6-isopropyl-2,4,7-trioxodecahydro-2H-pyrrolo[2',1':3,4]pyrazino[1,2-a][1,3,5]triazine-9-carboxamide 3F

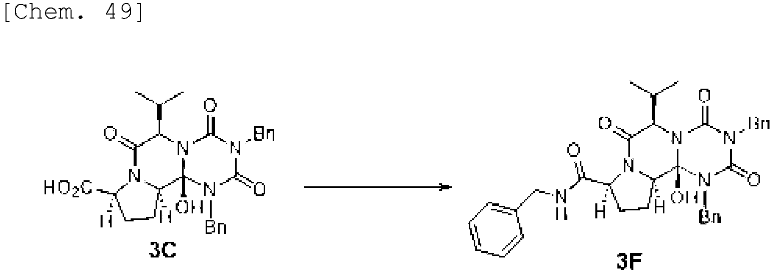

To compound 3C prepared in Example 57-1 contained in a 10 ml egg-plant shaped flask (20.0 mg, 39 µmol), dichloromethane (1.32 ml), then HATU (41.3 mg, 0.237 mmol), benzylamine (8.7 µl, 79 µmol), and finally diisopropylethylamine (41.3 µl, 0.237 mol) were added, and the mixture was stirred at room temperature for 2 hours. After distilling off the solvent, the residue was purified using a silica gel column Q-Pack SI30 size 10 with a two-connected system (hexane: ethyl acetate = 20:80). After distilling off the solvent again, the desired compound 3F (white solid) was obtained in an amount of 20.7 mg (35 µmol, 88%).

### Examples 58-61

According to the same reaction conditions as in Example 57, the four compounds shown in Table 9 were synthesized by replacing the amine raw material, benzylamine, with various amines.

**[Table 9-1]**

| Table 9 | | | | | |
|---|---|---|---|---|---|
| Exam .No. | Com. No. | Chemical Structure | Compound Name | Amine Raw materials | Yds (%) |
| 58 | 3G | 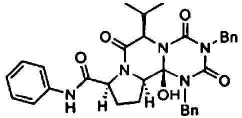 | (6R,9S,11aR,11bR)-1,3-Dibenzyl-11b-hydroxy-6-isopropyl-2,4,7-trioxo-phenyl-deca-hydro-2H-pyrrolo [2',1':3,4]pyrazino[1, 2-a][1,3,5]triazine-9-carboxamide | aniline | 85 |
| 59 | 3H | 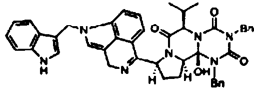 | (6R,95,11aR,11bR)-9-(1-((1H-indole-3-yl))-1,3-dihydropyrrolo[4,3, 2-de]isoquinolin-5-yl)-1,3-dibenzyl-11b-hydroxy-6-isopropylhexahydro -2H-pyrrolo [2',1':3,4]pyridazine [1,2-a][1,3,5] triazine-2,4,7 (3H. 6H)-trione | (1H-Indole-3)-methana mine | 55 |
| 60 | 3I | 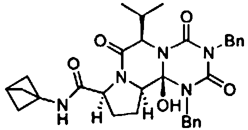 | (6 R,9 S,11aR,11bR)-1,3-Dibenzyl-N-(bicyclo[1,1,1]penta n-1-yl) | Bicyclo [1,1,1] pentan-1-amine hydrochlo ride | 89 |
| | | | 11b-Hydroxy-6-isopropyl-2,4,7-trioxodecahydro- | | |

**[Table 9-2]**

| | | | | | |
|---|---|---|---|---|---|
| | | | 2H-pyrrolo [2",1":3,4]pyrazino[ 1,2-a][1,3,5] triazine-9-carboxamide | | |
| 61 | 3J | 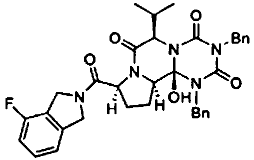 | (6R,9S,11aR,11bR)-1,3-Dibenzyl-9-(4-fluoroisoindolin-2-carbonyl)-11b-hydroxy-6-isopropylhexahydro -2H-pyrrolo[2',1':3,4]pyri dazino[1,2-a][1,3,5] triazine-2.4. 7(3H.6H)-trione | 4-Fluoro-2,3-dihydro-1H-isoindole | 94 |

### Example 62

### (6R,9S,11aR,11bR)-1,3-bis(fran-2-ylmethyl)-11b-hydroxy-6-isopropyl-2,4,7-trioxo-N-(quinolin-3-ylmethyl)decahydro-2H-pyrrolo[2',1':3,4]pyrazino[1,2-a][1,3,5]triazine-9-carbamide 28F

### Example 62-1: compound 28C

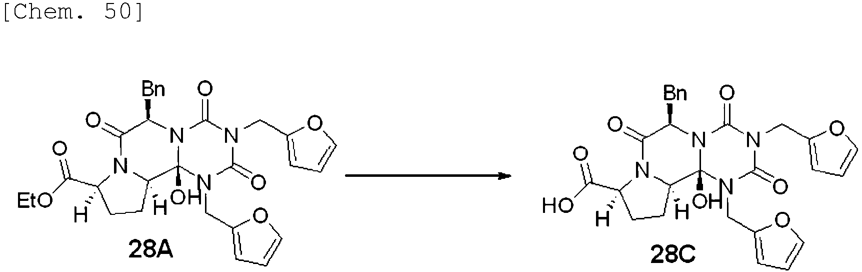

Compound 28A prepared in Example 44 (71.9 mg, 0.140 mmol) was added to a 25 ml egg-plant shaped flask, and was dissolved in tetrahydrofuran (0.368 ml), methanol (0.559 ml), and water (0.186 ml). Next, lithium hydroxide (58.6 mg, 1.397 mmol) was added thereto, and the mixture was stirred at room temperature for 3 hours. After the reaction was quenched with 1M hydrochloric acid aqueous solution, the mixture was extracted with ethyl acetate, and after distilling off the solvent, the desired compound 28C (yellowish-white solid) was obtained in an amount of 68 mg (0.140 mmol, 100%).

### Example 62-2 : the compound of Example 62

### (6R,9S,11aR,11bR)-1,3-bis(2-furylmethyl)-11b-hydroxy-6-isopropyl-2,4,7-trioxo-N-(3-quinolylmethyl)decahydro-2H-pyrrolo[2',1':3,4]pyrazino[1,2-a][1,3,5]triazine-9-carboxamide 28F

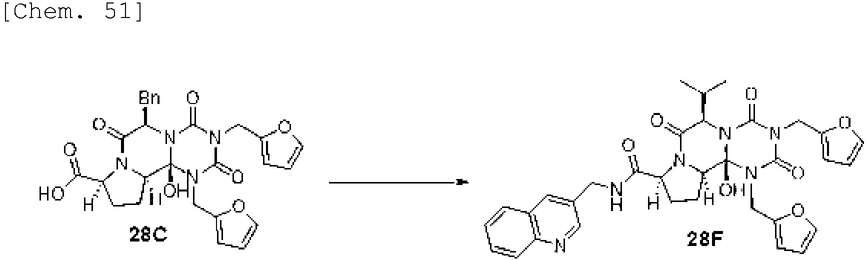

To compound 28C prepared in Example 62-1 (20 mg, 41 µmol) contained in a 10 ml egg-plant shaped flask, dichloromethane (1.64 ml), then HATU (31.3 mg, 82 µmol), C-quinoline-3-ylmethylamine dihydrochloride (28.2 mg, 0.118 mmol), and finally diisopropylethylamine (50.1 µl, 0.288 mol) were added, and the mixture was stirred at room temperature for 2 hours. After distilling off the solvent, the residue was purified using a silica gel column Q-Pack SI30 size 10 with a two-step gradient (chloroform:methanol = 100:0 to 95:5). After distilling off the solvent, the desired compound 28F (white solid) was obtained in an amount of 22.9 mg (36.5 µmol, 89%).

### Example 63

### (6R,9S,11aR,11bR)-1,3,6-Tribenzyl-11b-hydroxy-9-(((naphthalen-2-ylmethyl)amino)methyl)hexahydro-2H-pyrrolo[2',1':3,4]pyridazine[1,2-a][1,3,5]triazine-2,4,7(3H,6H)-trione 1F

### Example 63-1: compound 1C-1

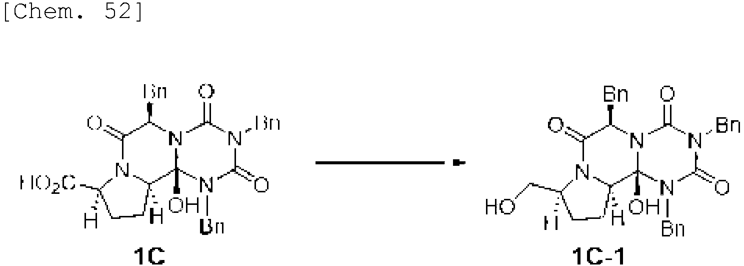

Compound 1C (101 mg, 0.183) prepared in Example 18-1 was added to a 25 ml egg-plant shaped flask, and was dissolved in the ultra-dehydrated tetrahydrofuran (1.00 ml). The solution was cooled to 0°C, and N-methylmorpholine (29 µl, 0.260 mmol) and isobutyl chloroformate (34.8 µl, 0.260 mmol) were added thereto, followed by stirring the mixture at 0°C for 30 minutes. Subsequently, a solution of sodium borohydride (53 mg, 1.40 mmol) dissolved in water (0.70 ml) was added thereto, and the mixture was stirred at 0°C for 1 hour. The reaction was stopped with a saturated ammonium chloride aqueous solution and the mixture was extracted with ethyl acetate. After distilling off the solvent, the residue was purified using a silica gel column Q-Pack SI30 size 20 with a two-stage elution (hexane: ethyl acetate = 20:80). After distilling off the solvent again, the desired compound 1C-1 (white solid) was obtained in an amount of 21.9 mg (40.5 µmol, 22%).

### Example 63-2 : the compound of Example 63

### (6R,9S,11aR,11bR)-1,3,6-Tribenzyl-11b-hydroxy-9-(((naphthalen-2-ylmethyl)amino)methyl)hexahydro-2H-pyrrolo[2',1':3,4]pyridazine[1,2-a][1,3,5]triazine-2,4,7(3H,6H)-trione 1F

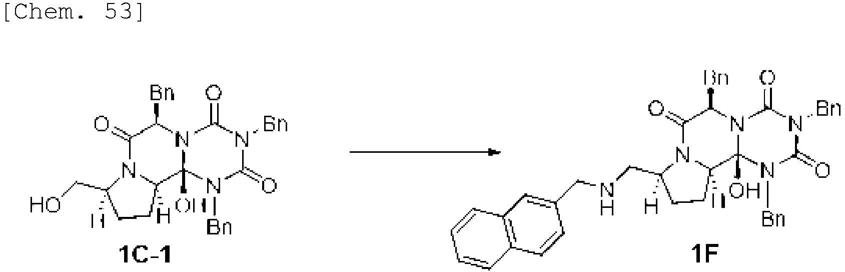

Compound 1C-1 prepared in the above example (7.1 mg, 13 µmol) was dissolved in dichloromethane (0.657 ml) in a 4 ml vial, and sodium bicarbonate (9.0 mg, 0.105 mmol) and Dess-Martin reagent (23.5 mg, 53 µmol), and the mixture was stirred at room temperature for 45 minutes. After distilling off the solvent, a saturated solution of sodium bicarbonate was added thereto and the mixture was extracted with ethyl acetate. After the mixture was dried with magnesium sulfate, the magnesium sulfate was filtered off, and after distilling off the solvent, the aldehyde, which was the desired intermediate, was obtained in an amount of 7.1 mg (13 µmol, 100%). To this material, 1-(2-naphthyl)methanamine (8.6 mg, 52 µmol) was added while washing with dichloroethane (0.80 ml), and the mixture was stirred at room temperature for 1 hour. After distilling off the solvent, the residue was purified using a silica gel column Q-Pack SI30 size 10 with a two-stage connection (chloroform: methanol = 98:2). After distilling off the solvent, the intermediate imine compound was obtained in an amount of 7.4 mg (11 µmol, 84%). This was dissolved in dichloroethane (0.546 ml), and triacetoxyborohydride (28.9 mg, 0.110 mmol) was added thereto and the mixture was stirred at room temperature for 15 hours. The reaction was stopped with a saturated sodium bicarbonate solution and the mixture was extracted with ethyl acetate. After distilling off the solvent, the residue was purified using CHROMATOREX DNH-30 size 10 (hexane: ethyl acetate = 90:10 to 0:100). After distilling off the solvent again, the desired compound 1F (white solid) was obtained in an amount of 5.4 mg (7.9 µmol, 73%).

The molecular weights, 1H-NMR, [M+H] or [M-H], and Retention Time (RT) of the compounds prepared in Examples 1 to 34 are summarized in Table 10.

**[Table 10-1]**

| Table 10 | | | | | |
|---|---|---|---|---|---|
| Exam. No. | Com. No. | MW | 1H-NMR δppm | [M+H] or [M-H] | RT (Min) |
| Example 1 | 1A | 582.66 | (CDCl₃); δ: 7.37-7.18 (13H. m). 7.09-7.02 (2H, m), 6.88 (1H, s). 5.53 (1H, dd. J = 10.4. 5.2 Hz), 4.83 (1H. d. J = 14.8 Hz), 4.81 (1H, d. J = 15.2 Hz), 4.73 (1H. d. J = 14.8 Hz), 4.64 (1H, d. J = 15.2 Hz), 4.60 (1H, d. J = 7.2 Hz), 4.38-4.23 (2H, m), 4.09-3.99 (1H, m), 3.53 (1H, dd, J = 13.6, 5,2 Hz), 3.17 (1H. dd, J = 13.6. 6.8 Hz), 2.12-1.99 (4H, m), 1.34 (3H, t, J = 7.2 Hz) | 581 | 1.35 |
| Example 1 | 1B | 564.64 | (CDCl₃); δ: 7.42-7.07 (13H. m), 6.68 (2H, d, J = 6.8 Hz), 5.28 (1H, dd, J = 10.4. 8.4 Hz), 5.08 (1H, d. J = 15.2 Hz), 4.88 (1H. d. J = 14.8 Hz), 4.77-4.71 (2H, m), 4.57 (1H, d. J = 15.6 Hz), 4.33-4.20 (2H, m), 2.98 (1H, dd. J = 13.6. 3.6 Hz), 2.87 (1H, ddd, J = 14.8, 12.0. 8.0 Hz), 2.68 (1H. dd. J = 13.8. 10.4 Hz), 2.63 (1H, dd, J = 15.0, 6.8 Hz). 2.35-2.17 (2H, m), 1.32 (3H, d. J = 7.2 Hz) | 565 | 1.35 |
| Table 2 | 2A | 506.56 | (CDCl₃); δ: 7.31-7.21 (10H, m), 6.92 (1H, s), 5.17 (1H, q. J = 6.8 Hz). 5.01-4.91 (2H, m), 4.76-4.66 (2H, m), 4.56 (1H, d. J = 7.2 Hz), 4.34-4.21 (2H, m). 4.06-4.00 (1H. m), 2.08-1.97 (4H, m), 1.65 (3H. d, J = 6.8 Hz), 1.32 (3H, t, J = 7.2 Hz) | 505 | 1.61 |
| Table 2 | 3A | 534.61 | (CDCl₃); δ: 7.43-7.16 (10H. m), 6.97 (1H, s). 5.01 (1H. d. J = 7.2 Hz), 4.97 (2H, s). 4.74 (1H, d. J = 14.8 Hz), 4.68 (1H. d. J = 15.2 Hz). 4.54 (1H, d. J = 7.2 Hz), 4.32-4.21 (2H, m), 3.98-3.94 (1H. m), 2.42-2.31 (1H, m), 2.09-1.92 (4H, m), 1.30 (3H. t, J = 6.8 Hz), 1.25 (3H. d. J = 6.8 Hz), 1.05 (3H, d. J = 6.8 Hz) | 533 | 1.32 |

**[Table 10-2]**

| | | | | | |
|---|---|---|---|---|---|
| Table 2 | 4A | 582.66 | (CDCl₃); δ: 7.37-7.18 (13H, m), 7.09-7.02 (2H, m), 6.88 (1H. s), 5.53 (1H. dd, J = 10.4, 5.2 Hz), 4.83 (1H, d. J = 14.8 Hz), 4.81 (1H, d. J = 15.2 Hz). 4.73 (1H, d. J = 14.8 Hz). 4.64 (1H. d. J = 15.2 Hz), 4.60 (1H, d. J = 7.2 Hz), 4.38-4.23 (2H. m), 4.09-3.99 (1H, m), 3.53 (1H, dd, J = 13.6, 5.2 Hz), 3.17 (1H, dd, J = 13.6, 6.8 Hz), 2.12-1.99 (4H, m), 1.34 (3H, t, J = 7.2 Hz) | 581 | 1.35 |
| Table 2 | 4B | 564.64 | (CDCl₃); δ: 7.42-7.07 (13H, m), 6.68 (2H, d, J = 6.8 Hz), 5.28 (1H, dd. J = 10.4, 8.4 Hz), 5.08 (1H, d. J = 15.2 Hz), 4.88 (1H, d. J = 14.8 Hz), 4.77-4.71 (2H, m), 4.57 (1H, d. J = 15.6 Hz), 4.33-4.20 (2H. m), 2.98 (1H. dd, J = 13.6, 3.6 Hz), 2.87 (1H, ddd. J = 14.8, 12.0. 8.0 Hz), 2.68 (1H, dd. J = 13.8, 10.4 Hz), 2.63 (1H. dd, J = 15.0, 6.8 Hz), 2.35-2.17 (2H. m), 1.32 (3H, d. J = 7.2 Hz) | 565 | 1.35 |
| Table 2 | 5A | 600.65 | (CDCl₃); δ: 7.36-7.20 (10H, m), 7.05-7.01 (2H, m), 6.99 (1H, s), 6.89 (2H, t, J = 8.8 Hz), 5.48 (1H, dd, J = 10.4, 5.2 Hz), 4.83 (1H, d. J = 15.6 Hz). 4.82 (1H, d. J = 14.8 Hz), 4.75 (1H, d. J = 14.8 Hz), 4.62 (1H. d. J = 15.2 Hz), 4.60 (1H, d. J = 7.2 Hz), 4.40-4.23 (2H. m), 4.06-4.01 (1H, m), 3.49 (1H, dd, J = 13.6, 5.2 Hz). 3.12 (1H, dd, J = 13.6, 6.4 Hz), 2.13-1.99 (4H, m), 1.35 (3H, t, J = 7.2 Hz) | 599 | 1.35 |
| Table 2 | 6A | 612.68 | (CDCl₃); δ: 7.38-7.32 (4H. m), 7.30-7.15 (6H, m), 7.03 (2H. dd, J = 8.0. 2.0 Hz). 6.90 (1H. s), 6.79 (2H, d, J = 8.8 Hz), 5.51 (1H, dd, J = 10.4, 5.2 Hz), 4.84 (1H, d. J = 14.8 Hz), 4.82 (1H. d, J = 15.6 Hz), 4.74 (1H, d. J = 14.8 Hz), 4.63 (1H, d. J = 15.2 Hz), 4.60 (1H, d. J = 7.2 Hz), 4.37-4.23 (2H, m), 4.06-4.00 (1H. m), 3.78 (3H, s), 3.48 (1H. dd, J = 14.0, 5.6 Hz), 3.11 (1H. dd, J = 14.0. 10.4 Hz), 2.13-2.01 (4H, m), 1.34 (3H, t, J = 7.2 Hz) | 611 | 1.32 |

**[Table 10-3]**

| | | | | | |
|---|---|---|---|---|---|
| Table 2 | 7A | 632.72 | (CDCl₃); δ: 7.83-7.70 (4H, m), 7.52-7.42 (3H. m), 7.34-7.25 (5H. m), 7.06 (1H, t, J = 7.6 Hz). 6.99 (1H, s), 6.93 (2H, t, J = 7.6 Hz). 6.87 (2H. d, J = 7.2 Hz), 5.68 (1H. dd. J = 10.4, 5.2 Hz), 4.78 (1H, d. J = 15.2 Hz), 4.75 (1H, d. J = 15.2 Hz), 4.68-4.61 (3H. m). 4.41-4.25 (2H, m), 4.07-4.01 (1H, m), 3.70 (1H, dd. J = 13.6, 5.6 Hz), 3.36 (1H, dd, J = 13.6, 10.4 Hz), 2.15-2.03 (4H, m), 1.36 (3H, t, J = 7.2 Hz) | 631 | 1.42 |
| Table 2 | 8A | 574.68 | (CDCla); δ: 7.43-7.21 (10H. m), 6.89 (1H, s), 5.26 (1H, dd, J = 9.6, 4.4 Hz), 5.00 (1H, d. J = 14.8 Hz), 4.96 (1H, d. J = 14.4 Hz), 4.75 (1H, d. J = 15.2 Hz), 4.68 (1H, d, J = 14.8 Hz), 4.55 (1H. d. J = 7.2 Hz), 4.39 (1H, d. J = 5.6 Hz), 4.33-4.19 (2H, m). 3.97 (1H. dd, J = 9.2, 6.4 Hz), 2.24-2.15 (1H, m), 2.10-1.86 (7H, m), 1.83-1.72 (1H, m), 1.66-1.45 (4H. m), 1.30-1.14 (2H, m) | 573 | 1.42 |
| Table 2 | 9B | 492.58 | (CDCl₃); δ: 7.37-7.14 (13H, m), 6.84 (2H. dd. J = 8.0, 1.6 Hz), 5.36 (1H, dd, J = 8.0. 4.8 Hz), 4.96 (1H. d. J = 14.4 Hz), 4.83 (1H. d. J = 14.4 Hz), 4.74 (1H, d. J = 16.0 Hz), 4.63 (1H, d. J = 16.0 Hz), 3.74 (1H, ddd, J = 12.0. 8.4, 3.6 Hz), 3.54 (1H. ddd, J = 11.6, 9.0, 7.4 Hz), 2.91 (1H, dd, J = 14.0. 4.4 Hz), 2.82 (1H, dd, J = 13.6, 8.0 Hz), 2.42-2.31 (2H, m), 2.00-1.7 (2H. m) | 493 | 1.32 |
| Table 2 | 11B | 598.7 | (CDCl₃); δ: 7.48-7.22 (12H, m), 7.22-7.12 (6H, m), 6.92 (2H. dd, J = 7.6, 1.6 Hz), 5.42 (1H, t, J = 6.0 Hz), 4.98 (1H, d, J = 14.4 Hz), 4.86 (1H, d. 16.4 Hz), 4.84 (1H, d, J = 14.4 Hz), 4.35 (1H, d. J = 16.4 Hz), 4.21 (2H, s), 3.99 (1H, t, J = 4.8 Hz), 3.89 (1H, d, 12.8 Hz), 3.56 (1H. dd. J = 12.8, 4.8 Hz). 2.94 (2H. d. J = 5.6 Hz), 2.48 (1H, d. J = 16.0 Hz), 2.26 (1H, dd, J = 16.0, 4.8 Hz) | 599 | 1.39 |

**[Table 10-4]**

| | | | | | |
|---|---|---|---|---|---|
| Table 2 | 12A | 524.62 | (CDCl₃); δ: 7.37 (2H. d. J = 1.4 Hz), 7.33-7.25 (7H, m), 7.16-7.09 (5H, m), 6.99 (2H, dd. J = 7.2, 2.6 Hz), 5.13 (1H, dd, J = 6.8, 2.8 Hz), 5.07 (1H. d. J = 14.4 Hz), 5.01 (1H, d. J = 14.0 Hz), 4.87 (1H. d. J = 15.6 Hz). 4.23 (1H, d. J = 15.2 Hz), 3.72 (1H. dd, J = 13.6, 2.8 Hz), 3.53-3.42 (2H, m), 3.19 (1H, dd, J = 13.6. 6.4 Hz), 2.19-2.09 (1H. m), 1.97-1.73 (2H. m), 1.67-1.62 (1H, dd, J = 10.8, 6.0 Hz) | 523 | 1.25 |
| Table 2 | 13B | 506.11 | (CDCl₃); δ: 7.37-7.13 (13H. m), 6.79 (2H. dd. J = 8.0.2.0 Hz), 5.22 (1H, dd. J = 8.4. 5.2 Hz), 4.94 (2H, d. J = 15.2 Hz), 4.85 (1H. d. J = 14.8 Hz). 4.58 (1H, d, J = 15.2 Hz), 4.24-4.16 (1H. m), 3.16-3.06 (1H. m), 2.71 (1H. dd. J = 14.0. 5.2 Hz), 2.58 (1H, dd, J = 14.0, 8.4 Hz), 2.54-2.46 (1H, m), 2.18-1.36 (5H, m) | 507 | 1.35 |
| Table 2 | 14A | 616.72 | (CDCl₃); δ: 7.49 (2H, d. J = 5.6 Hz), 7.36-7.21 (10H. m), 7.02-6.91 (6H. m), 6.79 (2H. d, J = 6.4 Hz), 5.81 (1H. br-s), 5.19 (1H, d. J = 14.4 Hz), 5.07 (1H. d. J = 14.4 Hz), 5.04 (1H. d. J = 18.4 Hz). 4.85 (1H, d. J = 5.6, 2.4 Hz), 4.35 (1H, d. J = 18.4 Hz). 3.88 (1H, d, J = 11.6 Hz), 3.85 (1H. d. J = 4.0 Hz), 3.72 (1H, d. J = 11.6 Hz). 3.54 (1H. d. J = 13.2 Hz), 3.43 (1H, dd, J = 15.6, 4.0 Hz), 3.39 (1H, dd. J = 13.6, 5.6 Hz), 3.06 (1H. dd. J = 14.0. 2.8 Hz), 2.47 (1H, dd. J = 9.2, 7.2 Hz), 1.98-1.88 (2H, m) | 615 | 1.35 |
| Table 2 | 15A | 616.72 | (CDCl₃); δ: 7.40-7.16 (18H. m), 7.09-7.05 (2H. m), 5.48 (1H, dd. J = 9.6, 4.8 Hz), 5.45 (1H. s), 4.87 (1H. d. J = 15.2 Hz), 4.85 (1H, d. J = 14.8 Hz). 4.78 (1H, d. J = 14.8 Hz), 4.67 (1H, d. J = 11.6 Hz), 4.55-4.45 (3H. m), 4.12-4.07 (1H. m), 3.99 (1H, dd, J = 8.8, 4.4 Hz), 3.54 (1H, dd. J = 13.6, 5.2 Hz), 3.20 (1H, dd. J = 13.6, 9.6 Hz), 3.12 (1H. dd, J = 12.8, 3.2 Hz), 2.22-2.06 (2H. m) | 615 | 1.35 |

**[Table 10-5]**

| | | | | | |
|---|---|---|---|---|---|
| Table 2 | 15B | 598.7 | (CDCl₃); δ: 7.38-7.10 (18H, m), 6.79 (2H. dd. J = 8.0, 1.6 Hz), 5.33 (1H. dd. J = 8.4, 4.8 Hz), 4.94 (1H. d. J = 14.4 Hz), 4.80 (1H, d. J = 14.8 Hz), 4.71 (2H, s), 4.47 (1H. d. J = 11.6 Hz), 4.42 (1H, d. J = 11.6 Hz), 4.17-4.08 (1H, m), 3.82 (1H, dd. J = 12.4. 4.0 Hz), 3.67 (1H. dd. J = 12.4. 6.0 Hz), 2.91 (1H. dd, J = 14.0. 4.8 Hz), 2.75 (1H, dd, J = 14.0, 8.8 Hz), 2.69-2.55 (2H. m) | 599 | 1.42 |
| Table 3 | 16B | 536.59 | (CDCl₃); δ: 7.44-7.26 (13H. m), 7.04 (2H, dd, J = 8.0. 1.6 Hz), 5.46 (1H. dd. J = 9.2. 4.8 Hz), 4.73 (1H. dd, J = 9.2, 1.2 Hz), 4.32-4.17 (2H. m), 3.32 (1H. dd. J = 14.0. 4.8 Hz), 3.25 (1H. dd, J = 14.0, 9.6 Hz), 2.24-2.12 (1H. m), 2.01-1.80 (3H, m), 1.31 (3H, t, J = 6.8 Hz) | 537 | 1.25 |
| Table 3 | 17A | 510.59 | (CDCl₃); δ: 7.38-7.13 (5H, m), 6.65 (1H, s), 5.45 (1H, dd, J = 10.4, 5.2 Hz), 4.59 (1H, d. J = 6.8 Hz), 4.40-4.25 (2H. m), 4.00 (1H. dd. J = 9.6, 5.6 Hz), 3.74 (1H, dd, J = 13.6. 6.4 Hz), 3.56-3.45 (3H. m), 3.17 (1H, dd, J = 13.6, 10.4 Hz), 3.03 (1H, dd, J = 13.6, 6.8 Hz), 2.21-1.96 (4H, m), 1.36 (3H. t. J = 7.2 HZ), 1.05-0.93 (1H, m), 0.61-0.05 (8H, m) | 509 | 1.32 |
| Table 3 | 18A | 594.7 | (CDCl₃); δ: 7.31-7.25 (3H, m), 7.20-7.12 (4H. m). 7.08 (1H. d. J = 2.4 Hz), 6.98 (1H. s), 6.96 (1H. dd. J = 5.2. 3.6 Hz), 6.91-6.83 (2H. m), 5.45 (1H. dd. J = 10.0, 5.2 Hz), 5.07 (1H, d. J = 15.2 Hz), 5.00 (1H, d. J = 14.4 Hz), 4.91 (1H, d. J = 14.8 Hz), 4.74 (1H. d. J = 15.6 Hz), 4.58 (1H, d. J = 7.2 Hz), 4.40-4.24 (2H. m), 3.96-3.90 (1H. m), 3.50 (1H. dd. J = 13.6, 4.8 Hz), 3.16 (1H. dd, J = 13.6. 2.4 Hz), 2.10-1.90 (4H. m), 1.36 (3H, t, J = 7.2 Hz) | 593 | 1.32 |

**[Table 10-6]**

| | | | | | |
|---|---|---|---|---|---|
| Example 18 | 1C | 554.6 | (CDCl₃); δ: 7.36-7.12 (14H, m), 7.09-6.98 (2H. m), 5.55-5.47 (1H. m), 4.85-4.67 (3H, m), 4.57-4.45 (2H, m). 3.93-3.82 (1H, m), 3.48-3.38 (1H, m). 3.19-3.07 (1H. m), 2.12-1.85 (4H. m) | not detected | not detected |
| Example 18 | 1D | 693.80 | (CDCl₃); δ: 8.18 (1H, s), 7.85-7.80 (3H. m), 7.75 (1H, s), 7.50-7.03 (18H, m), 6.56 (1H, br-t, J = 5.6 Hz), 5.52 (1H, dd. J = 10.0, 5.6 Hz), 4.84 (1H. d. J = 14.8 Hz), 4.80 (1H, d, J = 14.4 HZ), 4.73 (1H, d. J = 14.8 Hz), 4.68 (1H, d. J = 14.8 Hz), 4.71-4.56 (2H, m), 4.50 (1H. d, J = 5.2 Hz), 4.00 (1H. dd. J = 10.4. 5.6 Hz), 3.50 (1H. dd. J = 13.6. 5.6 Hz), 3.23 (1H. dd. J = 13.2, 10.0 Hz). 2.33-2.18 (1H, m), 2.10-1.97 (3H, m) | 692 | 1.85 |
| Table 4 | 2D | 617.71 | (CDCl₃); δ: 8.37 (1H, s), 7.84-7.80 (3H, m), 7.73 (1H, s), 7.51-7.21 (13H. m), 6.59 (1H. br-t, J = 6.0 Hz), 5.18 (1H, q. J = 7.2 Hz), 4.96 (2H. m), 4.78 (1H, d. J = 16.0 Hz). 4.69 (1H. d. J = 16.0 Hz), 4.61 (2H, m), 4.45 (1H. d. J = 6.8 Hz), 3.97 (1H, dd, J = 10.0, 5.2 Hz), 2.32-2.20 (1H, m), 2.03-1.92 (3H. m), 1.65 (3H, d. J = 6.8 Hz) | 616 | 1.74 |
| Table 4 | 3D | 645.76 | (CDCl₃); δ: 8.49 (1H, s), 7.81 (3H. d, J = 8.0 Hz), 7.71 (1H. s), 7.51-7.42 (4H, m), 7.36-7.20 (10H, m), 6.81 (1H, br-s), 4.99 (1H, d, J = 7.6 Hz), 4.96 (2H. s), 4.73 (2H, s), 4.60 (1H, dd. J = 15.2, 6.0 Hz), 4.54 (1H. dd. J = 14.8, 6.0 Hz), 4.48 (1H, d. J = 7.2 Hz), 3.94 (1H. dd. J = 10.4. 5.6 Hz), 2.38-2.28 (1H, m), 2.26-2.13 (1H. m), 2.06-1.87 (3H. m), 1.19 (3H. d, J = 6.4 Hz), 1.00 (3H, d. J = 6.8 Hz) | 644 | 1.49 |
| Table 4 | 4C | 554.60 | (CDCl₃); δ: 7.36-7.12 (14H. m), 7.09-6.98 (2H. m), 5.55-5.47 (1H. m), 4.85-4.67 (3H, m), 4.57-4.45 (2H, m), 3.93-3.82 (1H, m), 3.48-3.38 (1H, m). 3.19-3.07 (1H. m), 2.12-1.85 (4H. m) | not detected | not detected |

**[Table 10-7]**

| | | | | | |
|---|---|---|---|---|---|
| Table 4 | 4D | 693.80 | (CDCl₃); δ: 8.18 (1H, s), 7.85-7.80 (3H. m), 7.75 (1H. s), 7.50-7.03 (18H, m). 6.56 (1H, br-t, J = 5.6 Hz), 5.52 (1H, dd, J = 10.0. 5.6 Hz), 4.84 (1H, d. J = 14.8 Hz), 4.80 (1H, d. J = 14.4 HZ), 4.73 (1H, d. J = 14.8 Hz), 4.68 (1H. d. J = 14.8 Hz), 4.71-4.56 (2H. m), 4.50 (1H. d. J = 5.2 Hz), 4.00 (1H, dd, J = 10.4, 5.6 Hz), 3.50 (1H, dd, J = 13.6. 5.6 Hz), 3.23 (1H, dd. J = 13.2, 10.0 Hz). 2.33-2.18 (1H, m), 2.10-1.97 (3H, m) | 692 | 1.85 |
| Table 4 | 5D | 711.79 | (CDCl₃); δ: 8.43 (1H, s), 7.86-7.79 (2H. m), 7.75 (1H. s), 7.52-7.19 (14H, m), 7.03 (2H, d. J = 8.0 Hz), 6.87 (2H, t, J = 8.8 Hz). 6.51 (1H, br-t. J = 6.0 Hz), 5.48 (1H. dd. J = 10.8, 5.6 Hz), 4.85-4.58 (6H. m), 4.50 (1H, d, J = 6.8 Hz), 4.02 (1H, dd. J = 10.8, 6.0 Hz), 3.46 (1H. dd. J = 13.6, 5.2 Hz), 3.16 (1H, dd. J = 13.6, 10.8 Hz), 2.36-2.20 (1H, m), 2.11-1.98 (3H. m) | 710 | 1.46 |
| Table 4 | 6D | 723.83 | (CDCl₃); δ: 8.16 (1H, s), 7.85-7.80 (2H. m), 7.74 (1H. s), 7.52-7.45 (2H, m), 7.39-7.15 (12H. m), 7.04 (2H. d. J = 7.2 Hz), 6.75 (2H, d. J = 8.8 Hz), 6.55 (1H. br-t, J = 6.0 Hz), 5.50 (1H, dd, J = 10.4, 5.6 Hz), 4.88-4.57 (6H. m). 4.50-(1H, d. J = 7.2 Hz), 4.00 (1H, dd. J = 10.4. 6.0 Hz), 3.76 (3H. s), 3.45 (1H, dd. J = 13.6, 5.6 Hz), 3.17 (1H, dd. J = 13.6. 10.4 Hz). 2.35-2.18 (1H, m), 2.11-1.99 (3H, m) | 722 | 1.42 |
| Table 4 | 7D | 743.86 | (CDCl₃); δ: 8.35 (1H, s), 7.85-7.65 (6H. m), 7.74 (1H. s), 7.50-7.26 (12H, m), 7.06 (1H. t, J = 7.2 Hz), 6.94 (2H, t, J = 7.6 Hz), 6.88 (2H. d, J = 7.2 Hz), 6.59 (1H, br-s), 5.67 (1H. dd. J = 10.8, 5.6 Hz), 4.81-4.59 (6H. m). 4.52 (1H, d. J = 6.4 Hz), 4.01 (1H, dd. J = 10.4. 5.6 Hz). 3.66 (1H. dd. J = 14.0, 5.6 Hz). 3.41 (1H. dd. J = 13.6, 10.4 Hz), 2.38-2.21 (1H. m), 2.11-1.98 (3H, m) | 742 | 1.59 |

**[Table 10-8]**

| | | | | | |
|---|---|---|---|---|---|
| Table 4 | 8D | 685.83 | (CDCl₃); δ: 8.42 (1H. s), 7.84-7.78 (3H. m), 7.70 (1H, s), 7.51-7.20 (13H, m), 6.75 (br-t, J = 4.8 Hz), 5.25 (1H, dd, J = 9.6, 4.4 Hz), 4.98 (2H. s), 4.74 (2H, s), 4.64 (1H, dd, J = 14.4. 6.0 Hz), 4.52 (1H. dd. J = 15.2, 5.8 Hz), 4.48 (1H, d. J = 7.2 Hz). 2.25-1.70 (9H. m), 1.61-1.44 (4H. m), 1.22-1.07 (2H, m) | 684 | 1.59 |
| Table 4 | 17D | 621.74 | (CDCl₃); δ: 7.87 (3H. m), 7.77 (1H, s), 7.71 (1H. s), 7.53-7.46 (2H, m), 7.39 (1H, dd, J = 8.4, 1.8 Hz), 7.36 - 7.31 (2H, m), 7.27-7.20 (2H, m), 7.19-7.14 (1H, m), 6.52 (1H, br-s), 5.45 (1H, dd. J = 10.0. 5.2 Hz), 4.70 (1H, dd. J = 15.0, 5.8 Hz), 4.64 (1H. dd, J = 15.0, 5.6 Hz), 4.50 (1H, d, J = 5.6 Hz), 3.99 (1H, dd. J = 10.0, 5.2 Hz), 3.75 (1H, dd, J = 14.0, 6.4 Hz), 3.53 (2H, d, J = 7.2 Hz), 3.47 (1H, dd. J = 13.2, 5.2 Hz), 3.25 (1H, dd, J = 13.6. 10.0 Hz), 3.05 (1H, dd, J = 13.6, 7.2 Hz). 2.28-2.16 (1H. m), 2.13-2.02 (3H, m), 1.42-1.32 (1H, m), 1.06-0.96 (1H. m), 0.59-0.08 (8H, m) | 620 | 1.46 |
| Table 4 | 18D | 705.85 | (CDCl₃); δ: 8.24 (1H, s), 7.86-7.80 (3H. m), 7.75 (1H, s), 7.53-7.43 (2H, m), 7.38 (1H. dd. J = 8.4, 1.6 Hz), 7.28-7.23 (3H, m), 7.16-7.11 (4H, m). 7.06 (1H. d. J = 3.2 Hz), 6.94 (1H. dd. J = 5.2, 3.6 Hz), 6.90 (1H, dd. J = 3.2, 1.2 Hz), 6.68 (1H. dd. J = 5.2, 3.6 Hz), 6.70 (1H, br-t, J = 6.0 Hz). 5.43 (1H, dd. J = 10.0, 5.2 Hz). 5.07 (1H, d. J = 14.8 Hz), 5.02 (1H, d. J = 15.2 Hz). 4.93 (1H, d. J = 15.2 Hz), 4.76 (1H, d. J = 15.2 Hz), 4.69-4.57 (2H. m), 4.51-4.45 (1H, m), 3.90 (1H. dd. J = 10.4. 6.0 Hz), 3.44 (1H, dd, J = 13.6, 5.2 Hz), 3.21 (1H, dd. J = 13.6, 10.0 Hz). 2.28-2.13 (1H, m), 2.08-1.89 (3H, m) | 704 | 1.42 |

**[Table 10-9]**

| | | | | | |
|---|---|---|---|---|---|
| Example 28 | 16E | 647.74 | (CDCl₃); δ: 7.84-7.77 (4H. m), 7.51-7.21 (14H. m), 7.15 (2H, dd, J = 8.8, 1.2 Hz), 7.09 (2H. dd. J = 8.4. 1.6 Hz), 6.98 (1H, t. J = 5.8 Hz), 5.48 (1H. t, J = 6.8 Hz). 4.70 (1H. d. J = 8.0 Hz), 4.65 (2H, d. J = 6.0 Hz), 3.18 (2H. d, J = 6.8 Hz), 2.23 (1H. dd, J = 11.2, 6.4 Hz), 2.07-1.87 (2H, m), 1.82-1.75 (1H. m) | 648 | 1.32 |
| Example 29 | 1E | 675.79 | (CDCl₃); δ: 7.81-7.75 (3H, m), 7.74 (1H, s), 7.49-7.03 (16H, m). 6.60 (2H, d. J = 5.6 Hz), 5.29 (1H, dd. J = 9.2, 4.8 Hz), 4.95 (1H, d. 15.6 Hz), 4.92 (1H, d, J = 14.4 Hz), 4.77 (1H, d. J = 14.4 Hz), 4.71 (1H, d, J = 8.4 Hz). 4.69-4.57 (2H. m), 4.58 (1H, d, J = 15.6 Hz), 3.03-2.90 (1H. m), 2.76 (1H. dd, J = 14.0, 4.8 Hz), 2.60-2.44 (3H, m), 2.10-1.93 (1H, m) | 676 | 1.82 |
| Table 5 | 2E | 599.69 | (CDCl₃); δ: 7.84-7.75 (3H, m), 7.66 (1H, s), 7.50-7.14 (13H, m), 7.00 (1H, br-t, J = 6.0 Hz), 5.36 (1H, d, J = 15.2 Hz), 5.05 (1H, q. J = 7.2 Hz), 5.02 (2H. m). 4.69 (1H. d. J = 8.0 Hz), 4.66 (1H. dd, J = 14.4. 6.4 Hz), 4.45 (1H, dd, J = 14.8, 5.2 Hz), 4.39 (1H, d. J = 15.2 Hz), 3.04-2.95 (1H. m), 2.65 (1H, dd, J = 14.4. 7.6 Hz), 2.54 (1H, dd, J = 12.4, 7.6 Hz), 2.10-1.99 (1H, m), 0.84 (3H. d, J = 7.2 Hz) | 600 | 1.75 |
| Table 5 | 4E | 675.79 | (CDCl₃); δ: 7.81-7.75 (3H, m), 7.74 (1H. s), 7.49-7.03 (16H, m), 6.60 (2H, d, J = 5.6 Hz), 5.29 (1H, dd. J = 9.2, 4.8 Hz). 4.95 (1H, d. 15.6 Hz), 4.92 (1H, d. J = 14.4 Hz), 4.77 (1H, d. J = 14.4 Hz), 4.71 (1H, d. J = 8.4 Hz), 4.69-4.57 (2H. m), 4.58 (1H, d, J = 15.6 Hz), 3.03-2.90 (1H, m), 2.76 (1H, dd. J = 14.0. 4.8 Hz), 2.60-2.44 (3H. m), 2.10-1.93 (1H, m) | 676 | 1.82 |

**[Table 10-10]**

| | | | | | |
|---|---|---|---|---|---|
| Table 5 | 5E | 693.78 | (CDCl₃); δ: 7.81-7.75 (3H, m), 7.74 (1H, s), 7.49-7.23 (10H. m), 7.16-7.13 (2H, m). 6.97 (1H, t. J = 5.6 H), 6.70 (2H, t, J = 8.8 Hz), 6.45-6.38 (2H, m), 5.20 (1H. dd. J = 10.4. 4.8 Hz), 5.15 (1H, d. J = 15.2 Hz), 4.89 (1H, d, J = 14.8 Hz), 4.75 (1H, d. J = 14.8 Hz), 4.73-4.66 (2H. m), 4.56 (1H, dd. J = 14.8, 5.2 Hz), 4.50 (1H, d. J = 15.2 Hz), 3.17-3.04 (1H, m), 2.69-2.60 (2H. m), 2.54-2.47 (1H. m), 2.39 (1H. dd, J = 14.0, 10.4 Hz), 2.13-2.01 (1H. m) | 694 | 1.42 |
| Table 5 | 7E | 725.85 | (CDCl₃); δ: 7.80-7.72 (4H. m), 7.59 (2H, d, J = 8.8 Hz), 7.47-7.36 (4H, m), 7.35-7.25 (6H. m), 7.20-7.09 (8H, m), 6.90 (1H, dd. J = 8.4, 2.0 Hz), 5.40 (1H. dd, J = 8.4, 5.2 Hz), 4.88 (1H, d. J = 14.8 Hz), 4.72 (1H, d. J = 14.4 Hz), 4.71 (1H, d. J = 8.0 Hz), 4.69-4.57 (2H. m), 4.54 (1H. d, J = 15.6 Hz), 4.43 (1H, d. J = 16.0 Hz), 2.93-2.82 (2H, m), 2.77 (1H. dd. J = 14.0. 8.4 Hz), 2.45 (2H, td, J = 15.2, 7.6 Hz), 2.04-1.92 (1H, m) | 726 | 1.49 |
| Table 5 | 8E | 667.81 | (CDCl₃); δ: 7.82-7.75 (3H, m), 7.68 (1H, s), 7.50-7.44 (2H, m), 7.38 (2H, dd. J = 8.0, 1.6 Hz), 7.34-724 (7H, m), 7.22-7.16 (2H. m), 7.07 (1H. t, J = 5.6 Hz), 5.39 (1H. d. J = 15.2 Hz), 5.06 (1H, d, J = 14.4 Hz), 4.99 (1H, d, J = 15.2 Hz), 4.95 (1H, dd. J = 9.2, 5.6 Hz), 4.72 (1H, d, J = 8.4 Hz), 4.67 (1H. dd, J = 14.8, 6.8 Hz), 4.44 (1H, dd, J = 14.8, 5.2 Hz), 4.34 (1H, d, J = 15.2 Hz), 3.08-2.97 (1H, m), 2.67 (1H, dd, J = 14.4. 7.6 Hz), 2.58 (1H, dd, J = 12.4, 7.6 Hz), 2.58 (1H, dd, J = 12.4, 7.6 Hz), 2.12-2.00 (1H, m), 1.68-1.52 (1H, m). 1.47-1.23 (6H, m). 1.18-1.05 (2H, m). 0.97-0.86 (1H. m), 0.61-0.49 (1H. m) | not detected | not detected |

| | | | | | |
|---|---|---|---|---|---|
| Eam.No: Example Number Com N.: Compound Number MW: Molecular Weight | | | | | |

The molecular weights, 1H-NMR, [M+H] or [M-H], and Retention Time (RT) of the compounds from Examples 35 to 63 are summarized in Table 11.
The elution conditions:
Flow rate: 0.9 mL/min, Mobile phase A = 0.05% (v/v) formic acid aqueous solution, Mobile phase B = 0.05% (v/v) formic acid-containing acetonitrile;
0-0.9 minutes, Linear gradient A:B (95:5) - A:B (10:90), 0.9-3 minutes A:B (10:90)

**[Table 11-1]**

| Table 11 | | | | | |
|---|---|---|---|---|---|
| Exam . No. | Com. No. | MW | 1H-NMR δ ppm | [M+H] or [M-H] | RT (min) |
| Exam .3 | 3B | 516.60 | (CDCl₃); δ: 7.41-7.21 (10H. m), 5.35 (1H, d, J = 15.6 Hz), 5.07 (1H, d. J = 14.4 Hz), 5.02 (1H, d, J = 14.4 Hz), 4.72 (1H, dd, J = 9.2, 1.2 Hz), 4.41 (1H, d. J = 15.6 Hz), 4.26-4.13 (2H, m), 2.86-2.75 (1H, m), 2.65 (1H, dd, J = 14.8, 7.6 Hz), 2.35-2.23 (1H, m), 2.18 (1H, dd. J = 13.2, 3.6 Hz), 1.27 (3H. t, J = 7.2 Hz), 0.86 (3H, d. J = 6.8 Hz). 0.55 (3H. d. J = 6.8 Hz) | 517 | 1.32 |
| Table 7 | 19A | 524.62 | (CDCl₃); δ: 7.40-7.23 (9H, m), 7.18-7.06 (5H.m), 7.02-6.97 (2H, m), 5.13 (1H, dd, J = 6.4. 2.8 Hz), 5.07 (1H, d, J = 14.4 Hz), 5.01 (1H, d, J = 14.4 Hz), 4.88 (1H, d, J = 15.6 Hz), 4.23 (1H, d. J = 15.6 Hz), 3.72 (1H, dd. J = 13.6, 2.8 Hz), 3.53-3.41 (2H. m), 3.19 (1H, dd. J = 13.6, 6.4 Hz), 2.19-2.09 (1H, m), 1.97-1.73 (2H, m), 1.65 (1H, dd. J = 11.2. 6.4 Hz). 0.98 (3H. s) | 525 | 1.25 |
| Table 7 | 20A | 554.6 | (CDCl₃); δ: 7.33-7.21 (13H, m), 7.14 (2H, d, J = 7.2 Hz), 6.34 (1H, s), 5.34 (1H, t, J = 6.4 Hz), 5.15 (1H, d. J = 16.8 Hz), 4.99 (1H, d, J = 14.4 Hz), 4.84 (1H, d. J = 14.4 Hz), 4.56-4.44 (3H, m), 3.87 (3H, s), 3.46 (1H. dd, J = 13.6, 6.0 Hz), 3.27 (1H. dd. J = 13.6, 7.2 Hz), 2.10 (1H, dt, J = 12.8, 6.0 Hz), 1.82 (1H. dt. J = 12.8, 7.6 Hz) | 553 | 1.25 |

**[Table 11-2]**

| | | | | | |
|---|---|---|---|---|---|
| Table 7 | 20C | 540.58 | (CDC13); δ: 7.31-6.96(15H. m), 5.28 (1H, brs), 4.92 (2H, br-d, J = 14.4 Hz), 4.73 (1H, br-d, J = 14.4 Hz), 4.45 (1H. br-d, J = 15.6 Hz), 4.27 (1H, brs), 4.14 (1H, br-s), 3.18 (1H, br-s), 3.00 (1H, brs), 1.98 (1H, br-s), 1.61 (1H. brs) | 539 | 1.15 |
| Table 7 | 21C | 566.61 | No data | 565 | 1.15 |
| Table 7 | 22A | 582.66 | (CDCl₃); δ: 7.48-7.44 (2H, m), 7.35-7.18 (9H, m), 7.09-7.06 (2H, m), 6.96 (2H. d, J = 6.8 Hz), 5.37 (1H. s), 5.12 (1H, d, J = 14.4 Hz), 5.06 (1H, d, J = 14.4 Hz), 4.89 (1H, dd, J = 7.2, 3.2 Hz), 4.67 (1H, d, J = 18.0 Hz), 4.51 (1H. d, J = 18.0 Hz), 4.39 (1H. t, J = 7.2 Hz), 4.29-4.13 (2H, m), 3.30 (1H, dd, J = 14.0, 7.2 Hz), 3.03 (1H, dd, J = 14.0, 3.2 Hz), 2.94 (1H, t, J = 7.2 Hz), 2.13-1.99 (2H. m), 1.80-1.69 (1H, m), 1.61-1.50 (1H, m), 1.28 (3H, t, J = 7.2 Hz) | 583 | 1.29 |
| Table 7 | 23A | 594.7 | (CDCl₃); δ: 7.30-7.25 (3H, m), 7.20-7.11 (4H, m), 7.08 (1H. d, J = 2.8 Hz), 6.98 (1H, s), 6.98-6.92 (1H, m), 6.90-6.83 (2H, m), 5.45 (1H, dd, J = 10.4, 4.8 Hz), 5.07 (1H, d. J = 15.2 Hz), 5.00 (1H. d, J = 14.8 Hz), 4.91 (1H, d, J = 14.8 Hz), 4.74 (1H, d. J = 15.2 Hz), 4.58 (1H. d. J = 7.2 Hz), 4.41-4.23 (2H. m), 3.93 (1H, dd, J = 9.6, 6.0 Hz), 3.50 (1H, dd. J = 13.6, 4.8 Hz), 3.16 (1H. dd. J = 13.6. 10.4 Hz). 2.11-1.90 | 593 | 1.29 |

**[Table 11-3]**

| | | | | | |
|---|---|---|---|---|---|
| | | | (4H. m), 1.36 (3H. t, J = 7.2 Hz) | | |
| Table 7 | 24A | 558.64 | (CDCl₃); δ: 7.97 (1H, d. J = 8.0 Hz), 7.37-7.23 (10H, m), 7.19-7.03 (8H, m), 5.65 (1H, dd, J = 6.4, 5.2 Hz), 5.08 (1H, d, J = 15.6 Hz), 5.01 (2H, s), 4.40 (1H, d, J = 16.0 Hz), 4.38-4.33 (1H, m), 3.56 (1H, dd, J = 14.0, 5.3 Hz), 3.39 (1H, dd, J = 14.4, 11.2 Hz), 3.35 (1H, dd, J = 14.0, 6.8 Hz), 2.98 (1H, dd, J = 14.8, 8.0 Hz), 2.05 (1H, m) | 559 | 1.32 |
| Table 7 | 25A | 582.66 | (CDCl₃); δ: 7.37-7.16 (13H, m), 7.01-6.95 (2H, m), 6.50 (1H, s), 5.54 (1H, dd, J = 10.8, 5.2 Hz), 5.04 (1H, dd, J = 6.4, 2.6 Hz), 4.90 (1H, d, J = 15.2 Hz), 4.83 (1H, d, J = 14.8 Hz), 4.74 (1H, d, J = 14.8 Hz), 4.57 (1H, d, J = 15.2 Hz), 3.92-3.83 (1H, m), 3.86 (3H, s), 3.59 (1H, dd, J = 13.6, 5.2 Hz), 3.23 (1H, dd, J = 13.6, 10.8 Hz), 2.37-2.25 (1H, m), 2.06-1.93 (1H, m), 1.89-1.72 (2H, m), 1.69-1.53 (2H, m) | 583 | 1.29 |
| Table 7 | 26A | 534.61 | (CDCl₃); δ: 7.43-7.16 (10H, m), 6.97 (1H, s), 5.02 (1H, d, J = 7.2 Hz), 4.97 (2H, s), 4.74 (1H, d, J = 14.8 Hz), 4.68 (1H, d, J = 15.2 Hz), 4.54 (1H, d, J = 7.2 Hz), 4.32-4.21 (2H, m), 3.98-3.94 (1H. m), 2.42-2.31 (1H, m), 2.09-1.92 (4H. m), 1.30 (3H, t, J = 6.8 Hz), 1.25 (3H, d, J = 6.8 Hz), 1.05 (3H, d, J = 6.8 Hz) | 533 | 1.32 |

**[Table 11-4]**

| | | | | | |
|---|---|---|---|---|---|
| Table 7 | 27A | 546.66 | (CDCl₃); δ: 7.21 (2H, ddd, J = 6.0, 4.8, 1.2 Hz), 7.09 (2H, d, J = 3.6 Hz), 7.04 (1H, s), 6.92 (2H, ddd. J = 5.2, 3.2, 1.2 Hz), 5.19 (1H, d, J = 15.2 Hz), 5.13 (1H, d, J = 15.2 Hz), 5.06 (1H, d, J = 15.2 Hz), 5.02 (1H, d, J = 6.8 Hz), 4.73 (1H, d, J = 14.8 Hz), 4.53 (1H, d, J = 7.6 Hz), 4.36-4.22 (2H. m), 3.91 (1H, dd, J = 9.2, 7.2 Hz), 2.42-2.31 (1H, m), 2.09-1.85 (4H, m), 1.33 (3H, t, J = 6.8 Hz), 1.22 (3H, d, J = 6.8 Hz), 1.01 (3H, d, J = 6.8 Hz) | 545 | 1.29 |
| Table 7 | 28A | 514.54 | (CDCl₃); δ: 7.34-7.32 (2H, m), 6.83 (1H, s), 6.33 (2H, d, J = 1.6 Hz), 6.30 (2H, d, J = 1.6 Hz), 5.06 (1H, d, J = 15.2 Hz), 5.00 (1H, d, J = 7.6 Hz), 4.99 (1H, d, J = 15.2 Hz), 4.87 (1H. d, J = 16.0 Hz), 4.66 (1H, d, J = 16.0 Hz), 4.52 (1H, d, J = 7.2 Hz), 4.34-4.21 (2H, m), 3.94 (1H, dd. J = 10.0, 6.0 Hz), 2.40-2.27 (1H, m), 2.12-1.76 (4H, m), 1.32 (3H, t, J = 7.2 Hz), 1.23 (3H, d, J = 6.4 Hz), 1.00 (3H, d, J = 6.8 Hz) | 515 | 1.18 |
| Table 7 | 29A | 520.59 | (CDCl₃); δ: 7.43-7.18 (10H, m), 6.92 (1H, s), 5.14 (1H, dd, J = 9.6, 5.2 Hz), 4.97 (2H, s), 4.73 (1H, d, J = 15.2 Hz), 4.69 (1H, d, J = 15.2 Hz), 4.56 (1H, d, J = 7.6 Hz), 4.33-4.17 (2H, m), 3.99 (1H, dd, J = 9.2, 6.4 Hz), 2.33-2.19 (1H, m), 2.13-1.85 (5H, m), 1.32 (3H, t, J = 7.2 Hz), 1.07 (3H, t, J = 7.2 Hz) | 521 | 1.22 |
| Table 7 | 30A | 548.64 | (CDCl₃); δ: 7.43-7.22 (10H, m), 6.91 (1H, s), 5.27 (1H, dd, J = 9.6, 4.4 Hz), 4.98 (2H, s), 4.74 (1H. d. J = 14.8 Hz), 4.68 (1H, d, J = 14.8 Hz), 4.55 (1H, d, J = 7.2 Hz), 4.34-4.19 (2H, m), 3.96 | 549 | 1.32 |

**[Table 11-5]**

| | | | | | |
|---|---|---|---|---|---|
| | | | (1H, dd, J = 9.6, 6.4 Hz), 2.09-1.76 (7H, m), 1.32 (3H, t, J = 7.2 Hz), 1.02 (3H, d. J = 6.4 Hz), 0.98 (3H, d, J = 6.8 Hz) | | |
| Table 8 | 20D | 679.78 | (CDCl₃); δ: 8.76 (1H, s), 7.84-7.78 (3H, m), 7.72 (1H, s), 7.53-7.45 (2H, m), 7.38-7.16 (16H, m), 5.36 (1H, dd, J = 8.0, 6.0 Hz), 5.14 (1H, d, J = 16.4 Hz), 4.98 (1H, d, J = 14.8 Hz), 4.80 (1H, d, J = 14.4 Hz), 4.67-4.49 (3H, m), 4.43-4.30 (2H, m), 3.46 (1H, dd, J = 13.6, 10.0 Hz), 3.27 (1H, dd, J = 13.6, 8.0 Hz), 2.17-2.06 (1H, m), 1.83-1.73 (1H, m) | 678 | 1.42 |
| Table 8 | 21D | 705.82 | (CDCl₃); δ: 8.40 (1H, s), 7.82-7.71 (3H, m), 7.68 (1H, s), 7.49-7.42 (2H, m), 7.36-7.07 (15H, m), 6.41 (1H, t, J = 6.0 Hz), 5.01 (1H, d, J = 17.2 Hz), 4.98 (1H, d, J = 14.4 Hz), 4.93 (1H, d, J = 14.4 Hz), 4.58 (1H, d, J = 17.2 Hz), 4.56 (1H, dd, J = 14.8, 6.0 Hz), 4.39 (1H, dd, J = 14.8, 5.6 Hz), 4.21 (1H, br-t, J = 4.0 Hz), 4.14 (1H, d, J = 16.0 Hz), 4.07 (1H, dd. J = 10.8, 6.0 Hz), 3.69 (1H, d, J = 16.4 Hz), 3.61 (1H, d, J = 16.4 Hz), 3.18 (1H, d, J = 16.0 Hz), 2.38-2.24 (1H, m), 1.79-1.67 (3H, m) | 704 | 1.39 |
| Table 8 | 26D | 645.76 | (CDCl₃); δ: 8.52 (1H, s), 7.81 (3H, d, J = 8.0 Hz), 7,71 (1H, s), 7.51-7.42 (4H, m), 7.36-7.20 (10H, m), 6.81 (1H, br-s), 4.99 (1H, d, J = 7.6 Hz), 4.96 (2H, s), 4.73 (2H, s), 4.60 (1H, dd, J = 14.8, 6.0 Hz), 4.54 (1H, dd, J = 14.8, 6.0 Hz), 4.48 (1H, d, J = 7.2 Hz), 3.94 (1H, dd, J = 10.4, 5.6 Hz), 2.38-2.28 (1H, | 644 | 1.49 |

**[Table 11-6]**

| | | | | | |
|---|---|---|---|---|---|
| | | | m), 2.26-2.13 (1H, m), 2.06-1.87 (3H, m), 1.19 (3H, d. J = 6.4 Hz), 1.00 (3H, d, J = 6.8 Hz) | | |
| Table 8 | 27D | 657.8 | (CDCl₃); δ: 7.84-7.78 (3H, m), 7.71 (1H, s), 7.50-7.43 (2H, m), 7.36 (1H, dd, J = 8.4, 1.6 Hz), 7.23 (1H, dd, J = 4.8, 1.2 Hz), 7.17 (1H, dd, J = 5.2, 1.2 Hz), 7.17-7.12 (1H, m), 7.11 (1H, dd. J = 3.6, 1.2 Hz), 7.00 (1H, dd, J = 3.6, 1.2 Hz), 6.95 (1H, dd, J = 5.2, 3.6 Hz), 6.91 (1H, dd, J = 5.2, 3.6 Hz), 5.21 (1H, d, J = 16.0 Hz), 5.15 (2H, s), 4.96 (1H, d, J = 8.0 Hz), 4.68-4.50 (4H, m), 3.89 (1H, dd, J = 8.4, 7.6 Hz), 2.32-2.17 (3H, m), 2.13-2.03 (2H. m), 1.27 (3H, d, J = 6.8 Hz), 0.90 (3H, d, J = 6.8 Hz) | 658 | 1.25 |
| Table 8 | 28D | 625.68 | (CDCl₃); δ: 8.39 (1H, br-s), 7.85-7.78 (3H, m), 7.71 (1H, s), 7.53-7.42 (2H, m), 7.36-7.29 (3H, m), 6.96 (1H, t, J = 6.0 Hz), 6.35-6.27 (4H. m), 5.06 (1H, d, J = 15.2 Hz), 4.98 (1H, d, J = 15.2 Hz), 4.98 (1H, d, J = 7.6 Hz), 4.88 (1H, d. J = 16.0 Hz), 4.61 (1H, dd, J = 14.8, 6.4 Hz), 4.51 (1H, dd, J = 14.8, 5.6 Hz), 4.47 (1H, d, J = 7.2 Hz), 3.92 (1H, dd, J = 10.4, 6.4 Hz), 2.37-2.24 (1H, m), 2.20-1.87 (3H, m), 1.85-1.75 (1H, m), 1.15 (3H, d, J = 6.4 Hz), 0.94 (3H, d, J = 6.8 Hz) | 624 | 1.32 |

**[Table 11-7]**

| | | | | | |
|---|---|---|---|---|---|
| Table 8 | 29C | 492.53 | (CDCl₃); δ: 7.40-7.21 (10H, m), 5.09 (1H, brs), 5.00 (1H, brd, J = 14.8 Hz), 4.95 (1H, brd, J = 14.4 Hz), 4.38 (1H, br-s), 3.84 (1H, br-s), 2.23-1.80 (6H, m), 1.01 (3H, br-t, J = 7.2 Hz) | 493 | 1.12 |
| Table 8 | 29D | 631.73 | (CDCl₃); δ: 8.47 (1H, s), 7.85-7.79 (3H, m), 7.70 (1H, s), 7.52-7.44 (2H, m), 7.42 (2H, d, J = 7.2 Hz), 7.36-7.20 (9H. m), 6.88-6.81 (1H, m), 5.12 (1H, dd, J = 10.0, 4.8 Hz), 4.99 (1H, d, J = 14.4 Hz), 4.94 (1H, d, J = 14.4 Hz), 4.76 (1H, d, J = 15.2 Hz), 4.71 (1H, d, J = 15.2 Hz), 4.64 (1H, dd, J = 14.4, 6.4 Hz), 4.50 (1H, dd, J = 14.4, 5.2 Hz), 4.48 (1H, d, J = 7.2 Hz), 3.96 (1H, dd, J = 10.4, 6.0 Hz), 2.30-2.10 (2H, m), 2.07-1.87 (4H, m), 1.01 (3H, t, J = 7.2 Hz) | 630 | 1.35 |
| Table 9 | 25E | 689.82 | (CDCl₃); δ: 7.85-7.78 (3H, m), 7.73 (1H, s), 7.51-7.43 (2H, m), 7.38 (1H, dd, J = 8.4, 1.6 Hz), 7.32-7.05 (12H, m), 6.76-6.70 (2H, m), 6.41 (1H, t, J = 5.6 Hz), 5.24 (1H, dd, J = 8.4, 6.0 Hz), 4.94 (1H, d, J = 14.8 Hz), 4.92 (1H, d, J = 15.6 Hz), 4.89 (1H, dd, J = 6.4, 4.0 Hz), 4.83 (1H, d, J = 14.8 Hz), 4.67 (1H, dd, J = 14.8, 6.0 Hz), 4.58 (1H, dd, J = 14.8, 6.0 Hz), 4.55 (1H, d, J = 15.2 Hz), 2.64 (1H, dd, J = 14.0, 6.0 Hz), 2.57-2.37 (3H, m), 2.27-2.17 (1H, m), 2.06-1.93 (1H, m), 1.93-1.82 (1H, m), 1.60-1.47 (1H, m) | 690 | 1.39 |

**[Table 11-8]**

| | | | | | |
|---|---|---|---|---|---|
| Exam . 54 | 18F | 579.69 | (CDCl₃); δ: 8.60 (1H, s), 7.32-7.23 (3H, m), 7.20-7.08 (5H, m), 6.95 (1H, dd, J = 5.2, 3.2 Hz), 6.90-6.84 (2H, m), 6.74-6.68 (1H, m), 5.43 (1H, dd, J = 10.4, 5.2 Hz), 5.07 (1H, d, J = 15.2 Hz), 5.02 (1H, d, J = 15.2 Hz), 4.92 (1H, d, J = 14.8 Hz), 4.78 (1H. d, J = 15.2 Hz), 4.45 (1H, d, J = 7.2 Hz), 3.90 (1H, dd, J = 10.4, 6.4 Hz), 3.45 (1H, dd, J = 13.6, 5.2 Hz), 3.22 (1H, dd, J = 13.6, 10.4 Hz), 2.87 (3H, d, J = 4.8 Hz), 2.24-2.12 (1H, m), 2.06-1.84 (3H, m) | not detected | not detected |
| Exam . 55 | 18G | 608.73 | (CDCl₃); δ: 7.29-7.24 (3H, m), 7.19-7.13 (4H. m), 7.09 (1H. s), 7.09-7.06 (1H. m), 6.96 (1H, dd, J = 5.2, 3.6 Hz), 6.89-6.84 (2H, m), 5.44 (1H, dd, J = 10.4, 5.2 Hz), 5.13 (1H, quin, J = 6.4 Hz), 5.07 (1H, d, J = 15.2 Hz), 5.00 (1H, d, J = 14.8 Hz), 4.91 (1H, d, J = 14.8 Hz), 4.74 (1H, d, J = 15.6 Hz), 4.54 (1H, d, J = 7.2 Hz), 3.96-3.90 (1H, m), 3.50 (1H, dd, J = 13.6, 5.2 Hz), 3.16 (1H, dd, J = 13.6, 10.4 Hz), 2.10-1.90 (4H, m), 1.37 (3H, d, J = 6.4 Hz), 1.31 (3H, d, J = 6.0 Hz) | 607 | 1.35 |
| Exam . 55 | 18H | 721.89 | (CDCl₃); δ: 10.19 (1H. br-s), 8.22 (1H, brs), 7.30-7.20 (3H, m), 7.19-7.07 (5H, m), 6.96 (1H, dd, J = 5.2, 3.6 Hz), 6.90-6.82 (2H, m), 5.37 (1H, dd, J = 9.6, 5.2 Hz), 5.07 (1H, d, J = 15.2 Hz), 5.00 (1H, d. J = 15.2 Hz), 4.92 (1H, d, J = 14.8 Hz), 4.78 (1H, d, J = 15.2 Hz), 4.05 (1H, brd, J = 13.6 Hz), 3.98-3.93 (1H, m), 3.39 (2J, dd, J = 13.4, 5.3 Hz), 3.34-3.17 (2H, m), 3.12 (1H, dd, | 722 | 0.85 |

**[Table 11-9]**

| | | | | | |
|---|---|---|---|---|---|
| | | | J = 13.2, 10.0 Hz), 2.86 (1H, br-t, J = 11.2 Hz), 2.30 (6H, brs), 2.22-1.53 (5H, m), 1.19 (3H, t, J = 7.2 Hz), 0.92-0.76 (1H, m) | | |
| Exam . 56 | 181 | 604.7 | (CDCl₃); δ: 7.52 (1H, s), 7.28-7.24 (3H, m), 7.22-7.13 (4H, m), 7.06 (1H. br-d, J = 3.6 Hz), 6.97 (1H, dd, J = 4.8, 3.2 Hz), 6.91 (1H, dd, J = 3.2, 1.2 Hz), 6.88 (1H, dd, J = 4.8, 3.2 Hz), 5.46 (1H, dd, J = 10.0, 5.2 Hz), 5.34 (1H. d. J = 7.6 Hz), 5.09 (1H, d, J = 15.2 Hz), 5.02 (1H, d, J = 14.8 Hz), 4.94 (1H, d, J = 14.8 Hz), 4.78 (1H, d. J = 15.2 Hz), 4.01 (1H, dd, J = 10.0, 6.0 Hz), 3.40 (1H, dd, J = 13.6, 4.8 Hz), 3.14 (1H, dd, J = 13.6, 10.0 Hz), 2.45 (3H, s), 2.25-1.95 (4H. m) | 603 | 1.25 |
| Table 10 | 3F | 595.7 | (CDCl₃); δ: 8.50 (1H, s), 7.44 (2H, d, J = 7.2 Hz), 7.37-7.20 (13H, m), 6.74 (1H, t, J = 5.6 Hz), 4.99 (1H, d, J = 14.8 Hz), 4.99 (1H, d, J = 7.6 Hz), 4.78-4.68 (2H, m), 4.50 (1H. dd, J = 14.8, 6.4 Hz), 4.45 (1H, d. J = 7.6 Hz), 4.33 (1H, dd, J = 14.8, 5.2 Hz), 3.94 (1H, dd, J = 10.8, 6.0 Hz), 2.42-2.31 (1H, m), 2.27-2.14 (1H, m), 2.06-1.90 (3H, m), 1.21 (3H, d, 6.4 Hz), 1.03 (3H, d. J = 7.2 Hz) | 596 | 1.35 |
| Table 10 | 3G | 581.67 | (CDCl₃); δ: 9.37 (1H, s), 8.46 (1H, s), 7.46 (2H, d, J = 7.2 Hz), 7.37-7.18 (10H. m), 7.03-6.97 (2H. m), 6.89 (1H. t, J = 3.4 Hz), 5.11 (1H. d. J = 7.2 Hz), 5.03 (1H, d, J = 14.4 Hz), 4.96 (1H, d, J = 14.4 Hz), 4.77 (3H, br-s), 3.99 (1H. dd, J = 10.0. 5.2 Hz), 2.54-2.44 (1H. | 582 | 1.35 |

**[Table 11-10]**

| | | | | | |
|---|---|---|---|---|---|
| | | | m), 2.35-2.19 (1H, m), 2.13-2.02 (3H, m), 1.27 (3H, d, J = 6.8 Hz), 1.10 (3H, d, J = 6.8 Hz) | | |
| Table 10 | 3H | 745.88 | (CDCl₃); δ: 9.26 (1H, s), 8.22 (1H, s), 8.14 (1H, s), 7.59 (1H, d, J = 8.0 Hz), 7.54-7.48 (3H, m), 7.43 (1H, d, J = 8.0 Hz), 7.40-7.07 (13H, m), 5.24 (1H, d, J = 14.4 Hz), 5.05 (1H, d, J = 7.2 Hz), 4.98 (2H, s), 4.93 (1H, d, J = 8.0 Hz), 4.84 (1H, d, J = 14.8 Hz), 4.75 (1H, d, J = 14.8 Hz), 4.65 (1H, d, J = 16.8 Hz), 4.55 (1H, d, J = 16.8 Hz), 4.39 (1H, d, J = 14.8 Hz), 3.93 (1H, dd, J = 10.8, 6.0 Hz), 2.61-2.49 (1H, m), 2.25-2.10 (1H, m), 1.97-1.67 (3H, m), 1.34 (3H, d, J = 6.8 Hz), 1.14 (3H, d, J = 6.8 Hz) | 746 | 1.42 |
| Table 10 | 3I | 571.68 | (CDCl₃); δ: 8.53 (1H, s), 7.43 (2H, d, J = 6.8 Hz), 7.35-7.19 (8H, m), 6.65 (1H, s), 4.99 (1H, d, J = 14.4 Hz), 4.99 (1H, d, J = 7.6 Hz), 4.94 (1H, d, J = 14.4 Hz), 4.76 (1H, d, J = 15.2 Hz), 4.71 (1H, d, J = 15.2 Hz), 4.28 (1H,d, J = 6.4 Hz), 3.91 (1H, dd, J = 10.0, 6.0 Hz), 2.43-2.33 (1H, m), 2.28-1.88 (11H, m), 1.24 (3H, d, J = 6.8 Hz), 1.05 (3H, d, J = 7.2 Hz) | 570 | 1.35 |
| Table 10 | 3J | 625.70 | (CDCl₃); δ: 8.73 (1H, d, J = 1.6 Hz), 7.45 (2H, d, J = 8.4 Hz), 7.37-7.21 (9H, m), 7.10-6.97 (2H, m), 5.15 (1H, d, J = 13.6 Hz), 5.02-4.68 (9H, m), 4.03-3.96 (1H, m), 2.46-2.32 (1H, m), 2.31-1.92 (4H, m), 1.25 (3H, d, J = 6.4 Hz), 1.05 (3H, d, J = 6.8 Hz) | 626 | 1.39 |

**[Table 11-11]**

| | | | | | |
|---|---|---|---|---|---|
| Exam . 62 | 28F | 626.67 | (CDCl₃); δ: 8.77 (1H, d, J = 2.4 Hz), 8.25 (1H, s), 8.09-8.05 (2H. m), 7.80 (1H. dd, J = 8.4, 0.8 Hz), 7.71 (1H, ddd. J = 8.4, 5.6, 1.2 Hz), 7.59-7.49 (2H, m), 7.34-7.30 (2H, m), 6.34-6.28 (4H, m), 5.06 (1H, d, J = 15.2 Hz), 4.98 (1H, d, J = 15.2 Hz), 4.97 (1H, d, J = 7.2 Hz), 4.88 (1H, d, J = 15.6 Hz), 4.59 (1H, dd, J = 15.6, 6.0 Hz), 4.53 (1H, dd, J = 15.2, 6.0 Hz), 4.49 (1H, d, J = 7.6 Hz), 3.92 (1H, dd, J = 10.4, 6.0 Hz), 2.36-2.19 (1H, m), 2.17-1.70 (4H, m), 1.13 (3H, d, J = 6.4 Hz), 0.93 (3H, d, J = 7.2 Hz) | 627 | 1.05 |
| Exam . 63 | 1F | 679.82 | (CDCl₃); δ: 7.75 (1H, d. J = 8.4 Hz), 7.70 (1H, s), 7.59 (2H, t, J = 8.4 Hz), 7.48-7.13 (17H, m), 7.07-7.02 (2H, m), 5.49 (1H, dd. J = 10.0, 5.2 Hz), 4.82 (1H, d, J = 14.4 Hz), 4.74 (1H, d, J = 14.4 Hz), 4.68 (1H, d, J = 15.2 Hz), 4.56 (1H, d, J = 15.6 Hz), 4.33-4.27 (1H, m), 4.00-3.88 (2H, m), 3.80 (2H, d, J = 12.4 Hz), 3.60 (1H, dd, J = 13.6, 4.8 Hz), 3.28 (1H, dd, J = 13.2, 10.0 Hz), 2.78 (1H, d, J = 12.8 Hz), 2.09-1.76 (4H, m) | 680 | 0.88 |

The compounds of the present invention were investigated for their anti-SARS-CoV-2 activities.

### Test Example 1

### Test for Anti-SARS-CoV-2 Activity In Vitro

To evaluate the anti-SARS-CoV-2 activity of the compounds according to the present invention, the positive control compound Remdesivir and compound 1D of the present invention were compared each other. SARS-CoV-2 induces cytopathic effect (CPE) in infected cells (Zhu N, Wang W, Liu Z, Liang C, Wang W, Ye F, Huang B, Zhao L, Wang H, Zhou W, Deng Y, Mao L, Su C, Qiang G, Jiang T, Zhao J, Wu G, Song J, Tan W. Morphogenesis and cytopathic effect of SARS-CoV-2 infection in human airway epithelial cells. Nat Commun. 2020 Aug 6;11(1):3910.). Thus, an anti-SARS-CoV-2 activity test was conducted using CPE inhibition as an indicator. Specifically, a miner modification was applied to the procedure described in the literature: Riva, L., Yuan, S., Yin, X. et al. Discovery of SARS-CoV-2 antiviral drugs through large-scale compound repurposing. Nature 586, 113-119 (2020). https://doi.org/10.1038/s41586-020-2577-1, and the experiments were conducted as follows. After seeding the VeroE6-TMPRSS2 cells in a 96-well plate, the cells were infected with SARS-CoV-2 at a Multiplicity of Infection (MOI) of 0.01 for 1 hour. Following the infection, the cells were washed with phosphate-buffered saline (PBS) and then replaced with fresh DMEM medium containing 10 µM of each compound concentration. The cell survival rates of each well were measured on the third Day Post Infection (dpi: days post infection) using the CellTiterGlo (registered trademark) luminescent assay. The experiment was conducted using a double repetition test and was repeated twice for confirmation.

As a result, the positive control compound Remdesivir showed 90% or more inhibition of CPE in the assay at a concentration of 10 µM. On the other hand, compound 1D prepared in Example 19 was evaluated and showed approximately 100% inhibition effect, demonstrating that compound 1D showed antiviral activity against SARS-CoV-2 which was comparable to or superior to that of Remdesivir. For reference, the inhibitory effect in the non-treated group of virus-infected cells was 0%, while that in the virus-non-infected cells was 100%.

### Test Example 2

### Measurement of 50% Effective Concentration (EC₅₀) in SARS-CoV-2 virus proliferation inhibitory activity

Compound 1D, which demonstrated anti-SARS-CoV-2 activity comparable to or superior to that of the positive control compound Remdesivir in the CPE inhibition assay, was assayed as follows according to the methods described in the above literature.

To determine the EC₅₀, VeroE6-TMPRSS2 or Caco-2 was used to conducted to viral load reduction assay at an MOI of 0.01A at 48 hpi (hours post infection). The supernatant of the culture medium was collected from the infected cells to quantify the virus using qRT-PCR analysis. Specifically, 100 µl of the viral supernatant was dissolved in 400 µl of the buffer AVL, and then the total RNA was extracted using QIAamp Viral RNA Mini Kit (Qiagen, Venlo, Netherlands). SARS-CoV-2 virus was quantified by Real-time one-step qRT-PCR using QuantiNova Probe RT-PCR kit (Qiagen) and LightCycler 480 Real-Time PCR System (Roche, Rotkreuz, Switzerland). 10 µl of QuantiNova Probe RT-PCR Master Mix (2x), 1.2 µl of RNase-free water, 0.2 µl of QuantiNova Probe RT-Mix, each 1.6 µl of 10 µM forward and reverse primers, 0.4 µl of 10 µM probe, and 5 µl of extracted RNA as a template were mixed, and a total reaction solution volume was adjusted to be 20 µl. In the PCR reaction, the mixture was incubated at 45°C for 10 minutes for reverse transcription, and incubated at 95°C for 5 minutes for denaturation, while being subjected to 45 cycles of 5 seconds at 95°C and 30 seconds at 55°C, with signal detection and measurement performed at each cycle after the annealing step. As primer and probe sequences, a corresponding region to the RNA-dependent RNA polymerase/helicase (RdRP/Hel) gene region of SARS-CoV-2 was utilized:
Forward primer: 5'-CGCATACAGTCTTRCAGGCT-3' (SEQ ID No: 1);
Reverse primer: 5'-GTGTGATGTTGAWATGACATGGTC-3' (SEQ ID No: 2);
Probe: 5'-FAM-TTAAGATGTGGCTTGCATACGTAGAC-IABkFQ-3' (SEQ ID No: 3)

The EC₅₀ was calculated using GraphPad. The repeated test was conducted three times.

### Test Example 3

### Measurement of 50% cytotoxic concentration (CC₅₀)

To evaluate the cytotoxicity of the selected compound, CellTiter-Glo (registered trademark) Luminescent Cell Viability Assay (Promega Corporation, Madison, WI, USA) was conducted. Briefly, VeroE6-TMPRSS2 or Caco-2 cells (4×10⁴ cells/well) were incubated with different concentrations of the compound for 48 hours, and then, the number of cells was measured as the amount of ATP in the cell lysate supernatant using CellTiter-Glo (registered trademark) Luminescent Cell Viability Assay method. The CC₅₀ of the compound was calculated by GraphPad. The repeated test was conducted three times.

The results of the measurements of EC₅₀ and CC₅₀ in Test Examples 2 and 3 showed that compound 1D has an EC₅₀ of 3.98 µM against the Omicron variant of SARS-CoV-2 virus, and has a CC₅₀ of 144.7 µM against VeroE6-TMPRSS2 cells. Additionally, the compound showed an EC₅₀ of 1.71 µM against the Delta variant of SARS-CoV-2 virus and a CC₅₀ of greater than 400 µM against Caco-2 cells. These results suggested that the compound has a selectivity index (CC₅₀/EC₅₀) of approximately 36 times and 234 times or more against the Omicron variant and the Delta variant, respectively. The compound of the present invention is useful for the treatment and prevention of COVID-19.

The materials used in the above tests are as follows.

### Cell lines

VeroE6 (ATCC CRL-1586 (registered trademark)) and Vero E6-TMPRSS2 (BPS Bioscience Catalog #78081) as well as Caco-2 cells (ATCC HTB-37 (registered trademark)) were purchased as commercial products and they were passaged in Dulbecco's Modified Eagle Medium (DMEM, Gibco, CA, USA) supplemented with 10% heat-inactivated fetal bovine serum (FBS, Gibco, CA, USA), 50 U/mL penicillin, and 50 µg/mL streptomycin. All antibiotics and supplements were purchased from Thermo Fisher Scientific, Inc. (Waltham, MA). All cell lines were negative for mycoplasma test.

### Virus strains

SARS-CoV-2 HKU-001a (GenBank accession number: MT230904) and SARS-CoV-2 Omicron BA.5.2 S (GISAID: EPI_ISL_13777659) as well as B.1.617.2/Delta (GenBank: OM212471) were isolated from the nasopharyngeal aspirate samples of COVID-19 patients in Hong Kong. The virus were propagated in VeroE6 cells and stored in aliquots at -80°C until use. The titer of the cultured SARS-CoV-2 was determined using the Plaque Forming Unit (PFU) assay.

### Test Example 4

### Test for Anti-SARS-CoV-2 virus Activity In vitro

For the compounds of the present invention other than compound 1D, a CPE inhibition assay was conducted to evaluate the anti-SARS-CoV-2 virus activity by measuring the inhibition of cytopathic effect (CPE), as referenced in the literature: Riva, L., Yuan, S., Yin, X. et al. "Discovery of SARS-CoV-2 antiviral drugs through large-scale compound repurposingNature 586, 113-119 (2020) with a miner modification. After seeding the VeroE6-TMPRSS2 cells in a 96-well plate, the cells were infected with SARS-CoV-2 at a Multiplicity Of Infection (MOI) of 0.01 for 1 hour. Following the infection, the cells were washed with phosphate-buffered saline (PBS) and then replaced with fresh DMEM medium containing 10 µM of each compound concentration. The cell survival rates of each well were measured on the third Day Post Infection (dpi) using the CellTiterGlo (registered trademark) luminescent assay. The repeated test was conducted three times, and the average was taken.

In the following tables, at a compound concentration of 10µM, compounds that showed a CPE inhibition effect of 5% or more were classified as A, compounds that showed a CPE inhibition effect of less than 5% but 1% or more were classified as B, and those that showed less than 1% were classified as C. For reference, the inhibitory effect in the non-treated group of virus-infected cells was 0%, while that in the virus-non-infected cells was 100%.

**[Table 12-1]**

| Table 12: Anti SARS CoV 2 activity of the compound of the present invention at a concentration of 10 µM | | | | | |
|---|---|---|---|---|---|
| Example No. | Compound No. | Anti SARS-CoV-2 activity | Example No. | Compound No. | Anti SARS-CoV-2 activity |
| 1 | 1A | C | 18 | 1C | C |
| 1 | 1B | C | 18 | 1D | A |
| 2 | 2A | C | 19 | 2D | - |
| 3 | 3A | - | 20 | 3D | A |
| 4 | 4A | C | 21 | 4C | C |
| 4 | 4B | A | 21 | 4D | A |
| 5 | 5A | - | 22 | 5D | A |
| 6 | 6A | C | 23 | 6D | B |
| 7 | 7A | C | 24 | 7D | B |
| 8 | 8A | - | 25 | 8D | c |
| 9 | 9B | C | 26 | 17D | A |
| 10 | 11B | A | 27 | 18D | C |
| 11 | 12A | B | 28 | 16E | B |
| 12 | 13B | - | 29 | 1E | C |
| 13 | 14A | B | 30 | 2E | - |
| 14 | 15A | C | 31 | 4E | B |
| 14 | 15B | C | 32 | 5E | C |
| 15 | 16B | - | 33 | 7E | B |
| 16 | 17A | - | 34 | 8E | C |
| 17 | 18A | A | | | |

**[Table 12-2]**

| Example No. | Compound No. | Anti SARS-CoV-2 activity | Example No. | Compound No. | Anti SARS-CoV-2 activity |
|---|---|---|---|---|---|
| 3 | 3B | A | 49 | 26D | A |
| 12 | 13B | B | 50 | 27D | A |
| 35 | 19A | A | 51 | 28D | A |
| 36 | 20C | - | 52 | 29D | A |
| 37 | 21C | - | 53 | 25E | B |
| 38 | 22A | B | 54 | 18F | B |
| 39 | 23A | A | 55 | 18G | A |
| 40 | 24A | C | 55 | 18H | A |
| 41 | 25A | A | 56 | 18I | B |
| 42 | 26A | A | 57 | 3F | A |
| 43 | 27A | A | 58 | 3G | A |
| 44 | 28A | A | 59 | 3H | A |
| 45 | 29A | A | 60 | 3I | A |
| 46 | 30A | A | 61 | 3J | A |
| 47 | 20D | A | 62 | 28F | A |
| 48 | 21D | B | 63 | 1F | C |

| | | | | | |
|---|---|---|---|---|---|
| A: CPE inhibition effect of 5% or more B: CPE inhibition effect of less than 5% but 1% or more C: CPE inhibition effect of less than 1% : No data | | | | | |

As a further aspect, the present invention encompasses the following.

### <Compounds>

A compound represented by formula (I), its enantiomer, or a pharmaceutically acceptable salt thereof: 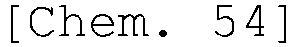 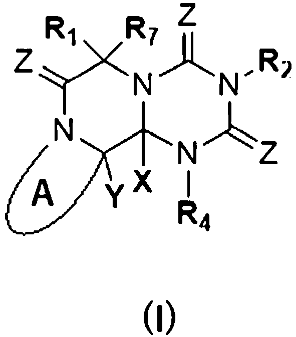 wherein
ring A is a moiety represented by 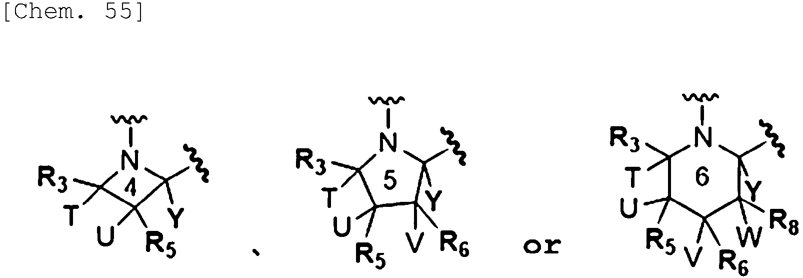
R1 and R7 are each independently a hydrogen, an optionally substituted Cl-6 alkyl, an optionally substituted C6-10 aryl, or an optionally substituted C5-10 heteroaryl, or R1 and R7 crosslink together with the carbon to which they are attached to form a C3-6 spiro ring;
R2 and R4 are each independently a hydrogen, an optionally substituted C1-6 alkyl group, an optionally substituted C6-10 aryl group, an optionally or substituted C5-10 heteroaryl group, an optionally substituted carbonyl, an optionally substituted sulfonyl, or an optionally substituted sulfinyl;
X is a hydrogen, a hydroxy, an optionally substituted C1-6 alkyl, or an optionally substituted C1-6 alkoxy;
Y is a hydrogen or an optionally substituted C1-6 alkyl, or
X and Y combine with an adjacent carbon atom to form a double bond;
Z is independently either an oxygen or a sulfur;
R3 is an optionally substituted C1-6 alkyl, an optionally substituted C5-10 heteroaryl, or an optionally substituted carbonyl;
R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
in which the substituent in "optionally substituted" are selected from the following:
   a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.

[102]
The compound according to [101] represented by formula (I), its enantiomer, or a pharmaceutically acceptable salt thereof: 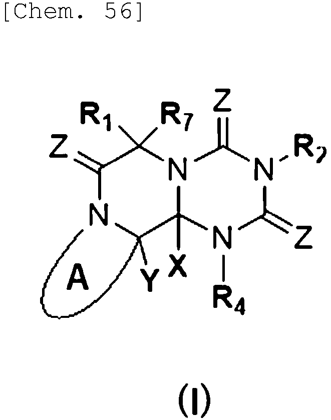 wherein
ring A is a moiety represented by 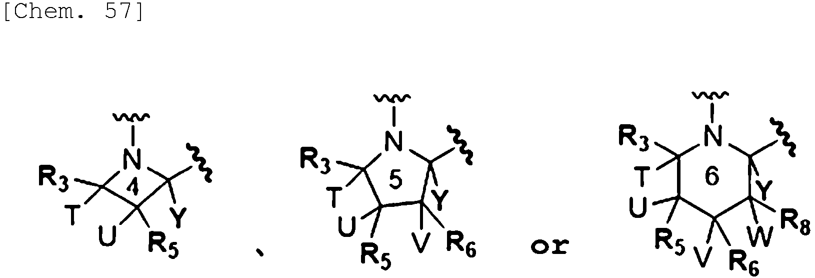
R1 and R7 are each independently a hydrogen, an optionally substituted C1-6 alkyl, an optionally substituted C6-10 aryl, or an optionally substituted C5-10 heteroaryl, or R1 and R7 crosslink together with the carbon to which they are attached to form a C3-6 spiro ring;
R2 and R4 are each independently a hydrogen, an optionally substituted C1-6 alkyl group, an optionally substituted C6-10 aryl group, or an optionally substituted C5-10 heteroaryl group;
X is a hydrogen, or a hydroxy;
Y is a hydrogen or an optionally substituted methyl, or
X and Y combine with an adjacent carbon atom to form a double bond;
Z is independently either an oxygen or a sulfur;
R3 is an optionally substituted C1-6 alkyl, an optionally substituted C5-10 heteroaryl, or an optionally substituted carbonyl;
R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
in which the substituent in "optionally substituted" are selected from the following:
   a hydroxy, a halogen, a cyano, a carbamoyl, an amino, a carboxy, an optionally substituted Cl-6 alkyl, an optionally substituted C1-6 alkoxy, an optionally substituted C6-10 aryl, an optionally substituted C5-10 heteroaryl, or a protecting group.

[103]
The compound according to [101] or [102] represented by formula (II), its enantiomer, or a pharmaceutically acceptable salt thereof: 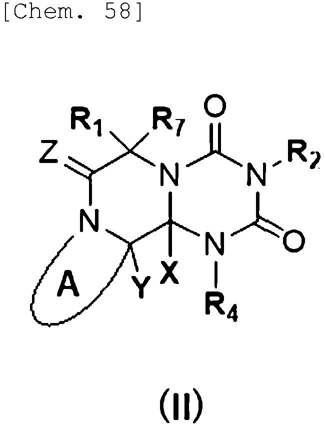 wherein
ring A, R1 and R7, R2 and R4, X and Y, as well as Z are the same as defined above

[104]
The compound according to any one of [101] to [103] represented by formula (I), its enantiomer, or a pharmaceutically acceptable salt thereof:
wherein
ring A is a moiety selected from 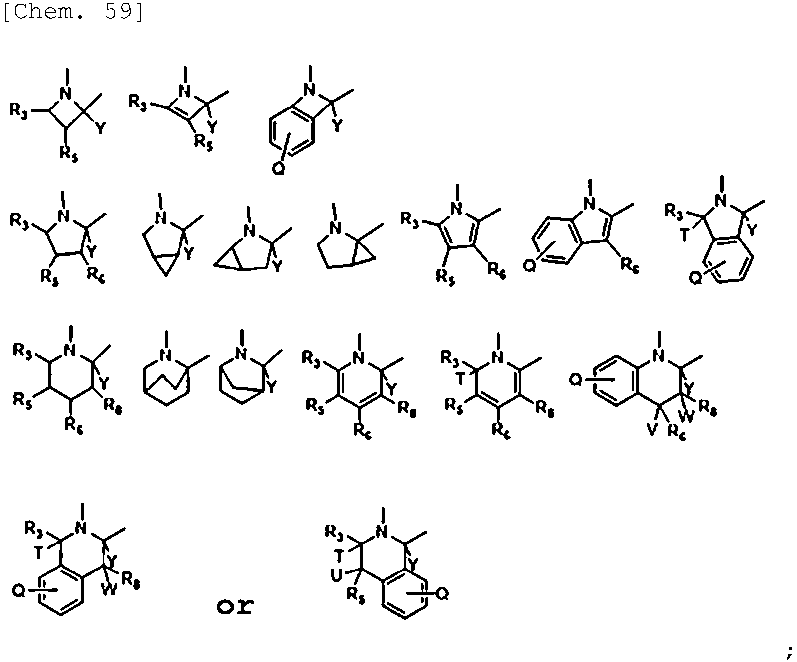 wherein
R3, R5, R6 and R8, Y, T, U, V, as well as W are the same as defined above;
Q is a hydrogen, a hydroxy, a halogen, an optionally substituted C1-6 alkyl, an optionally substituted C1-6 alkoxy, an optionally substituted C1-6 alkylthio, an optionally substituted C1-6 alkylamino, an optionally substituted C6-10 aryl, or an optionally substituted C5-10 heteroaryl.

[105]
The compound according to [104] represented by formula (I), its enantiomer, or a pharmaceutically acceptable salt thereof:
wherein
ring A is a moiety selected from 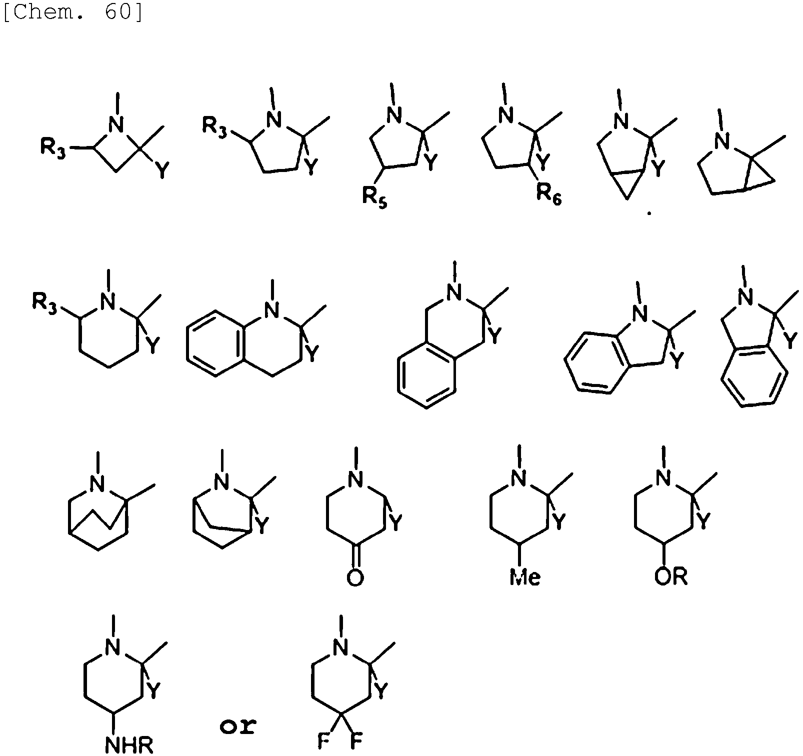 wherein
R3, R5, R6 and Y are the same as defined above, and
R is a hydrogen, an optionally substituted carbonyl, an optionally substituted C1-6 alkyl, an optionally substituted C6-10 aryl, or an optionally substituted C5-10 heteroaryl; and
X is a hydroxy.

[106]
The compound according to any one of [101] to [105] represented by formula (I), its enantiomer, or a pharmaceutically acceptable salt thereof, wherein R2 and R4 are the same group.

### <Preferred compounds>

The compound according to any one of [101] to [106] represented by formula (III), its enantiomer, or a pharmaceutically acceptable salt thereof: 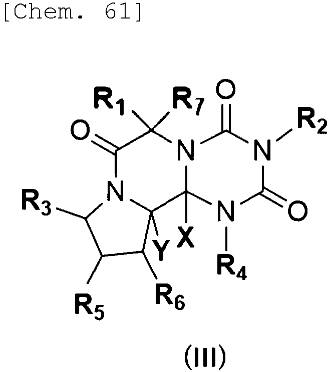 wherein
R1 and R7 are each independently a hydrogen, an optionally substituted C1-6 alkyl, an optionally substituted C6-10 aryl, or an optionally substituted C5-10 heteroaryl, or R1 and R7 crosslink together with the carbon to which they are attached to form a C3-6 spiro ring;
R2 and R4 are each independently an optionally substituted C1-6 alkyl group, an optionally substituted C6-10 aryl group, or an optionally substituted C5-10 heteroaryl group;
R3 is an optionally substituted C1-6 alkyl, an optionally substituted C5-10 heteroaryl, or an optionally substituted carbonyl;
R5, and R6 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
X is a hydroxy;
Y is a hydrogen or an optionally substituted methyl, or
X and Y combine with an adjacent carbon atom to form a double bond;
in which the substituent in "optionally substituted" are selected from the following:
   a hydroxy, a halogen, a cyano, a carbamoyl, an amino, a carboxy, an optionally substituted Cl-6 alkyl, an optionally substituted C1-6 alkoxy, an optionally substituted C6-10 aryl, an optionally substituted C5-10 heteroaryl, or a protecting group.

[108]
The compound according to [107], its enantiomer, or a pharmaceutically acceptable salt thereof:
wherein
either R1 or R7 is a hydrogen, and the other is a group represented by the formula: 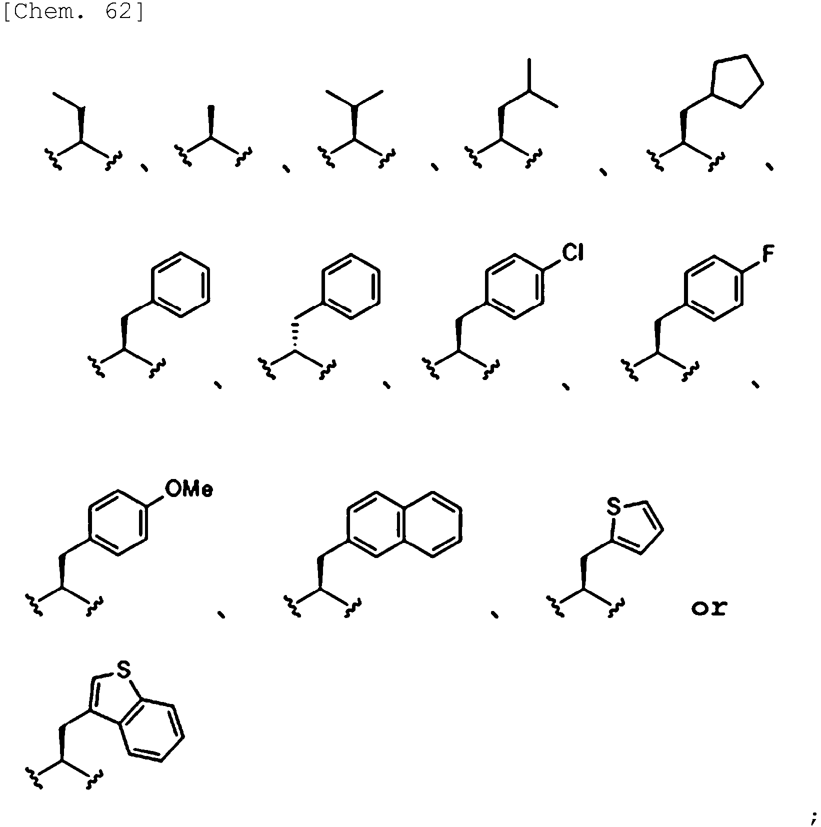 or
R1 and R7 combine together to form a spiro ring represented by the formula: 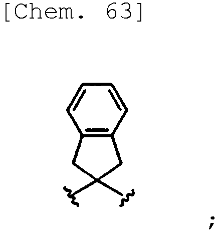
the triazacyclohexane ring by which R2, R4, and X are substituted, is selected from the groups represented by the formula:

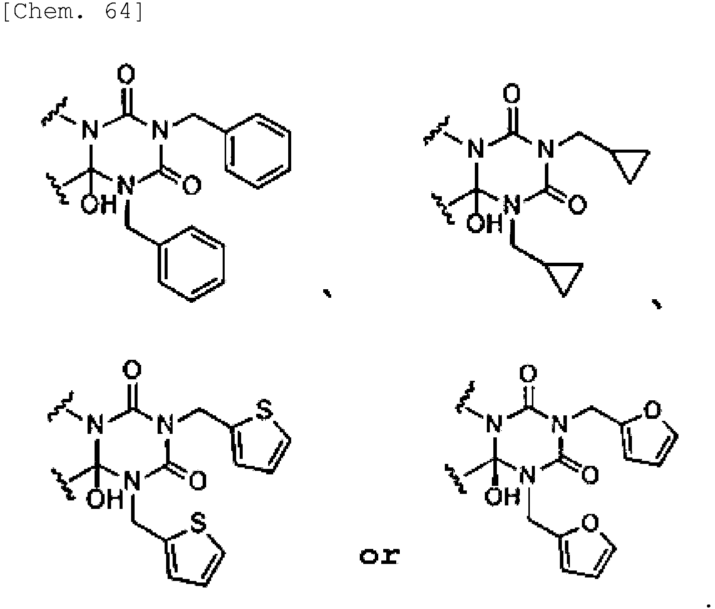

[109]
The compound according to [107] or [108], its enantiomer, or a pharmaceutically acceptable salt thereof, wherein X and Y combine together with an adjacent carbon to form a double bond.

[110]
The compound according to [109], its enantiomer, or a pharmaceutically acceptable salt thereof, wherein the compound is represented by either:

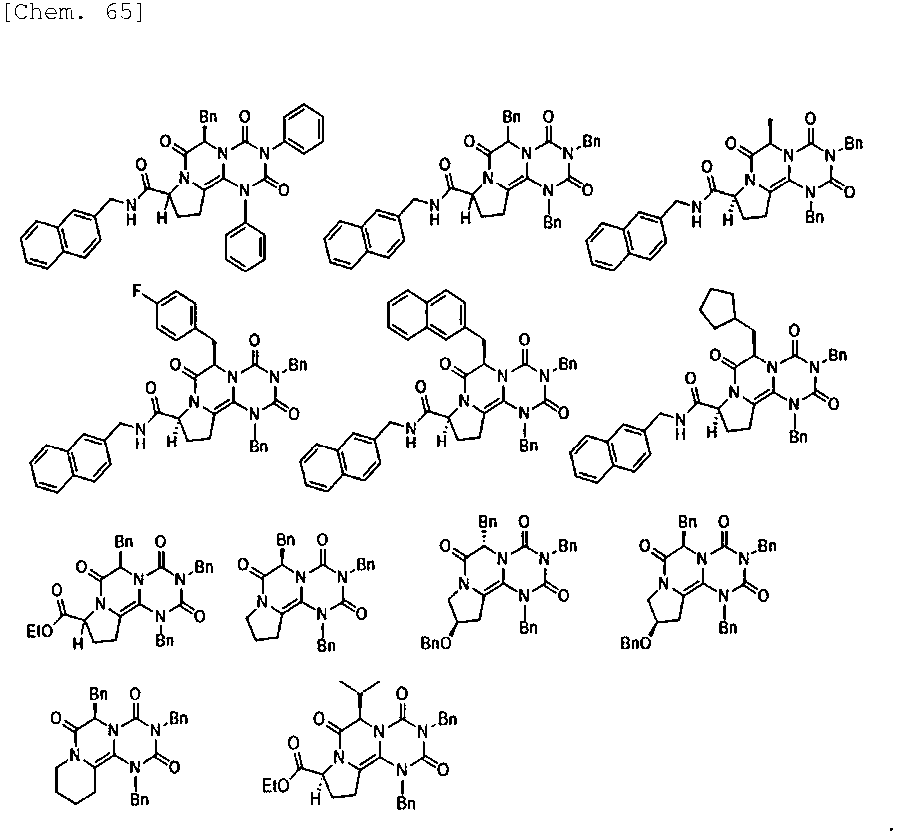

[111]
The compound according to any one of [107] to [110], its enantiomer, or a pharmaceutically acceptable salt thereof: wherein
R3 is a group selected from: 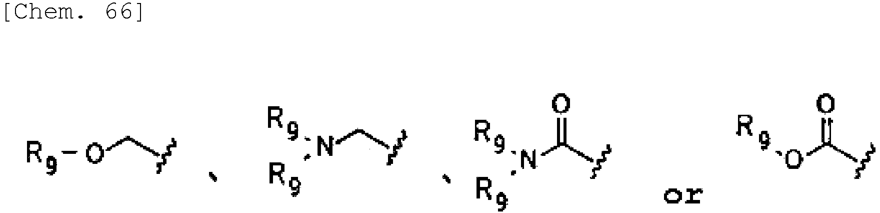 wherein
R9 is independently a hydrogen, an optionally substituted Cl-6 alkyl, an optionally substituted C6-10 aryl, an optionally substituted C5-10 heteroaryl, or a protecting group, or
when R9 appears twice, they crosslink together with the nitrogen to which they are attached to form an optionally substituted 3- to 6-membered ring.

[111-2]
The compound according to [111-2], its enantiomer, or a pharmaceutically acceptable salt thereof:
wherein
R3 is a group represented by: 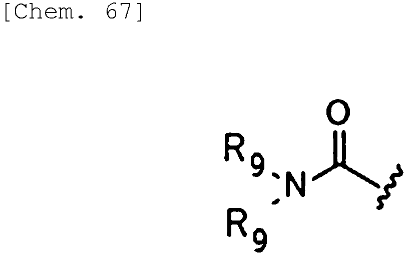 wherein
R9s crosslink together with the nitrogen to which they are attached to form an optionally substituted 5-membered ring.

[111-3]
The compound according to [111-2], its enantiomer, or a pharmaceutically acceptable salt thereof:
wherein
the group represented by: 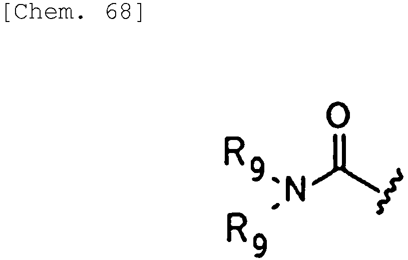 is an optionally substituted 5-membered ring, and is a group represented by: 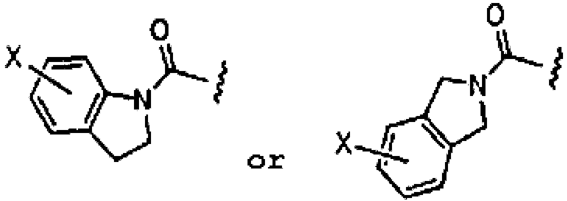 wherein
X is a hydrogen, a hydroxy, a halogen, a cyano, a carbamoyl, an optionally substituted Cl-6 alkyl, an optionally substituted Cl-6 alkoxy, or an optionally substituted C1-6 alkylamino.

[112]
The compound according to any one of [111] to [111-3], its enantiomer, or a pharmaceutically acceptable salt thereof:
wherein
R3 is a group selected from: 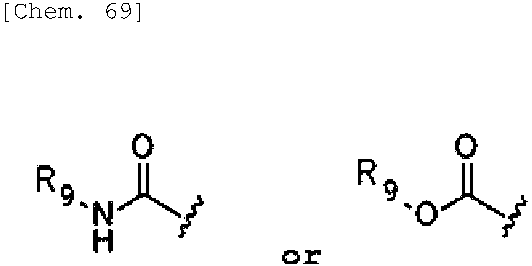 wherein
R9 is independently a hydrogen, an optionally substituted C1-6 alkyl groups, an optionally substituted C6-10 aryl groups, an optionally substituted C5-10 heteroaryl groups, or a protecting group.

[113]
The compound according to [112], its enantiomer, or a pharmaceutically acceptable salt thereof:
wherein
R3 is a group represented by:

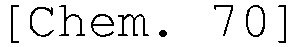

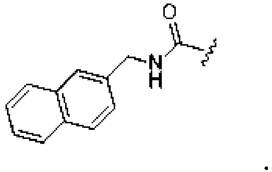

[114]
The compound according to any one of [107] to [113], its enantiomer, or a pharmaceutically acceptable salt thereof, wherein R2 and R4 are the same group.

[115]
The compound according to any one of [107] to [114], its enantiomer, or a pharmaceutically acceptable salt thereof, wherein X and Y combine together with an adjacent carbon atom to form a double bond.

### <Pharmaceutical compositions>

A pharmaceutical composition which comprises the compound according to any one of [101] to [115], its enantiomer, or a pharmaceutically acceptable salt thereof.

[117]
The pharmaceutical composition according to [116] for the treatment or prevention of SARS-CoV-2 novel coronavirus infection.

### INDUSTRIAL APPLICABILITY

The present invention is useful in treatment or prevention of SARS-CoV-2 novel coronavirus infection.

## Claims

1. A compound represented by formula (I), its enantiomer, or a pharmaceutically acceptable salt thereof: 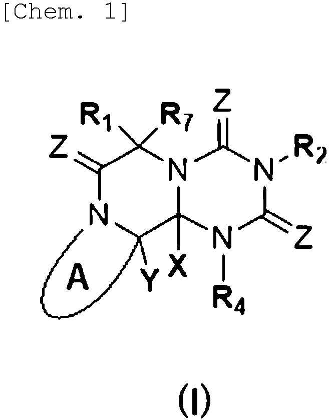 wherein
ring A is a moiety represented by 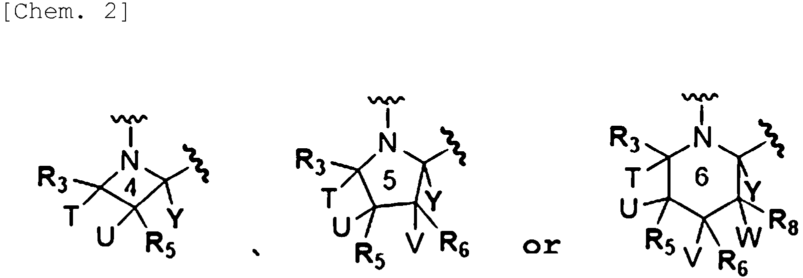
R1 and R7 are each independently a hydrogen, an optionally substituted Cl-6 alkyl, an optionally substituted C6-10 aryl, or an optionally substituted C5-10 heteroaryl, or R1 and R7 together with the carbon atom to which they are attached form a C3-6 spiro ring;
R2 and R4 are each independently a hydrogen, an optionally substituted C1-6 alkyl group, an optionally substituted C6-10 aryl group, an optionally substituted C5-10 heteroaryl group, an optionally substituted carbonyl, an optionally substituted sulfonyl, or an optionally substituted sulfinyl;
X is a hydrogen, a hydroxy, an optionally substituted C1-6 alkyl, or an optionally substituted C1-6 alkoxy;
Y is a hydrogen or an optionally substituted C1-6 alkyl, or
X and Y combine with an adjacent carbon atom to form a double bond;
Z is independently either an oxygen or a sulfur;
R3 is an optionally substituted C1-6 alkyl, an optionally substituted C5-10 heteroaryl, or an optionally substituted carbonyl;
R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
in which the substituents in "optionally substituted" are selected from the following:
a hydroxy, a halogen, a cyano, a carbamoyl, an amino, a carboxy, an optionally substituted Cl-6 alkyl, an optionally substituted C1-6 alkoxy, an optionally substituted C6-10 aryl, an optionally substituted C5-10 heteroaryl, or a protecting group.

2. The compound according to claim 1 represented by formula (I), its enantiomer, or a pharmaceutically acceptable salt thereof: 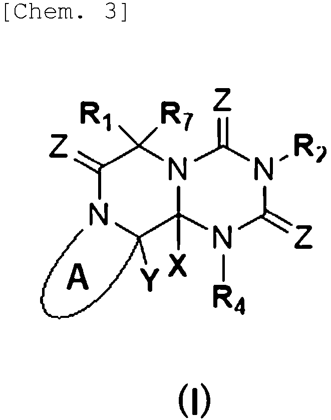 wherein
ring A is a moiety represented by 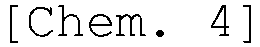 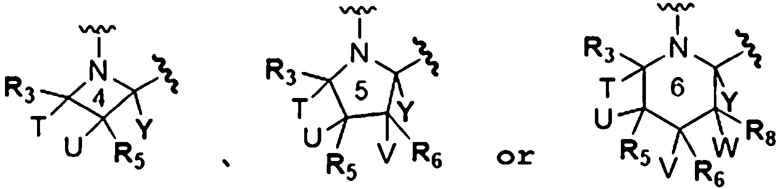
R1 and R7 are each independently a hydrogen, an optionally substituted C1-6 alkyl, an optionally substituted C6-10 aryl, or an optionally substituted C5-10 heteroaryl, or R1 and R7 together with the carbon atom to which they are attached form a C3-6 spiro ring;
R2 and R4 are each independently a hydrogen, an optionally substituted Cl-6 alkyl group, an optionally substituted C6-10 aryl group, or an optionally substituted C5-10 heteroaryl group;
X is a hydrogen, or a hydroxy;
Y is a hydrogen or an optionally substituted methyl, or
X and Y combine with an adjacent carbon atom to form a double bond;
Z is independently either an oxygen or a sulfur;
R3 is an optionally substituted C1-6 alkyl, an optionally substituted C5-10 heteroaryl, or an optionally substituted carbonyl;
R5, R6, and R8 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
T, U, V, and W are each independently a hydrogen, an optionally substituted C1-6 alkyl group, an optionally substituted C6-10 aryl group, or an optionally substituted C5-10 heteroaryl group, or T and U combine together with an adjacent carbon atom to form a double bond or an optionally substituted benzene ring, and/or V and W combine together with an adjacent carbon atom to form a double bond or an optionally substituted benzene ring;
in which the substituents in "optionally substituted" are selected from the following:
a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.

3. The compound according to claim 1 represented by formula (II), its enantiomer, or a pharmaceutically acceptable salt thereof: 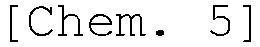 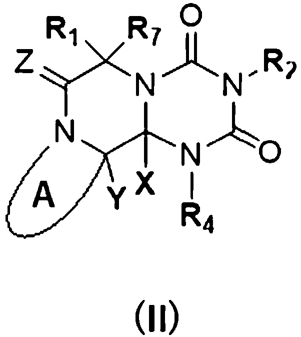 wherein
ring A, R1 and R7, R2 and R4, X and Y, as well as Z are the same as defined above

4. The compound according to claim 1 represented by formula (I), its enantiomer, or a pharmaceutically acceptable salt thereof:
wherein
ring A is a moiety selected from 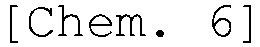 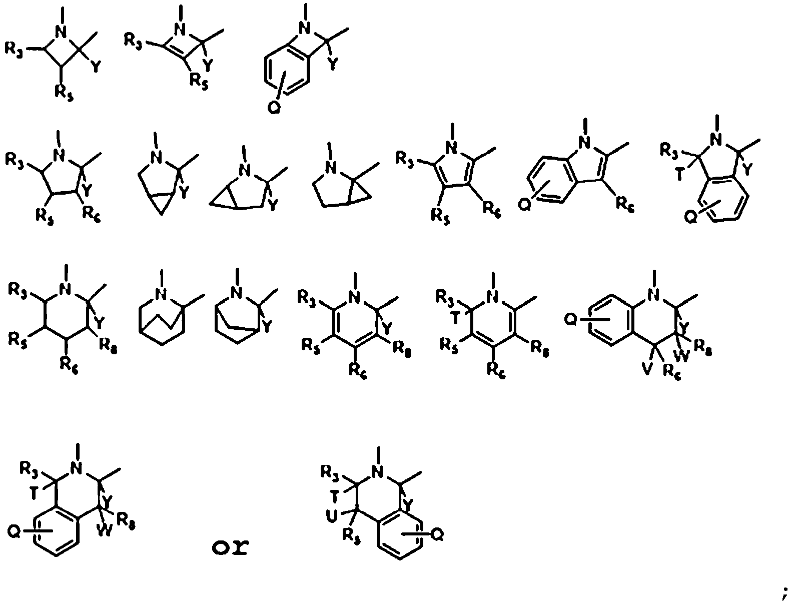 wherein
R3, R5, R6 and R8, Y, T, U, V, as well as W are the same as defined above;
Q is a hydrogen, a hydroxy, a halogen, an optionally substituted C1-6 alkyl, an optionally substituted Cl-6 alkoxy, an optionally substituted C1-6 alkylthio, an optionally substituted C1-6 alkylamino, an optionally substituted C6-10 aryl, or an optionally substituted C5-10 heteroaryl.

5. The compound according to claim 4 represented by formula (I), its enantiomer, or a pharmaceutically acceptable salt thereof:
wherein
ring A is a moiety selected from 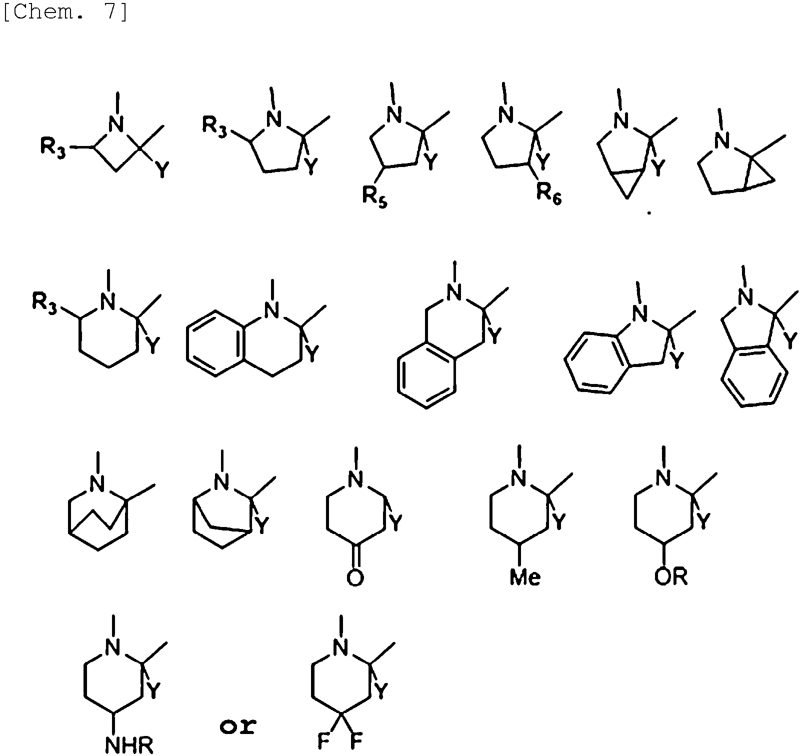 wherein
R3, R5, R6 and Y are the same as defined above, and
R is a hydrogen, an optionally substituted carbonyl, an optionally substituted C1-6 alkyl, an optionally substituted C6-10 aryl, or an optionally substituted C5-10 heteroaryl; and
X is a hydroxy.

6. The compound according to claim 1 represented by formula (I), its enantiomer, or a pharmaceutically acceptable salt thereof, wherein R2 and R4 are the same group.

7. The compound according to claim 1 represented by formula (III), its enantiomer, or a pharmaceutically acceptable salt thereof: 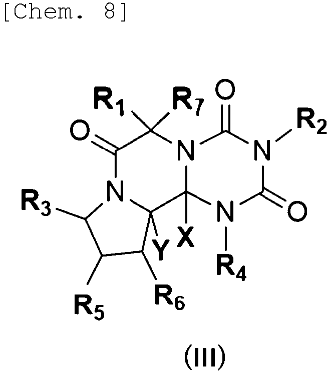 wherein
R1 and R7 are each independently a hydrogen, an optionally substituted C1-6 alkyl, an optionally substituted C6-10 aryl, or an optionally substituted C5-10 heteroaryl, or R1 and R7 together with the carbon atom to which they are attached form a C3-6 spiro ring;
R2 and R4 are each independently an optionally substituted C1-6 alkyl group, an optionally substituted C6-10 aryl group, or an optionally substituted C5-10 heteroaryl group;
R3 is an optionally substituted C1-6 alkyl, an optionally substituted C5-10 heteroaryl, or an optionally substituted carbonyl;
R5, and R6 are each independently a hydrogen, a hydroxy, a halogen, an amino, a cyano, an optionally substituted carbonyl, an optionally substituted C1-6 alkyl, an optionally substituted C2-6 alkenyl, an optionally substituted C2-6 alkynyl, an optionally substituted C6-10 aryl, an optionally substituted C5-10 heteroaryl, or an optionally substituted C1-6 alkoxy;
X is a hydroxy;
Y is a hydrogen or an optionally substituted methyl, or
X and Y combine with an adjacent carbon atom to form a double bond;
in which the substituents in "optionally substituted" are selected from the following:
a hydroxy, a halogen, a cyano, a carbamoyl, an amino, a carboxy, an optionally substituted C1-6 alkyl, an optionally substituted C1-6 alkoxy, an optionally substituted C6-10 aryl, an optionally substituted C5-10 heteroaryl, or a protecting group.

8. The compound according to claim 7 represented by formula (I), its enantiomer, or a pharmaceutically acceptable salt thereof:
wherein
either R1 or R7 is a hydrogen, and the other is a group represented by the formula: 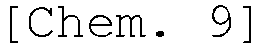 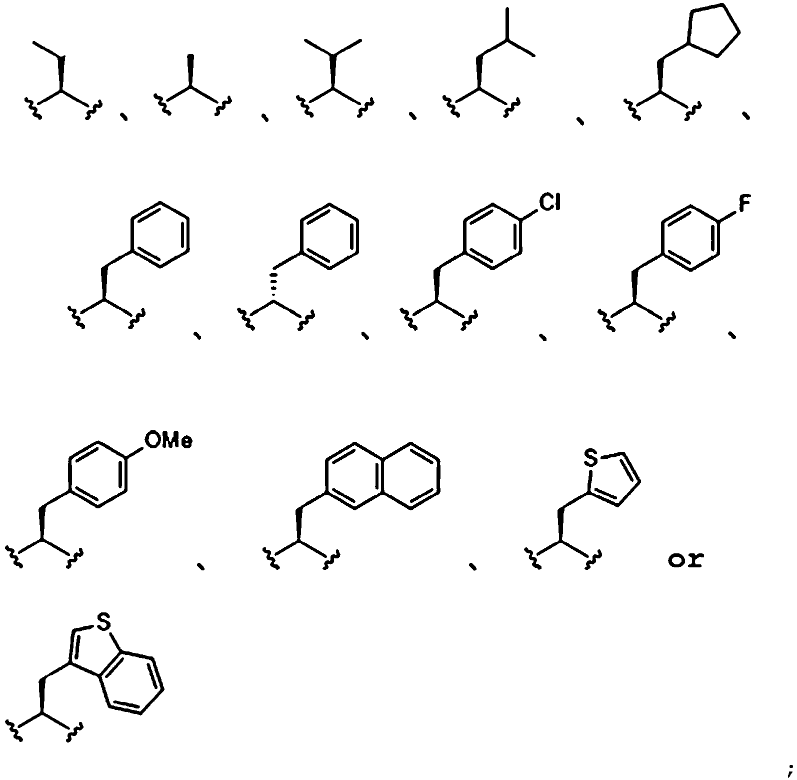 or
R1 and R7 combine together to form a spiro ring represented by the formula: 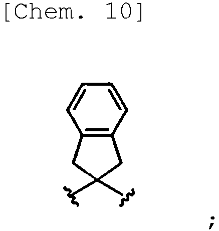
the triazacyclohexane ring by which R2, R4, and X are substituted, is selected from the groups represented by the formula:

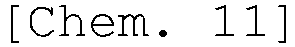

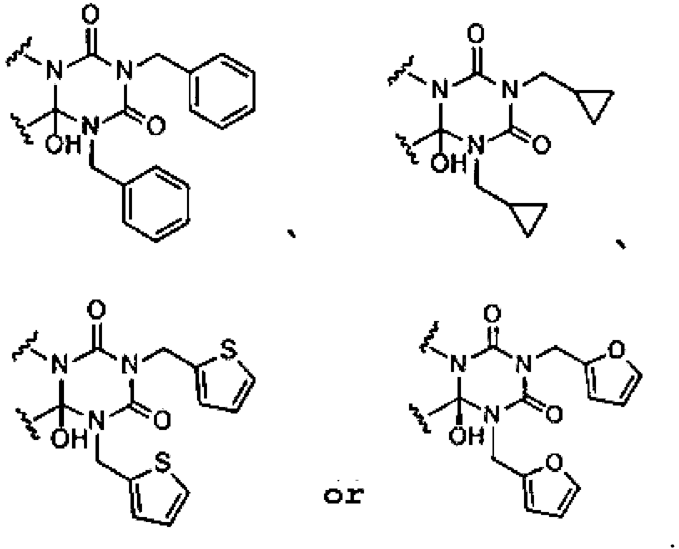

9. The compound according to claim 7, its enantiomer, or a pharmaceutically acceptable salt thereof, wherein X and Y combine together with an adjacent carbon to form a double bond.

10. The compound according to claim 9, its enantiomer, or a pharmaceutically acceptable salt thereof, wherein the compound is represented by either:

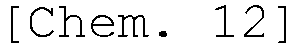

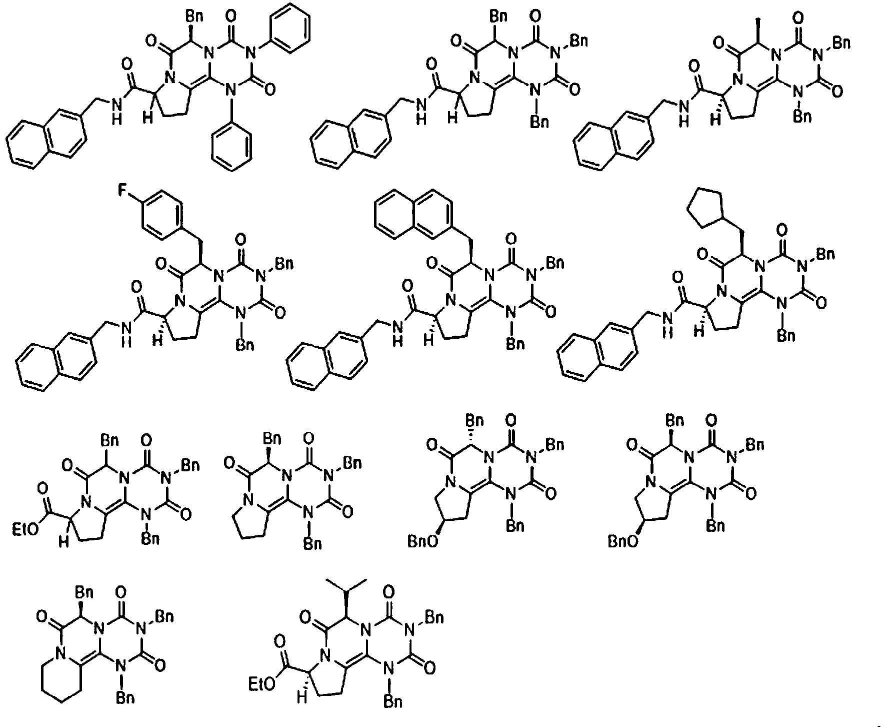

11. The compound according to claim 7, its enantiomer, or a pharmaceutically acceptable salt thereof:
wherein
R3 is a group selected from: 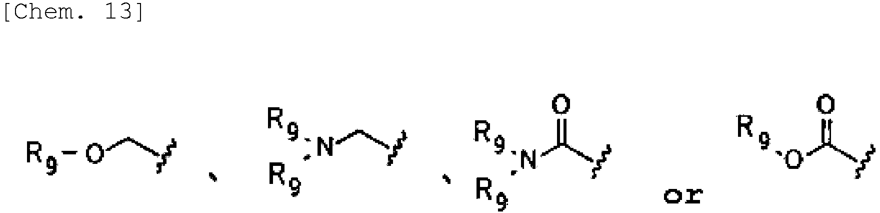 wherein
R9 is independently a hydrogen, an optionally substituted C1-6 alkyl, an optionally substituted C6-10 aryl, an optionally substituted C5-10 heteroaryl, or a protecting group, or
when R9 appears twice, they together with the nitrogen atom to which they are attached form an optionally substituted 3- to 6-membered ring.

12. The compound according to claim 11, its enantiomer, or a pharmaceutically acceptable salt thereof:
wherein
R3 is a group selected from: 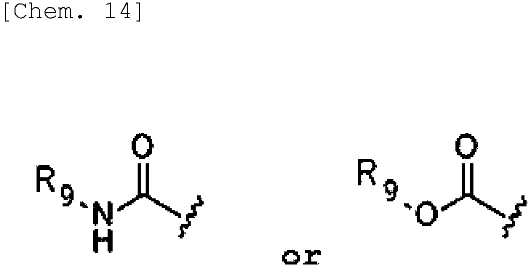 wherein
R9 is independently a hydrogen, an optionally substituted C1-6 alkyl groups, an optionally substituted C6-10 aryl groups, an optionally substituted C5-10 heteroaryl groups, or a protecting group.

13. The compound according to claim 12, its enantiomer, or a pharmaceutically acceptable salt thereof:
wherein
R3 is a group represented by:

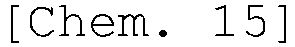

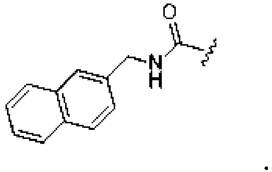

14. The compound according to claim 7, its enantiomer, or a pharmaceutically acceptable salt thereof, wherein R2 and R4 are the same group.

15. The compound according to claim 7, its enantiomer, or a pharmaceutically acceptable salt thereof, wherein X and Y combine together with an adjacent carbon atom to form a double bond.

16. A pharmaceutical composition which comprises the compound according to any one of claims 1 to 15, its enantiomer, or a pharmaceutically acceptable salt thereof.

17. The pharmaceutical composition according to claim 16 for the treatment or prevention of SARS-CoV-2 novel coronavirus infection.
